(19) Europäisches Patentamt / European Patent Office / Office européen des brevets

(11) **EP 2 188 258 B1**

(12) **EUROPÄISCHE PATENTSCHRIFT**


(45) Veröffentlichungstag und Bekanntmachung des Hinweises auf die Patenterteilung:
**28.11.2012 Patentblatt 2012/48**

(21) Anmeldenummer: **08802179.5**

(22) Anmeldetag: **15.09.2008**

(51) Int Cl.:
***C07D 213/82*** (2006.01) ***C07D 401/12*** (2006.01)
***C07D 405/12*** (2006.01) ***C07D 409/12*** (2006.01)
***A61K 31/33*** (2006.01) ***A61P 25/06*** (2006.01)

(86) Internationale Anmeldenummer:
**PCT/EP2008/007633**

(87) Internationale Veröffentlichungsnummer:
**WO 2009/036938 (26.03.2009 Gazette 2009/13)**


(54) **SUBSTITUIERTE NICOTINAMID-VERBINDUNGEN UND DEREN VERWENDUNG IN ARZNEIMITTELN**

SUBSTITUTED NICOTINAMIDE COMPOUNDS AND THE USE THEREOF IN PHARMACEUTICAL PRODUCTS

COMPOSÉS DE NICOTINAMIDE SUBSTITUÉS, ET LEUR UTILISATION DANS DES MÉDICAMENTS


(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **17.09.2007 DE 102007044277**

(43) Veröffentlichungstag der Anmeldung:
**26.05.2010 Patentblatt 2010/21**



(73) Patentinhaber: **Grünenthal GmbH**
**52078 Aachen (DE)**

(72) Erfinder:
- **MERLA, Beatrix**
**52078 Aachen (DE)**
- **KÜHNERT, Sven**
**52355 Düren (DE)**
- **FRANK, Robert**
**52070 Aachen (DE)**
- **KAULARTZ, Dagmar**
**52222 Stolberg (DE)**
- **SCHRÖDER, Wolfgang**
**52074 Aachen (DE)**
- **BAHRENBERG, Gregor**
**52156 Monschau-Konzen (DE)**
- **SCHIENE, Klaus**
**41363 Jüchen (DE)**



(56) Entgegenhaltungen:
**WO-A1-99/24404 WO-A1-2005/023802**



Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).





Printed by Jouve, 75001 PARIS (FR)

## Beschreibung

Die vorliegende Erfindung betrifft substituierte Nicotinamid-Verbindungen, Verfahren zu ihrer Herstellung, Arzneimittel enthaltend diese Verbindungen sowie die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Ein pathophysiologisches Merkmal von chronischen Schmerzen besteht in der Überregbarkeit von Neuronen. Die neuronale Erregbarkeit wird entscheidend von der Aktivität von $K^+$ Kanälen beeinflusst, da diese das Ruhemembranpotential der Zelle und somit die Erregbarkeitsschwelle maßgeblich bestimmen. Heteromere $K^+$ Kanäle vom molekularen Subtyp KCNQ2/3 (Kv7.2/7.3) sind in Neuronen verschiedener Regionen des zentralen (Hippocampus, Amygdala) und peripheren (Hinterwurzelganglien) Nervensystems exprimiert und regulieren deren Erregbarkeit. Die Aktivierung von KCNQ2/3 $K^+$ Kanälen führt zu einer Hyperpolarisation der Zellmembran und damit einhergehend zu einer Abnahme der elektrischen Erregbarkeit dieser Neurone. KCNQ2/3 exprimierende Neurone der Hinterwurzelganglien sind an der Übertragung nociceptiver Erregungen von der Peripherie ins Rückenmark beteiligt (Passmore et al., J Neurosci. 2003; 23(18):7227-36). Dementsprechend konnte für den KCNQ2/3 Agonisten Retigabine eine analgetische Wirksamkeit in präklinischen Neuropathie- und Entzündungsschmerzmodellen nachgewiesen werden (Blackburn-Munro and Jensen, Eur J Pharmacol. 2003; 460(2-3):109-16; Dost et al., Naunyn Schmiedebergs Arch Pharmacol 2004; 369(4): 382-390). Der KCNQ2/3 $K^+$ Kanal stellt somit einen geeigneten Ansatzpunkt zur Behandlung von Schmerz; insbesondere von Schmerz ausgewählt aus der Gruppe bestehend aus chronischem Schmerz, neuropathischem Schmerz, inflammatorischem Schmerz und muskulärem Schmerz (Nielsen et al., Eur J Pharmacol. 2004; 487(1-3): 93-103), insbesondere von neuropathischem und inflammatorischem Schmerz dar.

Darüber hinaus ist der KCNQ2/3 $K^+$ Kanal ein geeignetes Target für die Therapie einer Vielzahl weiterer Erkrankungen wie beispielsweise Migräne (US2002/0128277), kognitive Erkrankungen (Gribkoff, Expert Opin Ther Targets 2003; 7(6): 737-748), Angstzuständen (Korsgaard et al., J Pharmacol Exp Ther. 2005, 14(1): 282-92), Epilepsie (Wickenden et al., Expert Opin Ther Pat 2004; 14(4): 457-469) und Harninkontinenz (Streng et al., J Urol 2004;172: 2054-2058). WO 2005 023802 offenbart Nicotinamid-Verbindungen die Verwendbar sind zur Behandlung von Schmerz.

Eine Aufgabe der vorliegenden Erfindung bestand daher darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch KCNQ2/3 $K^+$ Kanäle vermittelt werden.

Überraschenderweise wurde nun gefunden, dass substituierte Nicotinamid-Verbindungen der nachstehend angegebenen allgemeinen Formel I sich zur Behandlung von Schmerzen eignen und auch eine ausgezeichnete Affinität zum KCNQ2/3 $K^+$ Kanal aufweisen und sich daher zur Behandlung von Störungen oder Krankheiten eignen, die zumindest teilweise durch KCNQ2/3 $K^+$ Kanäle vermittelt werden.

Ein Gegenstand der vorliegenden Erfindung sind daher substituierte Nicotinamid-Verbindungen der allgemeinen Formel I,

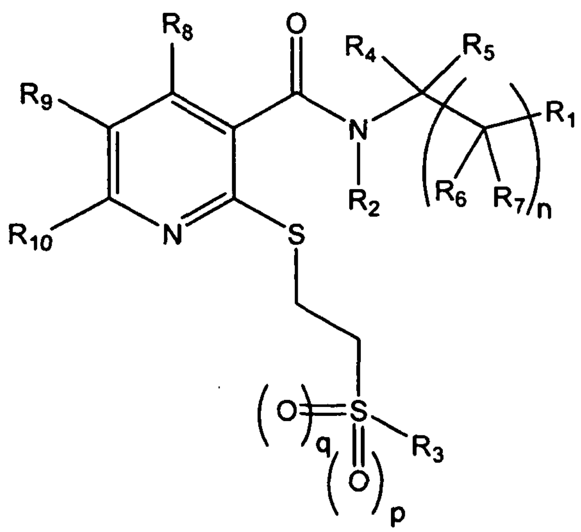

worin

n = 0, 1 oder 2

p = 0 oder 1

q = 0 oder 1

$R^1$ Aryl oder Heteroaryl, unsubstituiert oder einfach oder mehrfach substituiert; $C_{1-6}$-Alkyl, $C_{3-10}$-Cycloalkyl oder Heterocyclyl, unsubstituiert oder einfach oder mehrfach substituiert;

$R^2$ H; $C_{1-6}$-Alkyl, unsubstituiert oder einfach oder mehrfach substituiert;

$R^3$ Aryl oder Heteroaryl, unsubstituiert oder einfach oder mehrfach substituiert; $C_{1-6}$-Alkyl oder $C_{3-10}$-Cycloalkyl, jeweils unsubstituiert oder einfach oder mehrfach substituiert;

$R^4$, $R^5$, $R^6$ und $R^7$ unabhängig voneinander H; $C_{1-6}$-Alkyl, unsubstituiert oder einfach oder mehrfach substituiert;

$R^8$, $R^9$ und $R^{10}$ unabhängig voneinander H, F, Cl, Br, O-$C_{1-6}$-Alkyl, $CF_3$, $OCF_3$, $SCF_3$, $C_{1-6}$-Alkyl; bedeutet;

mit der Maßgabe, dass wenn $R^3$ 3-Trifluormethylphenyl oder 4-Trifluormethyl-2-pyridyl, $R^2$, $R^4$ und $R^5$ H sowie n 0 bedeuten, $R^1$ nicht 2-Pyridyl oder 2-Thienyl bedeutet;

und

wenn $R^3$ Phenyl oder Methyl, $R^2$, $R^4$ und $R^5$ H sowie n 0 bedeuten, $R^1$ nicht 2-Thienyl bedeutet;

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Im Zusammenhang mit "Phenyl", "Phenyloxy", "Benzyl", "Benzyloxy", "Alkylaryl" umfasst der Begriff jeweils die unsubstituierte Struktur als auch die durch F, Cl, $OCH_3$, $CF_3$, $OCF_3$, $SCF_3$ und $CH_3$ substituierte Struktur.

Der Ausdruck "$C_{1-6}$-Alkyl" umfasst im Sinne dieser Erfindung acyclische gesättigte oder ungesättigte Kohlenwasserstoffreste, die verzweigt- oder geradkettig sowie unsubstituiert oder ein- oder mehrfach substituiert sein können mit 1 bis 6 C-Atomen, d. h. $C_{1-6}$-Alkanyle, $C_{2-6}$-Alkenyle und $C_{2-6}$-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, Hexyl, Ethylenyl (Vinyl), Ethinyl, Propenyl ($-CH_2CH=CH_2$, $-CH=CH-CH_3$, $-C(=CH_2)-CH_3$), Propinyl (-CH-C=CH, -C=C-$CH_3$), Butenyl, Butinyl, Pentenyl, Pentinyl, Hexenyl und Hexinyl umfasst. Besonders vorteihaft sind Methyl, Ethyl und *tert.*-Butyl.

Der Ausdruck "Cycloalkyl" oder "$C_{3-10}$-Cycloalkyl" bedeutet für die Zwecke dieser Erfindung cyclische Kohlenwasserstoffe mit 3, 4, 5, 6, 7, 8, 9 oder 10 Kohlenstoffatomen, wobei die Kohlenwasserstoffe gesättigt oder ungesättigt (aber nicht aromatisch), unsubstituiert oder ein- oder mehrfach substituiert, verbrückt oder unverbrückt sein können. Vorteilhaft ist $C_{3-8}$-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Bicyclo[3.3.1]heptanyl und Adamantyl enthält.

Der Begriff "Heterocyclyl" umfasst gesättigte oder ungesättigte (aber nicht aromatische) Cycloalkyle mit drei bis acht Ringgliedern, in denen ein oder zwei Kohlenstoffatome durch ein Heteroatom S, N oder O ersetzt sind. Vorteilhaft sind Heterocyclyl-Reste aus der Gruppe Tetrahydropyranyl, Dioxanyl, Dioxolanyl, Morpholinyl, Piperidinyl, Piperazinyl, Pyrazolinonyl und Pyrrolidinyl.

Der Ausdruck "Aryl" bedeutet im Sinne dieser Erfindung aromatische Kohlenwasserstoffe mit bis zu 14 Ringgliedern, u.a. Phenyle und Naphthyle. Die Aryl-Reste können auch mit weiteren gesättigten, (partiell) ungesättigten oder aromatischen Ringsystemen, das gegebenenfalls ein oder zwei Heteroatome aus der Gruppe O, N oder S enthält, kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Vorteilhafterweise ist Aryl aus der Gruppe ausgewählt, die Phenyl, 1-Naphthyl, 2-Naphthyl, welche jeweils unsubstituiert oder ein- oder mehrfach substituiert sein können, enthält.

Der Ausdruck "Heteroaryl" steht für einen 5-, 6- oder 7-gliedrigen cyclischen aromatischen Rest, der mindestens

1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems mit bis zu 14 Ringgliedern sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxadiazolyl, Isoxazoyl, Pyridyl, Pyridazinyl, Pyrimidinyl, Pyrazinyl, Indazolyl, Purinyl, Indolizinyl, Chinolinyl, Isochinolinyl, Chinazolinyl, Carbazolyl, Phenazinyl, oder Oxadiazolyl enthält, wobei die Bindung an die Verbindungen der allgemeinen Struktur I über jedes beliebige und mögliche Ringglied des Heteroaryl-Restes erfolgen kann. Besonders bevorzugt sind Pyridyl, Furyl und Thienyl.

Im Zusammenhang mit "Alkyl", "Heterocyclyl" und "Cycloalkyl" versteht man unter dem Begriff "substituiert" im Sinne dieser Erfindung die Substitution eines Wasserstoffrestes durch F, Cl, Br, I, -CN, $NH_2$, NH-$C_{1-6}$-Alkyl, NH-$C_{1-6}$-Alkyl-OH, $C_{1-6}$-Alkyl, N($C_{1-6}$-Alkyl)$_2$, N($C_{1-6}$-Alkyl-OH)$_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, C(=O)$C_{1-6}$Alkyl, $CO_2H$, $CO_2$-$C_{1-6}$-Alkyl, Phenyl, Phenoxy, Morpholinyl, Piperidinyl, Pyrolidinyl oder Benzyl, wobei unter mehrfach substituierten Resten solche Reste zu verstehen sind, die entweder an verschiedenen oder an gleichen Atomen mehrfach, z.B. zwei- oder dreifach, substituiert sind, beispielsweise dreifach am gleichen C-Atom wie im Falle von $CF_3$ oder -$CH_2CF_3$ oder an verschiedenen Stellen wie im Falle von -CH(OH)-CH=CH-$CHCl_2$. Die Mehrfachsubstitution kann mit dem gleichen oder mit verschiedenen Substituenten erfolgen.

In Bezug auf "Aryl" und "Heteroaryl" versteht man im Sinne dieser Erfindung unter "ein- oder mehrfach substituiert" die ein- oder mehrfache, z.B. zwei-, drei- oder vierfache, Substitution eines oder mehrerer Wasserstoffatome des Ringsystems durch F, Cl, Br, I, CN, $NH_2$, NH-$C_{1-6}$-Alkyl, NH- $C_{1-6}$-Alkyl-OH, N($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alkyl, O-$C_{1-6}$Alkyl-OH, C(=O)$C_{1-6}$-Alkyl, C(=O)NH$C_{1-6}$-Alkyl; o-Pyridyl; C(=O)-Aryl; C(=O)-N-Morpholin; C(=O)-Piperidin; (C=O)-Pyrrolidin; (C=O)-Piperazin; $NHSO_2C_{1-6}$-Alkyl, $NHCOC_{1-6}$-Alkyl, $CO_2H$, $CH_2SO_2$-Phenyl, $CO_2$-$C_{1-6}$-Alkyl, $OCF_3$, $SCF_3$, $CF_3$,

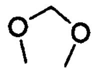, 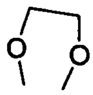,

$C_{1-6}$-Alkyl, Pyrolidinyl, Piperidinyl, Morpholinyl, Benzyloxy, Phenoxy, Phenyl, Pyridyl, Alkylaryl, Imidazolyl, Pyrazolyl, Thienyl oder Furyl; an einem oder ggf. verschiedenen Atomen, wobei ein Substituent ggf. seinerseits substituiert sein kann, jedoch nicht mit einem weiteren Aryl- oder Heteroarylring. Die Mehrfachsubstitution erfolgt dabei mit dem gleichen oder mit unterschiedlichen Substituenten. Für "Aryl" oder "Heteroaryl" sind bevorzugte Substituenten F, Cl, Br, $OCH_3$, $CF_3$, $OCF_3$, $SCF_3$ und $CH_3$.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Maleinsäure, Milchsäure, Zitronensäure, Glutaminsäure, 1,1-Dioxo-1,2-dihydro1$\lambda^6$-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Hippursäure, Phosphorsäure und/oder Asparaginsäure. Besonders bevorzugt sind die Zitronensäure und die Salzsäure.

Bevorzugt im Sinne dieser Erfindung sind substituierte Nicotinamid-Verbindungen der allgemeinen Formel I, worin

n = 0, 1 oder 2;
p = 0 oder 1;
q = 0 oder 1;
$R^1$ Aryl oder Heteroaryl, unsubstituiert oder einfach oder mehrfach substituiert; $C_{1-6}$-Alkyl, $C_{3-10}$-Cycloalkyl oder Heterocyclyl, unsubstituiert oder einfach oder mehrfach substituiert;
$R^2$ H; $C_{1-6}$-Alkyl, unsubstituiert oder einfach oder mehrfach substituiert;
$R^3$ Aryl oder Heteroaryl, unsubstituiert oder einfach oder mehrfach substituiert; $C_{1-6}$-Alkyl oder $C_{3-10}$-Cycloalkyl, jeweils unsubstituiert oder einfach oder mehrfach substituiert;
$R^4$, $R^5$, $R^6$ und $R^7$ unabhängig voneinander H; $C_{1-6}$-Alkyl, unsubstituiert oder einfach oder mehrfach substituiert;
$R^8$, $R^9$ und $R^{10}$ unabhängig voneinander H, F, Cl, Br, O-$C_{1-6}$-Alkyl, $CF_3$, $OCF_3$, $SCF_3$, $C_{1-6}$-Alkyl;
mit der Maßgabe, dass wenn $R^3$ 3-Trifluormethylphenyl oder 4-Trifluormethyl-2-pyridyl, $R^2$, $R^4$ und $R^5$ H sowie n 0

bedeuten, $R^1$ nicht 2-Pyridyl oder 2-Thienyl bedeutet;
und
wenn $R^3$ Phenyl oder Methyl, $R^2$, $R^4$ und $R^5$ H sowie n 0 bedeuten, $R^1$ nicht 2-Thienyl bedeutet;
wobei
"Alkyl substituiert", "Heterocyclyl substituiert" und "Cycloalkyl substituiert" für die Substitution eines Wasserstoffrestes durch F, Cl, Br, I, -CN, $NH_2$, NH-$C_{1-6}$-Alkyl, NH-$C_{1-6}$-Alkyl-OH, $C_{1-6}$-Alkyl, N($C_{1-6}$-Alkyl)$_2$, N($C_{1-6}$-Alkyl-OH)$_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, C(=O)$C_{1-6}$-Alkyl, $CO_2H$, $CO_2$-$C_{1-6}$-Alkyl, Phenyl, Phenoxy, Morpholinyl, Piperidinyl, Pyrolidinyl oder Benzyl, steht;
und "Aryl substituiert" und "Heteroaryl substituiert" für die ein- oder mehrfache, z.B. zwei-, drei- oder vierfache, Substitution eines oder mehrerer Wasserstoffatome des Ringsystems durch F, Cl, Br, I, CN, $NH_2$, NH-$C_{1-6}$-Alkyl, NH- $C_{1-6}$-Alkyl-OH, N($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alkyl, O-$C_{1-6}$Alkyl-OH, C(=O)$C_{1-6}$-Alkyl, C(=O) NH$C_{1-6}$-Alkyl; o-Pyridyl; C(=O)-Aryl; C(=O)-N-Morpholin; C(=O)-Piperidin; (C=O)-Pyrrolidin; (C=O)-Piperazin; $NHSO_2C_{1-6}$-Alkyl, NHCO$C_{1-6}$-Alkyl, $CO_2H$, $CH_2SO_2$-Phenyl, $CO_2$-$C_{1-6}$-Alkyl, $OCF_3$, $SCF_3$, $CF_3$,

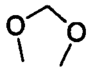,
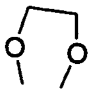,

$C_{1-6}$-Alkyl, Pyrolidinyl, Piperidinyl, Morpholinyl, Benzyloxy, Phenoxy, Phenyl, Pyridyl, Alkylaryl, Imidazolyl, Pyrazolyl, Thienyl oder Furyl;
bedeutet.

Bevorzugt sind substituierte Nicotinamid-Derivate der allgemeinen Formel I, worin p und q jeweils 1 bedeuten (Sulfone).

Bevorzugt sind ferner substituierte Nicotinamid-Derivate der allgemeinen Formel I, worin p und q jeweils 0 bedeuten (Thioether).

Weiterhin bevorzugt sind substituierte Nicotinamid-Derivate der allgemeinen Formel I, worin $R^8$, $R^9$ und $R^{10}$ H bedeuten.

Bevorzugt sind substituierte Nicotinamid-Derivate der allgemeinen Formel I, worin
$R^1$ für Pyrrolyl, Furyl, Thienyl, Pyrazolyl, Imidazolyl, Thiazolyl, Oxadiazolyl, Isoxazoyl, Pyridyl, Pyrimidinyl, Pyrazinyl, Thiadiazolyl, Oxazolyl, Isothiazolyl, Phenyl, Naphthyl, Cyclopropyl, Cyclobutyl, Cyclopentyl, Cyclohexyl, Cycloheptyl, Tetrahydropyranyl, Dioxanyl oder $C_{1-6}$-Alkyl, jeweils unsubstituiert oder einfach oder mehrfach substituiert;
insbesondere

$R^1$ für tert.-Butyl, Phenyl, Pyridyl, Thienyl, Furyl oder Cyclohexyl, unsubstituiert oder einfach oder mehrfach substituiert, bedeutet.

Besonders bevorzugt sind substituierte Nicotinamid-Derivate der allgemeinen Formel I, worin $R^1$ Cyclohexyl; Phenyl, unsubstituiert oder einfach oder mehrfach substituiert mit F, $CH_3$, Cl, Br, $CF_3$, $OCH_3$, $SCF_3$ oder $OCF_3$; Pyridyl, Thienyl oder Furyl, unsubstituiert oder einfach oder mehrfach substituiert mit $CH_3$; steht.

Es ist bevorzugt, dass $R^2$ für $CH_3$ oder H steht, insbesondere für H.

Weiterhin ist bevorzugt, dass $R^4$, $R^5$, $R^6$ und $R^7$ unabhängig voneinander für H oder $CH_3$ stehen, insbesondere für H.

Bevorzugt bedeutet n 0 oder 1, besonders bevorzugt 0.

Bevorzugt sind auch substituierte Nicotinamid-Derivate, worin $R^3$ Aryl oder Heteroaryl, unsubstituiert oder einfach oder mehrfach substituiert,
vorzugsweise
$R^3$ Phenyl oder Pyridyl, unsubstituiert oder einfach oder mehrfach substituiert, insbesondere Phenyl einfach oder mehrfach substituiert mit F, $CH_3$, $CF_3$, $OCF_3$, $OCH_3$, $SCF_3$ der Cl, bedeutet.

Besonders bevorzugt sind substituierte Nicotinamid-Derivate, worin $R^3$ Phenyl unsubstituiert oder substituiert mit $CF_3$ oder $CH_3$ bedeutet.

Besonders bevorzugt sind auch Verbindungen, bei denen die aufgeführten bevorzugten Definitionen für die Reste $R^1$-$R^7$ untereinander kombiniert sind.

Ganz besonders bevorzugt sind substituierte Nicotinamid-Derivate aus der Gruppe

| | |
|---|---|
| 1 | 2-(2-(Phenylsulfonyl)ethylthio)-N-(pyridin-2-ylmethyl)nicotinamid |
| 2 | 2-(2-(Phenylsulfonyl)ethylthio)-N-(pyridin-4-ylmethyl)nicotinamid |
| 3 | N-(3-Fluorophenethyl)-2-(2-(phenylsulfonyl)ethylthio)nicotinamid |
| 4 | N-Methyl-N-(3-methylbenzyl)-2-(2-(phenylsulfonyl)ethylthio)nicotinamid |

(fortgesetzt)

| | |
|---|---|
| 5 | N-(4-methylbenzyl)-2-(2-(phenylsulfonyl)ethylthio)nicotinamid |
| 6 | 2-(2-(Phenylsulfonyl)ethylthio)-N-(2-(trifluoromethyl)benzyl)nicotinamid |
| 7 | 2-(2-(Phenylsulfonyl)ethylthio)-N-(pyridin-3-ylmethyl)nicotinamid |
| 8 | N-(3,5-Difluorobenzyl)-2-(2-(phenylsulfonyl)ethylthio)nicotinamid |
| 9 | N-Methyl-N-phenethyl-2-(2-(phenylsulfonyl)ethylthio)nicotinamid |
| 10 | N-(3-Methoxybenzyl)-N-methyl-2-(2-(phenylsulfonyl)ethylthio)nicotinamid |
| 11 | N-(2-Fluorobenzyl)-2-(2-(phenylsulfonyl)ethylthio)nicotinamid |
| 12 | N-(3,4-Difluorobenzyl)-2-(2-(phenylsulfonyl)ethylthio)nicotinamid |
| 13 | N-(3-Bromobenzyl)-N-methyl-2-(2-(phenylsulfonyl)ethylthio)nicotinamid |
| 14 | N-(2-Methoxybenzyl)-2-(2-(phenylsulfonyl)ethylthio)nicotinamid |
| 15 | N-(3-Fluorobenzyl)-2-(2-(phenylsulfonyl)ethylthio)nicotinamid |
| 16 | N-(Furan-2-ylmethyl)-N-methyl-2-(2-(phenylsulfonyl)ethylthio)nicotinamid |
| 17 | N-(4-Methoxybenzyl)-2-(2-(phenylsulfonyl)ethylthio)nicotinamid |
| 18 | N-(2-Chlorobenzyl)-2-(2-(phenylsulfonyl)ethylthio)nicotinamid |
| 19 | N-(3,4-Dichlorobenzyl)-2-(2-(phenylsulfonyl)ethylthio)nicotinamid |
| 20 | N-(4-Fluorobenzyl)-2-(2-(phenylsulfonyl)ethylthio)nicotinamid |
| 21 | N-(2-Methoxyphenethyl)-2-(2-(phenylsulfonyl)ethylthio)nicotinamid |
| 22 | N-(2,6-Difluorobenzyl)-2-(2-(phenylsulfonyl)ethylthio)nicotinamid |
| 23 | N-(2-Methylbenzyl)-2-(2-(phenylsulfonyl)ethylthio)nicotinamid |
| 24 | N-(3,5-Dimethoxybenzyl)-2-(2-(phenylsulfonyl)ethylthio)nicotinamid |
| 25 | N-(3-Chlorobenzyl)-2-(2-(phenylsulfonyl)ethylthio)nicotinamid |
| 26 | N-(2,4-Dichlorobenzyl)-2-(2-(phenylsulfonyl)ethylthio)nicotinamid |
| 29 | N-(4-Chlorobenzyl)-2-(2-(phenylsulfonyl)ethylthio)nicotinamid |
| 30 | N-(2,3-Dichlorobenzyl)-2-(2-(phenylsulfonyl)ethylthio)nicotinamid |
| 31 | N-(4-Bromobenzyl)-N-methyl-2-(2-(phenylsulfonyl)ethylthio)nicotinamid |
| 32 | N-((1,3-Dioxolan-2-yl)methyl)-N-methyl-2-(2-(phenylsulfonyl)ethylthio)nicotinamid |
| 33 | N-Benzyl-N-methyl-2-(2-tosylethylthio)nicotinamid |
| 34 | N-(Pyridin-2-ylmethyl)-2-(2-tosylethylthio)nicotinamid |
| 35 | N-(Pyridin-4-ylmethyl)-2-(2-tosylethylthio)nicotinamid |
| 36 | N-(Thiophen-2-ylmethyl)-2-(2-tosylethylthio)nicotinamid |
| 37 | N-(3-Fluorophenethyl)-2-(2-tosylethylthio)nicotinamid |
| 38 | N-Methyl-N-(3-methylbenzyl)-2-(2-tosylethylthio)nicotinamid |
| 39 | N-(Furan-2-ylmethyl)-2-(2-tosylethylthio)nicotinamid |
| 40 | N-(Pyridin-3-ylmethyl)-2-(2-tosylethylthio)nicotinamid |
| 41 | N-(3.5-Difluorobenzyl)-2-(2-tosylethylthio)nicotinamid |
| 42 | N-(3-Methoxybenzyl)-N-methyl-2-(2-tosylethylthio)nicotinamid |
| 43 | N-(2-Fluorobenzyl)-2-(2-tosylethylthio)nicotinamid |
| 44 | N-(3-Methylbenzyl)-2-(2-tosylethylthio)nicotinamid |
| 45 | N-(3.4-Difluorobenzyl)-2-(2-tosylethylthio)nicotinamid |
| 46 | N-(3-Bromobenzyl)-N-methyl-2-(2-tosylethylthio)nicotinamid |
| 47 | N-(4-Methoxybenzyl)-2-(2-tosylethylthio)nicotinamid |
| 48 | N-(2-Chlorobenzyl)-2-(2-tosylethylthio)nicotinamid |
| 49 | N-(4-Fluorobenzyl)-2-(2-tosylethylthio)nicotinamid |
| 50 | N-(3,5-Dimethoxybenzyl)-2-(2-tosylethylthio)nicotinamid |
| 51 | N-(3-Chlorobenzyl)-2-(2-tosylethylthio)nicotinamid |
| 52 | 2-(2-Tosylethylthio)-N-(3-(trifluoromethyl)benzyl)nicotinamid |
| 54 | N-Benzyl-2-(2-(phenylsulfonyl)ethylthio)nicotinamid |
| 55 | N-Benzyl-N-methyl-2-(2-(phenylsulfonyl)ethylthio)nicotinamid |
| 56 | N-(Cyclohexylmethyl)-2-(2-(phenylsulfonyl)-ethylthio)nicotinamid |
| 57 | 2-(2-(Phenylsulfonyl)ethylthio)-N-(1-(3-(trifluormethyl)phenyl)-ethyl)nicotinamid |
| 58 | N-(2-Cyclohexyl-ethyl)-2-(2-(phenylsulfonyl)ethylthio)nicotinamid |

(fortgesetzt)

| | |
|---|---|
| 59 | 2-(2-(Cyclohexylthio)ethylthio)-N-(thiophen-2-ylmethyl)nicotinamid |
| 60 | N-(Neopentyl)-2-(2-(phenylsulfonyl)ethylthio)nicotinamid |
| 61 | N-(5-Methylfuran-2-yl-methyl)-2-(2-(phenylsulfonyl)ethylthio)nicotinamid |
| 62 | N-(Furan-2-yl-methyl)-2-(2-(phenylsulfonyl)ethylthio)nicotinamid |
| 63 | 2-(2-(Phenylsulfonyl)ethylthio)-N-(tetrahydro-2H-pyran-4-yl-methyl)nicotinamid |
| 64 | 2-(2-(Phenylsutfonyl)ethylthio)-N-(4-(trifluormethylthio)benzyl)nicotinamid |
| 65 | 2-(2-(Phenylsulfonyl)ethylthio)-N-(3-tolylmethyl)nicotinamid |
| 66 | R)-N-(1-Cyclohexyl-ethyl)-2-(2-(phenylsulfonyl)ethylthio)nicotinamid |
| 67 | N-(1-(3,4-Dimethylphenyl)ethyl)-2-(2-(phenylsulfonyl)ethylthio)nicotinamid |
| 68 | N-(1-Thiophen-2-ylethyl)-2-(2-(phenylsutfonyl)ethylthio)nicotinamid |
| 69 | N-(1-(3,5-Dimethylphenyl)methyl)-2-(2-(phenylsulfonyl)ethylthio)nicotinamid |
| 70 | N-(Cyclohexylmethyl)-2-(2-(3-trifluormethyl-phenylsulfonyl)ethylthio)nicotinamid |
| 71 | (S)-N-(1-Cyclohexyl-ethyl)-2-(2-(phenylsulfonyl)ethylthio)nicotinamid |
| 72 | N-(1-(3,5-Dimethylphenyl)ethyl)-2-(2-(phenylsulfonyl)ethylthio)nicotinamid |
| 73 | N-(Thiophen-2-ylmethyl)-2-(2-(3-(trifluormethyl)phenylthio)ethylthio)nicotinamid |
| 74 | N-(Cyclopentylmethyl)-2-(2-(phenylsulfonyl)ethylthio)nicotinamid |
| 75 | N-(Cyclobutylmethyl)-2-(2-(phenylsulfonyl)ethylthio)nicotinamid |
| 76 | N-((1,4-Dioxan-2-yl)methyl)-2-(2-(phenylsulfonyl)ethylthio)nicotinamid |
| 77 | 2-(2-(Phenylsulfonyl)ethylthio)-N-(4-(pyridin-2-yloxy)benzyl)nicotinamid |
| 78 | N-(2-Methylbutyl)-2-(2-(phenylsutfonyl)ethylthio)nicotinamid |
| 79 | N-(2-ethylbutyl)-2-(2-(phenylsulfonyl)ethylthio)nicotinamid |
| 80 | N-(Cyclopropylmethyl)-2-(2-(phenylsulfonyl)ethylthio)nicotinamid |
| 81 | N-(3-(2-Methoxyethoxy)propyl)-2-(2-(phenylsulfonyl)ethylthio)nicotinamid |
| 82 | 2-(2-(Phenylsulfonyl)ethylthio)-N-(1-(4-(trifluormethylthio)phenyl)ethyl)nicotinamid |
| 83 | N-(3-(1H-Pyrazol-1-yl)benzyl)-2-(2-(phenylsulfonyl)ethylthio)nicotinamid |
| 84 | N-((2,3-Dihydrobenzofuran-5-yl)methyl)-2-(2-(phenylsulfonyl)ethylthio)nicotinamid |
| 85 | N-(4-Phenoxybenzyl)-2-(2-(phenylsulfonyl)ethylthio)nicotinamid |
| 86 | N-(((1R,2S,5R)-6,6-dimethylbicyclo[3.1.1]heptan-2-yl)methyl)-2-(2-(phenylsulfonyl)-ethylthio)nicotinamid |
| 87 | N-(Thiophen-2-ylmethyl)-2-(2-(3-(trifluoromethyl)phenylsulfonyl)ethylthio)nicotinamid |
| 88 | 2-(2-(Phenylsulfonyl)ethylthio)-N-(3-(trifluoromethyl)benzyl)nicotinamid |
| 93 | N-Isobutyl-2-(2-(phenylsulfonyl)ethylthio)nicotinamid |
| 94 | 2-[2-(Benzolsulfonyl)ethylthio]-N-(2-tetrahydropyranylmethyl)-nicotinamid |
| 95 | 2-[2-(Benzolsulfonyl)ethylthio]-N-[(5-methyl-2-thienyl)methyl]-nicotinamid |
| 96 | 2-[2-(Benzolsulfonyl)ethylthio]-N-[(4-methyl-2-thienyl)methyl]-nicotinamid |
| 97 | N-(1-Adamantylmethyl)-2-[2-(benzolsulfonyl)ethylthio]-nicotinamid |
| 98 | 2-[2-(Benzolsulfonyl)ethylthio]-N-[(3-morpholinophenyl)methyl]-nicotinamid |
| 99 | 2-[2-(4-Chlorphenyl)sulfonylethylthio]-N-(2-thienylmethyl)-nicotinamid |
| 100 | 2-[2-(4-Fluorphenyl)sulfonylethylthio]-N-(2-thienylmethyl)-nicotinamid |
| 101 | N-(2-Thienylmethyl)-2-[2-[3-(trifluormethoxy)phenyl]sulfonylethylthio]-nicotinamid |
| 102 | N-(2-Thienylmethyl)-2-[2-[4-(trifluormethyl)phenyl]sulfonylethylthio]-nicotinamid |
| 103 | N-(2-Thienylmethyl)-2-[2-[4-(trifluormethoxy)phenyl]sulfonylethylthio]-nicotinamid |
| 104 | 2-[2-(m-Tolylsulfonyl)ethylthio]-N-(2-thienylmethyl)-nicotinamid |
| 105 | 2-[2-(m-Tolylthio)ethylthio]-N-(2-thienylmethyl)-nicotinamid |
| 106 | 2-[2-(3-Fluorphenyl)sulfonylethylthio]-N-(2-thienylmethyl)-nicotinamid |
| 107 | 2-[2-(Benzolsulfonyl)ethylthio]-N-(3,3-dimethylbutyl)-nicotinamid |
| 108 | 2-[2-(Benzolsulfonyl)ethylthio]-N-(2-benzothiophenylmethyl)-nicotinamid |
| 109 | 2-[2-(Phenylthio)ethylthio]-N-(2-thienylmethyl)-nicotinamid |
| 110 | 2-[2-(Benzolsulfinyl)ethylthio]-N-(2-thienylmethyl)-nicotinamid |
| 111 | 2-(2-Cyclohexylsulfonylethylthio)-N-(2-thienylmethyl)-nicotinamid |
| 112 | N-(2-Thienylmethyl)-2-[2-[[2-(trifluormethyl)phenyl]thio]ethylthio]-nicotinamid |
| 113 | N-(2-Thienylmethyl)-2-[2-[2-(trifluormethyl)phenyl]sulfinylethylthio]-nicotinamid |

(fortgesetzt)

| | |
|---|---|
| 114 | N-(2-Thienylmethyl)-2-[2-[2-(trifluormethyl)phenyl]sulfonylethylthio]-nicotinamid |
| 115 | 2-[2-(Benzolsulfonyl)ethylthio]-N-[(5-chlor-2-thienyl)methyl]-nicotinamid |
| 116 | 2-[2-(2-Fluorphenyl)sulfonylethylthio]-N-(2-thienylmethyl)-nicotinamid |
| 117 | 2-[2-[3,5-bis(Trifluormethyl)phenyl]sulfonylethylthio]-N-(2-thienylmethyl)-nicotinamid |
| 118 | 2-[2-(3-Methoxyphenyl)sulfonylethylthio]-N-(2-thienylmethyl)-nicotinamid |
| 119 | 2-[2-(4-Methoxyphenyl)sulfonylethylthio]-N-(2-thienylmethyl)-nicotinamid |
| 120 | 2-[2-(Benzolsulfonyl)ethylthio]-N-(4-tetrahydrothiopyranylmethyl)-nicotinamid |
| 121 | 2-[2-(4-Ethylphenyl)sulfonylethylthio]-N-(2-thienylmethyl)-nicotinamid |
| 122 | N-(2-Thienylmethyl)-2-[2-[[4-(trifluormethyl)phenyl]thio]ethylthio]-nicotinamid |
| 123 | 2-[2-(o-Tolylthio)ethylthio]-N-(2-thienylmethyl)-nicotinamid |
| 124 | 2-[2-[(3-Fluorphenyl)thio]ethylthio]-N-(2-thienylmethyl)-nicotinamid |
| 125 | 2-[2-[(3,4-Difluorphenyl)thio]ethylthio]-N-(2-thienylmethyl)-nicotinamid |
| 126 | 2-[2-[(2,4-Difluorphenyl)thio]ethylthio]-N-(2-thienylmethyl)-nicotinamid |
| 127 | 2-[2-(Benzolsulfonyl)ethylthio]-N-[2-(2-thienyl)ethyl]-nicotinamid |
| 128 | 2-[2-(Benzolsulfonyl)ethylthio]-N-phenthyl-nicotinamid |
| 129 | 2-[2-(Benzolsulfonyl)ethylthio]-N-(3-phenylpropyl)-nicotinamid |
| 130 | 2-[2-(3,4-Difluorphenyl)sulfonylethylthio]-N-(2-thienylmethyl)-nicotinamid |
| 131 | 2-[2-(2,4-Difluorphenyl)sulfonylethylthio]-N-(2-thienylmethyl)-nicotinamid |
| 132 | 2-[2-[(2-Fluorphenyl)thio]ethylthio]-N-(2-thienylmethyl)-nicotinamid |
| 133 | 2-[2-[(4-Fluorphenyl)thio]ethylthio]-N-(2-thienylmethyl)-nicotinamid |
| 134 | 2-[2-[(4-Chlorphenyl)thio]ethylthio]-N-(2-thienylmethyl)-nicotinamid |
| 135 | 2-[2-(p-Tolylthio)ethylthio]-N-(2-thienylmethyl)-nicotinamid |
| 136 | 2-[2-(Benzolsulfonyl)ethylthio]-N-isopentyl-nicotinamid |
| 137 | 2-[2-(Benzolsulfonyl)ethylthio]-N-(2-cyclopropylethyl)-nicotinamid |
| 138 | 2-[2-(Benzolsulfonyl)ethylthio]-N-(2-cyclopentylethyl)-nicotinamid |
| 139 | N-(3,3-Dimethylbutyl)-2-[2-[3-(trifluormethyl)phenyl]sulfonylethylthio]-nicotinamid |
| 140 | N-(Cyclopentylmethyl)-2-[2-[3-(trifluormethyl)phenyl]sulfonylethylthio]-nicotinamid |
| 141 | 2-[2-(Benzolsulfonyl)ethylthio]-6-methyl-N-(2-thienylmethyl)-nicotinamid |
| 142 | 2-[2-(Benzolsulfonyl)ethylthio]-N-(2-thienylmethyl)-6-(trifluormethyl)-nicotinamid |
| 143 | 2-[2-(Benzolsulfonyl)ethylthio]-6-fluor-N-(2-thienylmethyl)-nicotinamid |
| 144 | 2-[2-(Benzolsulfonyl)ethylthio]-N-[(3-methylcyclohexyl)methyl]-nicotinamid |
| 145 | 2-[2-(Benzolsulfonyl)ethylthio]-N-(cycloheptylmethyl)-nicotinamid |
| 146 | 2-[2-(Benzolsulfonyl)ethylthio]-N-[(2-methylcyclohexyl)methyl]-nicotinamid |
| 147 | 2-[2-(Benzolsulfonyl)ethylthio]-N-[(4-methylcyclohexyl)methyl]-nicotinamid |
| 148 | 2-[2-(Benzolsulfonyl)ethylthio]-5-fluor-N-(2-thienylmethyl)-nicotinamid |
| 149 | 2-[2-(Benzolsulfonyl)ethylthio]-5-methyl-N-(2-thienylmethyl)-nicotinamid |
| 150 | 2-[2-(Benzolsulfonyl)ethylthio]-N-(2-thienylmethyl)-5-(trifluormethyl)-nicotinamid. |

Die erfindungsgemäßen substituierten Nicotinamid-Verbindungen sowie jeweils die entsprechenden Säuren, Basen, Salze und Solvate eignen sich als pharmazeutische Wirkstoffe in Arzneimitteln.

Ein weiterer Gegenstand der vorliegenden Erfindung ist daher ein Arzneimittel enthaltend wenigstens eine erfindungsgemäße substituierte Nicotinamid-Verbindung der allgemeinen Formel I, worin

n = 0, 1 oder 2

p = 0 oder 1

q = 0 oder 1

$R^1$ Aryl oder Heteroaryl, unsubstituiert oder einfach oder mehrfach substituiert; $C_{1-6}$-Alkyl, $C_{3-10}$-Cycloalkyl oder Heterocyclyl, unsubstituiert oder einfach oder mehrfach substituiert;

$R^2$ H; $C_{1-6}$-Alkyl, unsubstituiert oder einfach oder mehrfach substituiert;

$R^3$ Aryl oder Heteroaryl, unsubstituiert oder einfach oder mehrfach substituiert; $C_{1-6}$-Alkyl oder $C_{3-10}$-Cycloalkyl, jeweils unsubstituiert oder einfach oder mehrfach substituiert;

$R^4$, $R^5$, $R^6$ und $R^7$ unabhängig voneinander H; $C_{1-6}$-Alkyl, unsubstituiert oder einfach oder mehrfach substituiert,

$R^8$, $R^9$ und $R^{10}$ unabhängig voneinander H, F, Cl, Br, O-$C_{1-6}$-Alkyl, $CF_3$, $OCF_3$, $SCF_3$, $C_{1-6}$-Alkyl;

sowie ggf. einen oder mehrere pharmazeutisch verträgliche Hilfsstoffe.
Bevorzugt sind Arzneimittel in den oben genannten bevorzugten Bereichen und deren Kombinationen.
Besonders bevorzugt sind Arzneimittel aus der folgenden Gruppe:

| | |
|---|---|
| 89 | 2-(2-(Phenylsulfonyl)ethylthio)-N-(thiophen-2-ylmethyl)nicotinamid |
| 90 | N-(Pyridin-2-ylmethyl)-2-(2-(3-(trifluoromethyl)phenylsulfonyl)ethylthio)nicotinamid |
| 91 | N-(Pyridin-2-ylmethyl)-2-(2-(5-(trifluoromethyl)pyridin-2-ylsulfonyl)ethylthio)nicotinamid |
| 92 | N-(Thiophen-2-ylmethyl)-2-(2-(5-(trifluoromethyl)pyridin-2-ylsulfonyl)ethylthio)nicotinamid. |

Diese erfindungsgemäßen Arzneimittel eignen sich zur Beeinflussung von KCNQ2/3-Kanälen und üben eine agonistische oder antagonistische, insbesondere eine agonistische Wirkung aus.
Bevorzugt eignen sich die erfindungsgemäßen Arzneimittel zur Behandlung von Störungen oder Krankheiten, die zumindest teilweise durch KCNQ2/3-Kanäle vermittelt werden.
Bevorzugt eignet sich das erfindungsgemäße Arzneimittel zur Behandlung von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz, Migräne; Epilepsie, Angstzuständen und Harninkontinenz. Besonders bevorzugt eignen sich die erfindungsgemäßen Arzneimittel zur Behandlung von Schmerz, ganz besonders bevorzugt von chronischem Schmerz, neuropathischem Schmerz, inflammatorischem Schmerz und muskulärem Schmerz.
Weiterhin eignen sich die erfindungsgemäßen Verbindungen bevorzugt zur Behandlung von Epilepsie.
Ein weiterer Gegenstand der vorliegenden Erfindung ist die Verwendung wenigstens einer erfindungsgemäßen substituierten Nicotinamid-Verbindung sowie ggf. eines oder mehrerer pharmazeutisch verträglicher Hilfsstoffe zur Herstellung eines Arzneimittels zur Behandlung von Störungen oder Krankheiten, die zumindest teilweise durch KCNQ2/3-Kanäle vermittelt werden.
Bevorzugt ist die Verwendung wenigstens einer erfindungsgemäßen substituierten Nicotinamid-Verbindung sowie ggf. eines oder mehrerer pharmazeutisch verträglicher Hilfsstoffe zur Herstellung eines Arzneimittels zur Behandlung von Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz; Migräne; Epilepsie, Angstzuständen und Harninkontinenz.
Besonders bevorzugt ist die Verwendung wenigstens einer erfindungsgemäßen substituierten Nicotinamid-Verbindung sowie ggf. eines oder mehrerer pharmazeutisch verträglicher Hilfsstoffe zur Herstellung eines Arzneimittels zur Behandlung von Schmerz, ganz besonders bevorzugt von chronischem Schmerz, neuropathischem Schmerz, inflammatorischem Schmerz und muskulärem Schmerz. Weiterhin besonders bevorzugt ist die Verwendung wenigstens einer erfindungsgemäßen substituierten Nicotinamid-Verbindung sowie ggf. eines oder mehrerer pharmazeutisch verträglicher Hilfsstoffe zur Herstellung eines Arzneimittels zur Behandlung von Epilepsie.
Die Wirksamkeit gegen Schmerz kann beispielsweise in den unten beschriebenen Bennett- bzw. Chung-Modell gezeigt werden. Die Wirksamkeit gegen Epilepsie kann beispielsweise im DBA/2 Maus Modell (De Sarro et al., Naunyn-Schmiedeberg's Arch. Pharmacol. 2001, 363, 330-336) nachgewiesen werden.
Die in den vorstehend beschriebenen Umsetzungen zum Einsatz kommenden Chemikalien und Reaktionskomponenten sind käuflich erhältlich oder können jeweils nach üblichen, dem Fachmann bekannten Methoden hergestellt werden.

**Allgemeine Synthese**

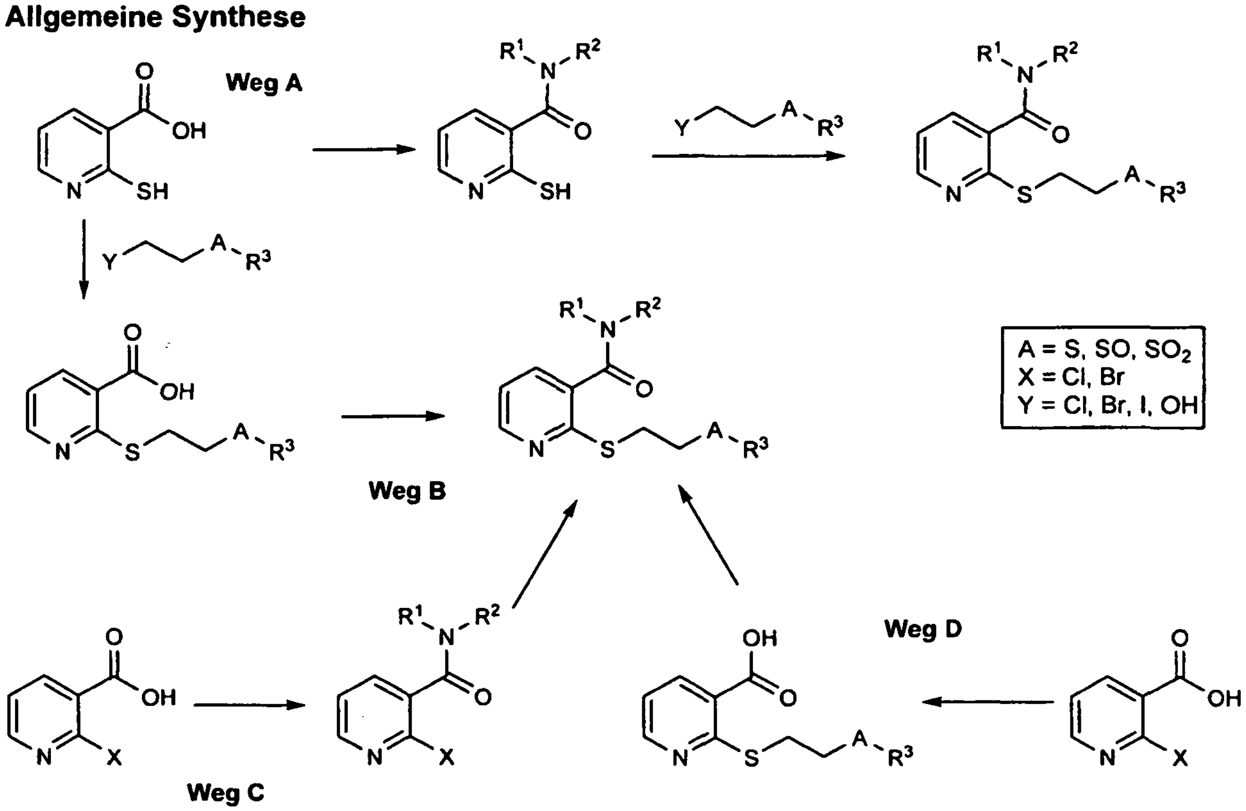


**Weg A (A = S, SO, $SO_2$)**

Die einleitende Acylierungsreaktion von Aminen mit Hilfe von Carbonsäuren, hier die Mercaptonicotinsäure, unter Verwendung von Basen und gegebenenfalls Kupplungsreagenzien kann in Lösungsmitteln, wie beispielsweise Methanol, DMF oder DCM durchgeführt werden. Als Basen können beispielsweise Natriummethanolat, Triethylamin, Diisopropylethylamin oder N-Methylmorpholin verwendet werden. Als Kupplungsreagenzien eignen sich beispielsweise EDCI, HOBt, DCC, CDI, HBTU, DMAP oder Pentafluorphenyldiphenylphosphinat. Die Reaktionszeit kann zwischen 1 h und 3 d variieren. Die Mercaptonicotinsäure kann aber auch zunächst in das Carbonsäurechlorid überführt werden. Hierzu eignen sich beispielsweise Reagenzien wie $COCl_2$, $PCl_3$, $POCl_3$, $P_2O_5$, $SOCl_2$ oder $SiCl_4$ in Lösungsmitteln wie beispielsweise Pyridin, DCM, DMF oder Toluol.

Für die anschließende Thioetherbildung ist es gegebenenfalls nötig den halogenierten Thioether Y-$CH_2$-$CH_2$-A-$R^3$ (mit A = S) herzustellen. Hierzu kann beispielsweise ein entsprechendes Thiol unter UV-Bestrahlung mit Vinylhalogeniden umgesetzt werden. Des weiteren kann der halogenierte Thioether Y-$CH_2$-$CH_2$-A-$R^3$ (mit A = S) beispielsweise durch Umsetzung eines entsprechenden Thiols mit einer Mischung aus Acetylen und Brom in Tetrachlorkohlenstoff erfolgen. Eine weitere Methode nutzt die Umsetzung von 1,2-Dihalogenalkanen mit einem Thiol in Benzol, Toluol oder Methanol in Gegenwart von Basen wie beispielsweise NaOH, KOH oder Natriummethanolat, gegebenenfalls unter Zugabe von Hydrazin oder Tricaprilmethylammoniumchlorid.

Gegebenfalls kann der entsprechende halogenierte Thioether Y-$CH_2$-$CH_2$-A-$R^3$ (mit A = S) zum entsprechenden Sulfoxid Y-$CH_2$-$CH_2$-A-$R^3$ (mit A = SO) oxidiert werden. Diese Oxidation kann mit Oxidationsmitteln wie beispielsweise $H_2O_2$, $NaIO_4$, $NaClO_2$, m-Chlorperbenzoesäure oder Oxon in Lösungsmitteln wie beispielsweise Eisessig, Wasser, Methanol, Ethanol, 2-Propanol DCM oder THF oder in Gemischen dieser Lösungsmittel erfolgen.

Die Umsetzung eines entsprechenden halogenierten Thiols Y-$CH_2$-$CH_2$-A-$R^3$ (mit A = S), Sulfoxids Y-$CH_2$-$CH_2$-A-$R^3$ (mit A = SO) oder Sulfons Y-$CH_2$-$CH_2$-A-$R^3$ (mit A = $SO_2$) mit dem Mercaptonicotinsäureamid kann sowohl mit Iodiden, Bromiden oder Chloriden in Gegenwart von Basen, wie beispielsweise Kaliumcarbonat, KOH, NaOH, Triethylamin, Diisopropylethylamin, Natriummethanolat oder -ethanolat oder Natriumacetat in Lösungsmitteln wie beispielsweise Diethylether, THF, DMF, Aceton, Acetonitril, DCM, Wasser, Ethanol oder Methanol durchgeführt werden.

Der Thioether kann auch durch Reaktion des Mercaptonicotinamids mit einem entsprechenden Alkohol HO-$CH_2$-$CH_2$-A-$R^3$ (mit A = S, SO, $SO_2$) unter Verwendung von Reagenzien wie beispielsweise Schwefelsäure, Phosphorsäure, Perchlorsäure, Acetanhydrid oder Zirconiumtetrachlorid gebildet werden. Neben diesen sauren Reagenzien können aber auch Basen, wie beispielsweise Natriumhydrid verwendet werden. Als weitere Kupplungsreagenzien eignen sich aber auch (N-Methyl-N-phenylamino)triphenylphosphoniumiodid, Phenylmethansulfonat, Hexamethylphosphorsäuretriamid oder 1-Pentyl-3-methylimidazoliumbromid. Die genannten Reagenzien können sowohl einzeln als auch in Kombinationen verwendet werden. Als Lösungsmittel eignen sich beispielsweise Wasser, Diethylether, Essigsäure oder DMF.

Den entsprechenden Alkohol HO-$CH_2$-$CH_2$-A-$R^3$ (mit A = SO) erhält man durch Oxidation des entsprechenden Thioethers HO-$CH_2$-$CH_2$-A-$R^3$ (mit A = S) mit Oxidationsmitteln wie beispielsweise $H_2O_2$, $NaIO_4$, $NaClO_2$, m-Chlorperbenzoesäure oder Oxon in Lösungsmitteln wie beispielsweise Eisessig, Wasser, Methanol, Ethanol, 2-Propanol DCM oder THF oder in Gemischen dieser Lösungsmittel erfolgen.

**Weg B (A = S, SO, $SO_2$)**

Der Mercaptonicotinsäure-Thioether kann durch Reaktion der Mercaptonicotinsäure mit einem entsprechenden Alkohol HO-$CH_2$-$CH_2$-A-$R^3$ (mit A = S, SO, $SO_2$) unter Verwendung von Reagenzien wie beispielsweise Schwefelsäure, Phosphorsäure, Perchlorsäure, Acetanhydrid oder Zirconiumtetrachlorid gebildet werden. Neben diesen sauren Reagenzien können aber auch Basen, wie beispielsweise Natriumhydrid verwendet werden. Als weitere Kupplungsreagenzien eignen sich aber auch (N-Methyl-N-phenylamino)triphenylphosphoniumiodid, Phenylmethansulfonat, Hexamethylphosphorsäuretriamid oder 1-Pentyl-3-methylimidazoliumbromid. Die genannten Reagenzien können sowohl einzeln als auch in Kombinationen verwendet werden. Als Lösungsmittel eignen sich beispielsweise Wasser, Diethylether, Essigsäure oder DMF.

Die Umsetzung eines entsprechenden halogenierten Verbindung HO-$CH_2$-$CH_2$-A-$R^3$ (mit A = S, SO, $SO_2$, Y = Cl, Br, I) mit der Mercaptonicotinsäure kann sowohl mit Iodiden, Bromiden oder Chloriden in Gegenwart von Basen, wie beispielsweise Kaliumcarbonat, KOH, NaOH, Triethylamin, Diisopropylethylamin, Natriummethanolat oder -ethanolat oder Natriumacetat in Lösungsmitteln wie beispielsweise Diethylether, THF, DMF, Aceton, Acetonitril, DCM, Wasser, Ethanol oder Methanol durchgeführt werden.

Die abschließende Acylierung unter Verwendung von Basen und gegebenenfalls Kupplungsreagenzien kann in Lösungsmitteln, wie beispielsweise Methanol, DMF oder DCM durchgeführt werden. Als Basen können beispielsweise Natriummethanolat, Triethylamin, Diisopropylethylamin oder N-Methylmorpholin verwendet werden. Als Kupplungsreagenzien eignen sich beispielsweise EDCI, HOBt, DCC, CDI, HBTU, DMAP oder Pentafluorphenyldiphenylphosphinat. Die Reaktionszeit kann zwischen 1 h und 3 d variieren.

Die Carbonsäure kann aber auch zunächst in das Carbonsäurechlorid überführt werden. Hierzu eignen sich beispielsweise Reagenzien wie $COCl_2$, $PCl_3$, $POCl_3$, $P_2O_5$, $SOCl_2$ oder $SiCl_4$ in Lösungsmitteln wie beispielsweise Pyridin, DCM, DMF oder Toluol.

**Weg C (A = S, $SO_2$)**

Die einleitende Acylierungsreaktion von Aminen mit Hilfe von Carbonsäuren, hier die halogenierte Nicotinsäure, kann unter Verwendung von Basen und gegebenenfalls Kupplungsreagenzien kann in Lösungsmitteln, wie beispielsweise Methanol, DMF oder DCM durchgeführt werden. Als Basen können beispielsweise Natriummethanolat, Triethylamin, Diisopropylethylamin oder N-Methylmorpholin verwendet werden. Als Kupplungsreagenzien eignen sich beispielsweise EDCI, HOBt, DCC, CDI, HBTU, DMAP oder Pentafluorphenyldiphenylphosphinat. Die Reaktionszeit kann zwischen 1 h und 3 d variieren.

Die Carbonsäure kann aber auch zunächst in das Carbonsäurechlorid überführt werden. Hierzu eignen sich beispielsweise Reagenzien wie $COCl_2$, $PCl_3$, $POCl_3$, $P_2O_5$, $SOCl_2$ oder $SiCl_4$ in Lösungsmitteln wie beispielsweise Pyridin, DCM, DMF oder Toluol.

Die anschließende Substitutionsreaktion mit den entsprechenden Thiolen HS-$CH_2$-$CH_2$-A-$R^3$ oder Thiolaten $^-$S-$CH_2$-$CH_2$-A-$R^3$ eignen sich sowohl die Chlor- als auch an Bromderivate (X = Cl, Br) der Nicotinsäure.

Die Substitution kann in Lösungsmitteln wie beispielsweise Methanol, Ethanol, 2-Propanol, 2-Methyl-2-propanol, Benzol, Toluol, THF, Dioxan, Acetonitril, Chloroform, DMF, DMSO oder Mischungen der Lösungsmittel verwendet werden.

Als Basen zur Erzeugung des Thiolats eignen sich beispielsweise KOH, NaOH, Kaliumcarbonat, Natriummethanolat, Natriumethanolat, Kalium-tert.-Butylat, Triethylamin, Natriumhydrid, aber auch beispielsweise Natrium. Als Additive können beispielsweise Verbindungen wie Natriumiodid, Tetrabutylammoniumbromid, -chlorid oder -hydrogensulfat oder HMPT verwendet werden.

**Weg D (A = S, $SO_2$)**

Für die Substitutionsreaktion mit Thiolen HS-$CH_2$-$CH_2$-A-$R^3$ oder Thiolaten -S-$CH_2$-$CH_2$-A-$R^3$ eignen sich sowohl die Chlor- als auch an Bromderivate der Nicotinsäure (X = Cl, Br).

Die Substitution kann in Lösungsmitteln wie beispielsweise Methanol, Ethanol, 2-Propanol, 2-Methyl-2-propanol, Benzol, Toluol, THF, Dioxan, Acetonitril, Chloroform, DMF, DMSO oder Mischungen der Lösungsmittel verwendet werden.

Als Basen zur Erzeugung des Thiolats eignen sich beispielsweise KOH, NaOH, Kaliumcarbonat, Natriummethanolat, Natriumethanolat, Kalium-tert.-Butylat, Triethylamin, Natriumhydrid, aber auch beispielsweise Natrium. Als Additive können beispielsweise Verbindungen wie Natriumiodid, Tetrabutylammoniumbromid, -chlorid oder -hydrogensulfat oder HMPT verwendet werden.

Die anschließende Acylierungsreaktion von Aminen mit Hilfe von Carbonsäuren unter Verwendung von Basen und gegebenenfalls Kupplungsreagenzien kann in Lösungsmitteln, wie beispielsweise Methanol, DMF oder DCM durchgeführt werden. Als Basen können beispielsweise Natriummethanolat, Triethylamin, Diisopropylethylamin oder N-Methylmorpholin verwendet werden. Als Kupplungsreagenzien eignen sich beispielsweise EDCI, HOBt, DCC, CDI, HBTU, DMAP oder Pentafluorphenyldiphenylphosphinat. Die Reaktionszeit kann zwischen 1 h und 3 d variieren.

Die Carbonsäure kann aber auch zunächst in das Carbonsäurechlorid überführt werden. Hierzu eignen sich beispielsweise Reagenzien wie $COCl_2$, $PCl_3$, $POCl_3$, $P_2O_5$, $SOCl_2$ oder $SiCl_4$ in Lösungsmitteln wie beispielsweise Pyridin, DCM, DMF oder Toluol.

Die vorstehend beschriebenen Umsetzungen können ferner jeweils unter üblichen, dem Fachmann geläufigen Bedingungen, beispielsweise in Hinblick auf Druck, Temperatur, Schutzgasatmosphäre oder Reihenfolge der Zugabe der Komponenten durchgeführt werden. Ggf. kann die unter den jeweiligen Bedingungen optimale Verfahrensführung vom Fachmann durch einfache Vorversuche ermittelt werden.

Sämtliche der vorstehend beschriebenen Verfahrensschritte sowie jeweils auch die Reinigung und/oder Isolierung von Zwischen- oder Endprodukten können teilweise oder vollständig unter einer Inertgasatmosphäre, vorzugsweise unter Stickstoffatmosphäre oder Argonatmosphäre, durchgeführt werden.

Die erfindungsgemäßen substituierten Nicotinamid-Verbindungen können sowohl in Form ihrer freien Basen, ihrer freien Säuren wie auch jeweils in Form entsprechender Salze, insbesondere physiologisch verträglicher Salze, isoliert werden.

Die freien Basen der jeweiligen erfindungsgemäßen substituierten Nicotinamid - Verbindungen können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Maleinsäure, Äpfelsäure, Bernsteinsäure, Weinsäure, Mandelsäure, Fumarsäure, Milchsäure, Zitronensäure, Glutaminsäure oder Asparaginsäure, in die entsprechenden Salze, vorzugsweise physiologisch verträglichen Salze, überführt werden.

Die freien Basen der jeweiligen erfindungsgemäßen substituierten Nicotinamid-Verbindungen können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der erfindungsgemäßen substituierten Nicotinamid-Verbindungen durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze $[NH_xR_{4-x}]^+$, worin x = 0, 1, 2, 3 oder 4 ist und R für einen linearen oder verzweigten $C_{1-4}$-Alkyl-Rest steht, genannt.

Die erfindungsgemäßen substituierten Nicotinamid-Verbindungen können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen substituierten Nicotinamid-Verbindungen nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden. Neben wenigstens einer erfindungsgemäßen substituierten Nicotinamid-Verbindung enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt

davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden substituierten Nicotinamid-Verbindungen können in einem Depot, in gelöster Form oder in einem Pflaster, gegebenenfalls unter Zusatz von die Hautpenetration fördernden Mitteln, als geeignete perkutane Applikationszubereitungen vorliegen.

Oral oder perkutan anwendbare Zubereitungsformen können die jeweilige erfindungsgemäße substituierte Nicotinamid-Verbindung auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remingtons Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge der jeweiligen erfindungsgemäßen substituierten Nicotinamid-Verbindung kann variieren und ist beispielsweise abhängig vom Gewicht oder Alter des Patienten sowie von der Applikationsart, der Indikation und dem Schweregrad der Erkrankung. Üblicherweise werden 0,005 bis 100 mg/kg, vorzugsweise 0,05 bis 75 mg/kg Körpergewicht des Patienten wenigstens einer solchen erfindungsgemäßen Verbindung appliziert.

Im Folgenden wird die Erfindung mit Hilfe einiger Beispiele erläutert. Diese dienen der Erläuterung der Erfindung und sind nicht einschränkend auszulegen.

**Synthese der Beispielverbindungen**

**<u>Synthesebeschreibung der Vorstufen</u>**

**Synthese von 2-Mercapto-N-(thiophen-2-ylmethyl)nicotinamid (Vorstufe V1)**

Eine Suspension aus 8,0 g (51,5 mmol) 2-Mercaptonicotinsäure, 5,8 g (51,5 mmol) 2-(Aminomethyl)-thiophen und 3,5 g (25,8 mmol) Phosphortrichlorid in Chlorbenzol (260 ml) wurde 3 h unter Rückfluss (145 °C) erhitzt. Nachdem sich die Reaktionslösung auf 60 °C abgekühlt hatte, wurde bei dieser Temperatur abgesaugt. Der erhaltene Feststoff wurde mit einer DCM / MeOH-Mischung (3:1, vv, 300 ml) aufgenommen und mit Wasser (2 x 50 ml) gewaschen. Die organische Phase wurde über $MgSO_4$ getrocknet, filtriert und im Vakuum eingeengt. Durch Kristallisation des Rückstandes aus Ethylacetat wurden 3,1 g (12,4 mmol, 24%) 2-Mercapto-N-(thiophen-2-ylmethyl)nicotinamid erhalten.
1H NMR (400 MHz, DMSO-$d_6$) d ppm 4,73 (d, *J* = 5,52 Hz, 2 H) 6,97 (dd, *J* = 5,02, 3,51 Hz, 1 H) 7,01 - 7,11 (m, 2 H) 7,41 (dd, *J* = 5,27, 1,25 Hz, 1 H) 7,98 (td, *J* = 6,27, 2,01 Hz, 1 H) 8,54 (dd, *J* = 7,53, 2,01 Hz, 1 H) 11,28 (t, *J*= 5,52 Hz, 1 H) 14,06 (br. s., 1 H)

**Vorstufen V2 und V3:**

**Synthese von (2-Bromethyl)(cyclohexyl)sulfan (Vorstufe V2)**

Zu einer Lösung von 3,7 ml (30,0 mmol) Cyclohexanthiol in DMF (46 ml) wurden 19,4 ml (225,0 mnol) 1,2-Dibromethan und 4,1 g (30,0 mmol) $K_2CO_3$ gegeben. Nach 2 h Rühren bei RT wurde mit Diethylether (200 ml) verdünnt und mit gesättigter, wässriger NaCl-Lösung gewaschen. Die organische Phase wurde über $MgSO_4$ getrocknet, filtriert und im Vakuum eingeengt. Die erhaltenen 6,1 g an Rohprodukt (2-Bromethyl)(cyclohexyl)sulfan wurden ohne weitere Aufreinigung zur weiteren Reaktion eingesetzt.

**Synthese von (2-Bromethyl)(3-(trifluormethyl)phenyl)sulfan (Vorstufe V3)**

Nach dem für Vorstufe **V2** beschrieben Verfahren wurden 10,5 g (58,9 mmol) 3-Trifluormethyl-thiophenol in 19,3 g Rohprodukt (2-Bromethyl)(3-(trifluormethyl)-phenyl)sulfan überführt, das ohne weitere Aufreinigung zur weiteren Reaktion eingesetzt wurde.

**Synthese von 2-Chlor-6-methyl-N-(thiophen-2-ylmethyl)nicotinamid (Vorstufe V4)**

Eine Lösung von 1,0 g (5,8 mmol) 2-Chlor-6-methyl-nicotinsäure in DMF (20 ml) wurde bei 0 °C mit 2,67 g (7,0

mmol) HATU und 4,0 ml (23,2 mmol) DIPEA versetzt und 20 min bei 0 °C gerührt. Bei dieser Temperatur wurden 656 mg (5,8 mmol) Thiophen-2-ylmethylamin zugegeben. Anschließend wurde 16 h bei RT gerührt. Dann wurde mit EE verdünnt und nacheinander mit einer ges. aq. $NaHCO_3$-Lösung und Brine gewaschen. Die organische Phase wurde über $Na_2SO_4$ getrocknet, filtriert und in Vakuum eingeengt. Nach SC (Hexan /EE 4:1) des Rückstands wurden 966 mg (3,6 mmol, 63%) 2-Chlor-6-methyl-N-(thiophen-2-ylmethyl)nicotinamid erhalten.

**Synthese von (2-Chlorethyl)(3,4-difluorphenyl)sulfan (Vorstufe V9)**

Eine Lösung von 2,0 g (13,7 mmol) 3,4-Difluor-thiophenol in DMF (20 ml) wurde mit 5,7 ml (68,4 mmol) 1-Brom-2-chlor-ethan und 1,9 g (13,7 mmol) $K_2CO_3$ versetzt. Anschließend wurde 5 h bei 60 °C und 16 h bei RT gerührt. Dann wurde mit EE (50 ml) verdünnt und nacheinander mit einer 1 M aq. $Na_2CO_3$-Lösung und Brine gewaschen. Die organische Phase wurde über $MgSO_4$ getrocknet, filtriert und in Vakuum eingeengt. Als Rückstand wurden 2,7 g (12,9 mmol, 95%) (2-Chlorethyl)-(3,4-difluorphenyl)sulfan erhalten, das ohne zusätzliche Reinigung weiter umgesetzt wurde.

**Synthese von 4-(2-Chlorethylsulfonyl)-1,2-difluorbenzol (Vorstufe V10)**

Zu einer Lösung von 1,04 g (5,0 mmol) (2-Chlorethyl)-(3,4-difluorphenyl)sulfan in DCM (10 ml) wurden bei 5-10°C eine Lösung von 3,06 g (12,5 mmol) m-Chlorper-benzoesäure in DCM (10 ml) getropft. Anschließend wurde 150 min bei 10 °C gerührt. Dann wurde je zweimal mit einer 1 M aq. $NaHCO_3$-Lösung und einer ges. aq. $Na_2SO_3$-Lösung gewaschen. Die organische Phase wurde über $MgSO_4$ getrocknet, filtriert und in Vakuum eingeengt. Als Rückstand wurden 1,2 g (4,94 mmol, 99%) 4-(2-Chlorethylsulfonyl)-1,2-difluorbenzol erhalten, das ohne zusätzliche Reinigung weiter umgesetzt wurde.

**Synthese von (2-Chlorethylsulfinyl)benzol (Vorstufe V11)**

Zu einer Lösung von 1,30 g (7,5 mmol) (2-Chlorethylsulfinyl)benzol in DCM (18 ml) wurden bei 5-10°C eine Lösung von 1,67 g (7,5 mmol) m-Chlorperbenzoesäure in DCM (10 ml) getropft. Anschließend wurde 120 min bei 10 °C gerührt. Dann wurde je zweimal mit einer 1 M aq. $NaHCO_3$-Lösung und einmal mit Brine gewaschen. Die organische Phase wurde über $MgSO_4$ getrocknet, filtriert und in Vakuum eingeengt. Als Rückstand wurden 1,34 g (7,1 mmol, 95%) (2-Chlorethylsulfinyl)benzol erhalten, das ohne zusätzliche Reinigung weiter umgesetzt wurde.

**Synthese von 1-(2-Chloroethylsulfonyl)-4-ethylbenzol (Vorstufe V12)**

Zu einer Lösung von 1,0 g (4,67 mmol) 2-(4-Ethyl-phenyl-sulfonyl)-ethanol und 56 μl (0,70 mmol) Pyridin in Toluol (20 ml) wurde eine Lösung von 1,0 ml (14,0 mmol) Thionylchlorid in Toluol (15 ml) unter Eiskühlung getropft. Anschließend wurde 3 h unter Rückfluss erhitzt. Dann wurde mit Eis und Wasser gequencht. Die Phasen wurden getrennt und die wässrige Phase wurde zweimal mit DCM extrahiert. Die vereinten organischen Phasen wurden über $MgSO_4$ getrocknet, filtriert und in Vakuum eingeengt. Als Rohprodukt wurden 1,19 gleicht verunreinigtes 1-(2-Chloroethylsulfonyl)-4-ethylbenzol erhalten, das ohne zusätzliche Reinigung weiter umgesetzt wurde.

Weitere Vorstufen wurden analog den beschriebenen Verfahren hergestellt. In Tabelle T1 ist zusammengefasst, welche Vorstufen analog welchem Verfahren hergestellt wurden. Dem Fachmann ist dabei ersichtlich, welche Ausgangsstoffe jeweils eingesetzt wurden.

**Tabelle T1**

| Vorstufe | Name | Synthese analog Vorstufe |
|---|---|---|
| **V5** | 2,6-Difluor-N-(thiophen-2-ylmethyl)-nicotinamid | V4 |
| **V6** | 2-Chlor-N-(thiophen-2-ylmethyl)-6-(trifluormethyl)nicotinamid | V4 |
| **V7** | 2-Chlor-N-(thiophen-2-ylmethyl)-5-(trifluormethyl)nicotinamid | V4 |
| **V8** | 2-Chlor-5-fluor-N-(thiophen-2-ylmethyl)nicotinamid | V4 |
| **V13** | (2-Chlorethyl)(4-fluorphenyl)sulfan | V9 |
| **V14** | (2-Chlorethyl)(2-trifluormethyl-phenyl)sulfan | V9 |
| **V15** | (2-Chlorethyl)(3-trifluormethyl-phenyl)sulfan | V9 |
| **V16** | (2-Chlorethyl)(4-trifluormethyl-phenyl)sulfan | V9 |

(fortgesetzt)

| Vorstufe | Name | Synthese analog Vorstufe |
|---|---|---|
| **V17** | (2-Chlorethyl)(3-trifluormethoxy-phenyl)sulfan | V9 |
| **V18** | (2-Chlorethyl)(4-trifluormethoxy-phenyl)sulfan | V9 |
| **V19** | (2-Chlorethyl)(2-methyl-phenyl)sulfan | V9 |
| **V20** | (2-Chlorethyl)(3-methyl-phenyl)suifan | V9 |
| **V21** | (2-Chlorethyl)(2,4-difluorphenyl)sulfan | V9 |
| **V22** | 1-(2-Chlorethylsulfinyl)-3-(trifluormethoxy)benzol | V11 |
| **V23** | 1-(2-Chloroethylsulfonyl)-2-fluor-benzol | V12 |
| **V24** | 1-(2-Chloroethylsulfonyl)-2-trifluormethyl-benzol | V10 |
| **V25** | 1-(2-Chloroethylsulfonyl)-4-trifluormethyl-benzol | V10 |
| **V26** | 1-(2-Chloroethylsulfonyl)-3-trifluormethoxy-benzol | V10 |
| **V27** | 1-(2-Chloroethylsulfonyl)-4-trifluormethoxy-benzol | V10 |
| **V28** | 1-(2-Chloroethylsulfonyl)-3-methyl-benzol | V10 |
| **V29** | 1-(2-Chloroethylsulfonyl)-3-methoxy-benzol | V12 |
| **V30** | 1-(2-Chloroethylsulfonyl)-4-methoxy-benzol | V12 |
| **V31** | 1-(2-Chloroethylsulfonyl)-2,4-difluor-benzol | V10 |
| **V32** | 1-(2-Chloroethylsulfonyl)-3,5-ditrifluormethyl-benzol | V12 |

**Eingesetzte Amine (Tabelle T2)**

Für die Synthese der Beispiele wurden die folgenden Amine verwendet:

**Tabelle T2**

| | |
|---|---|
| **A01** | Phenylmethanamin |
| **A02** | N-Methyl-1-phenylmethanamin |
| **A03** | Pyridin-2-ylmethanamin |
| **A04** | Pyridin-4-ylmethanamin |
| **A05** | Thiophen-2-ylmethanamin |
| **A06** | 2-(3-Fluorphenyl)ethanamin |
| **A07** | N-Methyl-1-m-tolylmethanamin |
| **A08** | Furan-2-ylmethanamin |
| **A09** | p-Tolylmethanamin |
| **A10** | (2-(Trifluormethyl)phenyl)methanamin |
| **A11** | Pyridin-3-ylmethanamin |
| **A12** | (3,5-Difluorphenyl)methanamin |
| **A13** | N-Methyl-2-phenylethanamin |
| **A14** | 1-(3-Methoxyphenyl)-N-methylmethanamin |
| **A15** | (2-Fluorphenyl)methanamin |
| **A16** | m-Tolylmethanamin |
| **A17** | (3,4-Difluorphenyl)methanamin |

(fortgesetzt)

| | |
|---|---|
| **A18** | 1-(3-Bromphenyl)-N-methylmethanamin |
| **A19** | (2-Methoxyphenyl)methanamin |
| **A20** | (3-Fluorphenyl)methanamin |
| **A21** | 1-(Furan-2-yl)-N-methylmethanamin |
| **A22** | (4-Methoxyphenyl)methanamin |
| **A23** | (2-Chlorphenyl)methanamin |
| **A24** | (3,4-Dichlorphenyl)methanamin |
| **A25** | (4-Fluorphenyl)methanamin |
| **A26** | 2-(2-Methoxyphenyl)ethanamin |
| **A27** | (2,6-Difluorphenyl)methanamin |
| **A28** | o-Tolylmethanamin |
| **A29** | (3,5-Dimethoxyphenyl)methanamin |
| **A30** | (3-Chlorphenyl)methanamin |
| **A31** | (2,4-Dichlorphenyl)methanamin |
| **A32** | (3-(Trifluormethyl)phenyl)methanamin |
| **A43** | (5-Methylfuran-2-yl)methanamin |
| **A44** | (4-Chlorphenyl)methanamin |
| **A45** | (2,3-Dichlorphenyl)methanamin |
| **A55** | 1-(4-Bromphenyl)-N-methylmethanamin |
| **A64** | 1-(1,3-Dioxolan-2-yl)-N-methylmethanamin |
| **A66** | Cyclopentylmethanamin |
| **A67** | Cyclobutylmethanamin |
| **A68** | (1,4-Dioxan-2-yl)methanamin |
| **A69** | (4-(Pyridin-2-yloxy)phenyl)methanamin |
| **A70** | 2-Methylbutan-1-amin |
| **A71** | 2-Ethylbutan-1-amin |
| **A72** | 2-Methylpropan-1-amin |
| **A73** | Cyclopropylmethanamin |
| **A74** | 3-(2-Methoxyethoxy)propan-1-amin |
| **A75** | 1-(3,4-Dimethylphenyl)ethanamin |
| **A76** | Cyclohexylmethanamin |
| **A77** | Methanamin |
| **A78** | 1-(3-(Trifluormethyl)phenyl)ethanamin |
| **A79** | 2-Cyclohexylethanamin |
| **A80** | 2,2-Dimethylpropan-1-amin |
| **A81** | (Tetrahydro-2H-pyran-4-yl)methanamin |
| **A82** | (4-(Trifluormethylthio)phenyl)methanamin |
| **A83** | (S)-1-Cyclohexylethanamin |
| **A84** | 1-(3,5-Dimethylphenyl)ethanamin |

(fortgesetzt)

| | |
|---|---|
| **A85** | 1-(Thiophen-2-yl)ethanamin |
| **A86** | (3,5-Dimethylphenyl)methanamin |
| **A87** | (R)-1-Cyclohexylethanamin |
| **A88** | 3-(1H-Pyrazol-1-yl)phenyl)methanamin |
| **A89** | (2,3-Dihydrobenzofuran-5-yl)methanamin |
| **A90** | (4-Phenoxyphenyl)methanamin |
| **A91** | 6,6-Dimethylbicyclo[3.1.1]heptan-2-yl)methanamin |
| **A92** | 1-(4-(Trifluormethylthio)phenyl)ethanamin |
| **A93** | (Tetrahydro-2H-pyran-2-yl)methanamin |
| **A94** | (5-Methylthiophen-2-yl)methanamin |
| **A95** | (4-Methylthiophen-2-yl)methanamin |
| **A96** | 1-Adamantylmethanamin |
| **A97** | (3-Morpholinophenyl)methanamin |
| **A98** | 3,3-Dimethylbutan-1-amin |
| **A99** | Benzo[b]thiophen-2-ylmethanamin |
| **A100** | (5-Chlorothiophen-2-yl)methanamin |
| **A101** | (Tetrahydro-2H-thiopyran-4-yl)methanamin |
| **A102** | 2-(Thiophen-2-yl)ethanamin |
| **A103** | 2-Phenylethanamin |
| **A104** | 3-Phenylpropan-1-amin |
| **A105** | 3-Methylbutan-1-amin |
| **A106** | 2-Cyclopropylethanamin |
| **A107** | 2-Cyclopentylethanamin |
| **A108** | Cycloheptylmethanamin |
| **A109** | (2-Methylcyclohexyl)methanamin |
| **A110** | (4-Methylcyclohexyl)methanamin |

Die genannten Amine sind entweder kommerziell bei Anbietern wie ABCR, ACBBlocks, Acros, Aldrich, Array Biopharma, BASF, Fulcrum Scientific, Indofine, Interchim, Lancaster, Matrix, Maybridge, Rare Chemicals oder Synchem erhältlich oder wurden wie im Falle von **A75**, **A82**, **A84** und **A92** synthetisiert.

**Synthese von 1-(3,4-Dimethylphenyl)ethylamin (A75)**

Eine Lösung von 4,46 g (30,0 mmol) 3,4-Dimethylacetophenon in einer 2 M ethanolischen Ammoniaklösung (75 ml) wurde mit 16,4 ml (150,0 mmol) Tetrapropylorthotitanat versetzt und 6 h bei RT gerührt. Anschließend wurden 1,7 g (45,0 mmol) Natriumborhydrid zugegeben und es wurde weitere 16 h bei RT gerührt. Danach wurde die Reaktionslösung in eine ges. aq. Ammoniaklösung (75 ml) gegossen. Der entstandene Niederschlag wurde abgesaugt und mit es wurde mit Ethylacetat nachgewaschen. Das wässrige Filtrat wurde im Vakuum aufkonzentriert und anschließend wurde zweimal mit Ethylacetat extrahiert. Die zusammengeführten Ethylacetat-Phasen wurden dreimal mit einer 2 M Salzsäure extrahiert. Die vereinten wässrigen Phasen wurden mit einer 2 M aq. NaOH-Lösung auf pH 11 eingestellt und anschließend dreimal mit Ethylacetat extrahiert. Die vereinten organischen Phasen wurden über $MgSO_4$ getrocknet, filtriert und im Vakuum eingeengt. Durch SC (Ethylacetat /MeOH 9:1) wurden 799 mg (5,4 mmol, 18%) 1-(3,4-Dimethylphenyl)ethylamin erhalten.
1H NMR (400 MHz, DMSO-$d_6$) d ppm 1,20 (d, *J* = 6,6 Hz, 3 H) 2,17 (s, 3 H) 2,19 (s, 3 H) 3,89 (q, *J* = 6,6 Hz, 1 H) 6,99 - 7,07 (m, 2 H) 7,08 - 7,15 (m, 1 H)

**Synthese von 4-(Trifluormethylthio)phenyl)methylamin (A82)**

Nach dem für Vorstufe **A75** beschrieben Verfahren wurden 5,0 g (24,2 mmol) 4-(Trifluormethylthio)-benzaldehyd in 64 mg (0,31 mmol, 1 %) 4-(Trifluormethylthio)-phenyl)methylamin überführt.
1H NMR (400 MHz, DMSO-$d_6$) d ppm 3.77 (s, 2 H) 7.50 (d, *J* = 8.03 Hz, 2 H) 7.65 (d, *J* = 8.03 Hz, 2 H)

**Synthese von 1-(3,5-Dimethylphenyl)ethylamin (A84)**

Nach dem für Vorstufe **A75** beschrieben Verfahren wurden 2,17 g (14,6 mmol) 3,5-Dimethalacetophenon in 1,08 g (7,2 mmol, 50%) 1-(3,5-Dimethylphenyl)ethylamin überführt. 1H NMR (400 MHz, DMSO-$d_6$) d ppm 1,20 (d, *J* = 7,0 Hz, 3 H) 2,23 (s, 6 H) 3,88 (q, *J* = 7,0 Hz, 1 H) 6,77 - 6,83 (m, 1 H) 6,91 - 7,00 (m, 2 H)

**Synthese von 1-(4-(Trifluormethylthio)phenyl)ethanamin (A92)**

Nach dem für Vorstufe **A75** beschrieben Verfahren wurden 4,4 g (20,0 mmol) 4'-(Trifluormethylthio)acetophenon in 1,78 g (8,0 mmol, 40%) 1-(4-(Trifluormethylthio)-phenyl)ethanamin überführt.
1H NMR (400 MHz, DMSO-$d_6$) d ppm 1,24 (d, *J*=6,6 Hz, 3 H) 4,03 (q, *J*=6,6 Hz, 1 H) 7,54 (d, *J*=8,53 Hz, 2 H) 7,64 (d, *J*=8,03 Hz, 2 H)

<u>**Eingesetzte Säuren**</u>

Die 2-(2-(Phenylsulfonyl)ethylthio)nicotinsäure **S1** ist kommerziell bei den Anbietern Alfa Aesar und ABCR erhältlich.

**Synthese von 2-(2-Tosylethylthio)nicotinsäure (Säure S2)**

Eine Lösung aus 7,3g (47 mmol) 2-Mercaptonicotinsäure und 9,6g (48 mmol) 2-(p-Toluolsulfonyl)-ethanol wurden in DMF (80 ml) gelöst. Anschließend wurde vorsichtig 0,5 ml konz.$H_2SO_4$ zugetropft und unter Rückfluß über Nacht gerührt. Der Reaktionsansatz wurde in der Genevac (EZ2) aufkonzentriert. Der Rückstand wurde in Acetonitril gelöst und der Feststoff abgetrennt. Die Mutterlauge wurde aufkonzentriert und mit MeOH versetzt. Der ausgefallene Feststoff wurde abfiltriert und getrocknet. Als Feststoff wurden 2,35g (7 mmol, 14,8%) 2-(2-Tosylethylthio)nicotinsäure erhalten.
1H NMR (400 MHz, $CDCl_3$) d ppm 2,48 (s, 3 H) 3,31 - 3,44 (m, 2 H) 3,48 - 3,59 (m, 2 H) 7,09 (dd, *J*=7,78, 4,77 Hz, 1 H) 7,40 (d, *J*=8,03 Hz, 2 H) 7,85 (d, *J=8,53* Hz, 2 H) 8,27 (dd, *J*=7,78, 1,76 Hz, 1 H) 8,40 (dd, *J*=4,52, 2,01 Hz, 1 H)

**Synthese von 2-(2-(3-(Trifluormethyl)phenylsulfonyl)-ethylthio)nicotinsäure (Säure S3)**

Eine Suspension von 349 mg (2,25 mmol) 2-Mercaptonicotinsäure in DMF (5 ml) wurde mit 683 mg (4,95 mmol) $K_2CO_3$ versetzt und 30 min bei RT gerührt. Anschließend wurden 614 mg (2,25 mmol) 2-Chlorethyl-(3-(trifluormethyl) phenyl)-sulfon zugegeben und es wurde weitere 72 h bei RT gerührt. Die Reaktionslösung wurde im Vakuum eingeengt und der erhaltene Rückstand wurde mit Ethylacetat aufgenommen und mit Wasser versetzt. Mit einer 2 M Salzsäure wurde pH 3 eingestellt und die Phasen wurden getrennt. Die wässrige Phase wurde mit Ethylacetat extrahiert. Die vereinten organischen Phasen wurden über $MgSO_4$ getrocknet, filtriert und im Vakuum eingeengt. Als Rückstand wurden 778 mg (1,99 mmol, 88%) 2-(2-(3-(Trifluormethyl)phenylsulfonyl)ethylthio)nicotinsäure erhalten.
1H NMR (400 MHz, DMSO-$d_6$) d ppm 3,20 - 3,29 (m, 2 H) 3,72 - 3,86 (m, 2 H) 7,23 (dd, *J* = 7,78, 4,77 Hz, 1 H) 7,96 (t, *J* = 8,03 Hz, 1 H) 8,15 - 8,22 (m, 2 H) 8,23 - 8,32 (m, 2 H) 8,34 (dd, *J* = 4,77, 1,76 Hz, 1 H).

**Synthese von 5-Methyl-2-(2-(phenylsulfonyl)ethylthio)nicotinsäure (Säure S4)**

a) Synthese von 2-Mercapto-5-methylnicotinnitril

Eine Lösung von 1,7 g (20 mmol) 2-Cyano-thioacetamid und 2,3 g (20,0 mmol) 3-Ethoxymethacrolein in EtOH (50 ml) wurde mit einer katalytischen Menge (16 µl) 2-(Dimethylamino)-ethanol versetzt und 24 h unter Rückfluss erhitzt. Anschließend wurde im Vakuum weitgehend eingeengt. Der enstandene Niederschlag wurde abfiltriert und mit kaltem Ethanol gewaschen. Dabei wurden 1,45 g (9,6 mmol, 48%) 2-Mercapto-5-methylnicotinnitril erhalten, das ohne zusätzliche Reinigung weiter umgesetzt wurde.

b) Synthese von 5-Methyl-2-(2-(phenylsulfonyl)ethylthio)nicotinnitril

Zu einer Lösung von 1,40 g (9,3 mmol) 2-Mercapto-5-methylnicotinnitril in Aceton (30 ml) wurden 1,9 g (14,0 mmol) $K_2CO_3$ und 1,9 g (9,3 mmol) (2-Chlorethylsulfonyl)-benzol gegeben und anschließend wurde 16 h bei 60 °C gerührt. Dann wurde abfiltriert und das Filtrat wurde im Vakuum eingeengt. Der Rückstand wurde mit Wasser aufgenommen und mit EE extrahiert (3 x 50 ml). Die vereinten organischen Phasen wurden mit Brine gewaschen, über $Na_2SO_4$ getrocknet, filtriert und im Vakuum eingeengt. Nach SC (Hexan / EE 4:1) des Rückstands wurden 1,41 g (4,4 mmol, 47%) 5-Methyl-2-(2-(phenylsulfonyl)ethylthio)nicotinnitril erhalten.

c) Synthese von 5-Methyl-2-(2-(phenylsulfonyl)ethylthio)nicotinsäure

Eine Lösung von 1,40 g (4,4 mmol) 5-Methyl-2-(2-(phenylsulfonyl)ethylthio)-nicotin-nitril in 50%-iger aq. Schwefelsäure (10 ml) wurde 4 d unter Rückfluss erhitzt. Anschließend wurde auf Eiswasser gegossen und der enstandene Niederschlag wurde abfiltriert. Es wurde mit kaltem Wasser nachgewaschen. Als Rückstand wurden 1,2 g (3,6 mmol, 81%) 5-Methyl-2-(2-(phenylsulfonyl)ethylthio)nicotinsäure erhalten, die ohne zusätzliche Reinigung weiter umgesetzt wurde.

**Synthesebeschreibung der Beispiele**

**Beispiele 1 - 52:**

Es wurden zunächst bei Raumtemperatur 100 μmol der entsprechenden Säure-Lösung (0,05 M in DCM, 2 ml) in ein Reaktionsgefäß (Heidolph) gegeben, mit 105 μmol CDI-Lösung (0,105 M in DCM, 1 ml) versetzt und 1 h bei Raumtemperatur geschüttelt. Anschließend wurde bei Raumtemperatur mit 100 μmol des entsprechenden Amins (0,1 M in DCM, 1 ml) versetzt und weitere 12 h bei RT geschüttelt. Nach beendeter Reaktion wurde mit 3 ml Wasser versetzt, 15 min geschüttelt und anschließend die organische Phase abgetrennt. Das Lösungsmittel wurde in der Genevac abgezogen und die Produkte mittels HPLC gereinigt.

Nach dieser Methode wurden die folgenden Verbindungen synthetisiert (**Tabelle T3**):

**Tabelle T3**

| Beispiel | Name | MS m/z $[M+H]^+$ |
|---|---|---|
| 1 | 2-(2-(Phenylsulfonyl)ethylthio)-N-(pyridin-2-ylmethyl)nicotinamid | 414.09 |
| 2 | 2-(2-(Phenylsulfonyl)ethylthio)-N-(pyridin-4-ylmethyl)nicotinamid | 414.09 |
| 3 | N-(3-Fluorphenethyl)-2-(2-(phenylsulfonyl)ethylthio)nicotinamid | 445.10 |
| 4 | N-Methyl-N-(3-methylbenzyl)-2-(2-(phenylsulfonyl)ethylthio)nicotinamid | 441.12 |
| 5 | N-(4-methylbenzyl)-2-(2-(phenylsulfonyl)ethylthio)nicotinamid | 427.11 |
| 6 | 2-(2-(Phenylsulfonyl)ethylthio)-N-(2-(trifluormethyl)benzyl)nicotinamid | 481.08 |
| 7 | 2-(2-(Phenylsulfonyl)ethylthio)-N-(pyridin-3-ylmethyl)nicotinamid | 414.09 |
| 8 | N-(3,5-Difluorbenzyl)-2-(2-(phenylsulfonyl)ethylthio)nicotinamid | 449.07 |
| 9 | N-Methyl-N-phenethyl-2-(2-(phenylsulfonyl)ethylthio)nicotinamid | 441.12 |
| 10 | N-(3-Methoxybenzyl)-N-methyl-2-(2-(phenylsulfonyl)ethylthio)nicotinamid | 457.12 |
| 11 | N-(2-Fluorbenzyl)-2-(2-(phenylsulfonyl)ethylthio)nicotinamid | 431.08 |
| 12 | N-(3,4-Difluorbenzyl)-2-(2-(phenylsulfonyl)ethylthio)nicotinamid | 449.07 |
| 13 | N-(3-Brombenzyl)-N-methyl-2-(2-(phenylsulfonyl)ethylthio)nicotinamid | 505.02 |
| 14 | N-(2-Methoxybenzyl)-2-(2-(phenylsulfonyl)ethylthio)nicotinamid | 443.10 |
| 15 | N-(3-Fluorbenzyl)-2-(2-(phenylsulfonyl)ethylthio)nicotinamid | 431.08 |
| 16 | N-(Furan-2-ylmethyl)-N-methyl-2-(2-(phenylsulfonyl)ethylthio)nicotin-amid | 417.09 |
| 17 | N-(4-Methoxybenzyl)-2-(2-(phenylsulfonyl)ethylthio)nicotinamid | 443.10 |
| 18 | N-(2-Chlorbenzyl)-2-(2-(phenylsulfonyl)ethylthio)nicotinamid | 447.05 |

(fortgesetzt)

| Beispiel | Name | MS m/z $[M+H]^+$ |
|---|---|---|
| 19 | N-(3,4-Dichlorbenzyl)-2-(2-(phenylsulfonyl)ethylthio)nicotinamid | 481.01 |
| 20 | N-(4-Fluorbenzyl)-2-(2-(phenylsulfonyl)ethylthio)nicotinamid | 431.08 |
| 21 | N-(2-Methoxyphenethyl)-2-(2-(phenylsulfonyl)ethylthio)nicotinamid | 457.12 |
| 22 | N-(2,6-Difluorbenzyl)-2-(2-(phenylsulfonyl)ethylthio)nicotinamid | 449.07 |
| 23 | N-(2-Methylbenzyl)-2-(2-(phenylsulfonyl)ethylthio)nicotinamid | 427.11 |
| 24 | N-(3,5-Dimethoxybenzyl)-2-(2-(phenylsulfonyl)ethylthio)nicotinamid | 473.11 |
| 25 | N-(3-Chlorbenzyl)-2-(2-(phenylsulfonyl)ethylthio)nicotinamid | 447.05 |
| 26 | N-(2,4-Dichlorbenzyl)-2-(2-(phenylsulfonyl)ethylthio)nicotinamid | 481.01 |
| 29 | N-(4-Chlorbenzyl)-2-(2-(phenylsulfonyl)ethylthio)nicotinamid | 447.05 |
| 30 | N-(2,3-Dichlorbenzyl)-2-(2-(phenylsulfonyl)ethylthio)nicotinamid | 481.01 |
| 31 | N-(4-Brombenzyl)-N-methyl-2-(2-(phenylsulfonyl)ethylthio)nicotinamid | 505.02 |
| 32 | N-((1,3-Dioxolan-2-yl)methyl)-N-methyl-2-(2-(phenylsulfonyl)ethylthio)-nicotinamid | 423.10 |
| 33 | N-Benzyl-N-methyl-2-(2-tosylethylthio)nicotinamid | 441.12 |
| 34 | N-(Pyridin-2-ylmethyl)-2-(2-tosylethylthio)nicotinamid | 428.10 |
| 35 | N-(Pyridin-4-ylmethyl)-2-(2-tosylethylthio)nicotinamid | 428.10 |
| 36 | N-(Thiophen-2-ylmethyl)-2-(2-tosylethylthio)nicotinamid | 433.06 |
| 37 | N-(3-Fluorphenethyl)-2-(2-tosylethylthio)nicotinamid | 459.11 |
| 38 | N-Methyl-N-(3-methylbenzyl)-2-(2-tosylethylthio)nicotinamid | 455.14 |
| 39 | N-(Furan-2-ylmethyl)-2-(2-tosylethylthio)nicotinamid | 417.09 |
| 40 | N-(Pyridin-3-ylmethyl)-2-(2-tosylethylthio)nicotinamid | 428.10 |
| 41 | N-(3,5-Difluorbenzyl)-2-(2-tosylethylthio)nicotinamid | 463.09 |
| 42 | N-(3-Methoxybenzyl)-N-methyl-2-(2-tosylethylthio)nicotinamid | 471.13 |
| 43 | N-(2-Fluorbenzyl)-2-(2-tosylethylthio)nicotinamid | 445.10 |
| 44 | N-(3-Methylbenzyl)-2-(2-tosylethylthio)nicotinamid | 441.12 |
| 45 | N-(3,4-Difluorbenzyl)-2-(2-tosylethylthio)nicotinamid | 463.09 |
| 46 | N-(3-Brombenzyl)-N-methyl-2-(2-tosylethylthio)nicotinamid | 519.03 |
| 47 | N-(4-Methoxybenzyl)-2-(2-tosylethylthio)nicotinamid | 457.12 |
| 48 | N-(2-Chlorbenzyl)-2-(2-tosylethylthio)nicotinamid | 461.07 |
| 49 | N-(4-Fluorbenzyl)-2-(2-tosylethylthio)nicotinamid | 445.10 |
| 50 | N-(3,5-Dimethoxybenzyl)-2-(2-tosylethylthio)nicotinamid | 487.13 |
| 51 | N-(3-Chlorbenzyl)-2-(2-tosylethylthio)nicotinamid | 461.07 |
| 52 | 2-(2-Tosylethylthio)-N-(3-(trifluormethyl)benzyl)nicotinamid | 495.09 |

**Beispiel 54:**

**Synthese von N-Benzyl-2-(2-(phenylsulfonyl)ethylthio)nicotinamid**

Eine Lösung von 250 mg (0,77 mmol) 2-(2-(Phenylsulfonyl)-ethylthio)nicotinsäure in DCM (6 ml) wurde mit 132 mg (0,81 mmol) CDI versetzt und 1 h bei RT gerührt. Anschließend wurde eine Lösung von 82 mg (0,77 mmol) Benzylamin in DCM (6 ml) zugegeben und es wurde weitere 16 h bei RT gerührt. Dann wurde die Reaktionslösung jeweils dreimal mit eine ges. aq. Ammoniumchloridlösung und gesättigter, wässriger $NaHCO_3$ -Lösung gewaschen Die organische

Phase wurde über $MgSO_4$ getrocknet, filtriert und im Vakuum eingeengt. Als Rückstand wurden 266 mg (0,64 mmol, 83%) N-Benzyl-2-(2-(phenylsulfonyl)ethylthio)-nicotinamid erhalten.
1H NMR (600 MHz, DMSO-$d_6$) d ppm 3,16 - 3,26 (m, 2 H) 3,56 - 3,68 (m, 2 H) 4,38 - 4,49 (m, 2 H) 7,19 (dd, *J*=7,55, 5,29 Hz, 1 H) 7,22 - 7,28 (m, 1 H) 7,29 - 7,37 (m, 4 H) 7,70 (t, *J*=7,55 Hz, 2 H) 7,79 (t, *J*=7,18 Hz, 1 H) 7,88 (d, *J*=6,80 Hz, 1 H) 7,95 (d, *J*=7,55 Hz, 2 H) 8,31 (d, *J*=3,78 Hz, 1 H) 9,02 (t, *J*=5,67 Hz, 1 H)

**Beispiel 55:**

**Synthese von N-Benzyl-N-methyl-2-(2-(phenylsulfonyl)ethylthio)nicotinamid**

Eine Lösung von 250 mg (0,77 mmol) 2-(2-(Phenylsulfonyl)-ethylthio)nicotinsäure in DCM (6 ml) wurde mit 132 mg (0,81 mmol) CDI versetzt und 1 h bei RT gerührt. Anschließend wurde eine Lösung von 93 mg (0,77 mmol) N-Benzyl-N-methylamin in DCM (6 ml) zugegeben und es wurde weitere 16 h bei RT gerührt. Dann wurde die Reaktionslösung jeweils dreimal mit eine ges. aq. Ammoniumchloridlösung und gesättigter, wässriger $NaHCO_3$ -Lösung gewaschen. Die organische Phase wurde über $MgSO_4$ getrocknet, filtriert und im Vakuum eingeengt. Durch SC mit dem Rückstand (DCM $\rightarrow$ DCM / MeOH 99,5 : 0,5) wurden 152 mg (0,36 mmol, 46%) N-Benzyl-N-methyl-2-(2-(phenylsulfonyl)ethylthio) nicotinamid erhalten.
1H NMR (600 MHz, DMSO-$d_6$) d ppm 3,30 (s, 3 H) 3,31 - 3,37 (m, 2 H) 3,58 - 3,71 (m, 2 H) 4,28 (s, 1 H) 4,65 (s, 1 H) 7,12 - 7,18 (m, 1 H) 7,19 - 7,27 (m, 1 H) 7,28 - 7,34 (m, 1 H) 7,35 - 7,43 (m, 2 H) 7,65 (d, J=6,80 Hz, 1 H) 7,68 - 7,75 (m, 2 H) 7,79 (t, J=6,42 Hz, 1 H) 7,95 (d, J=7,55 Hz, 2 H) 8,25 - 8,35 (m, 1 H)

Beispiel 56:

Synthese von N-(Cyclohexylmethyl)-2-(2-(phenylsulfonyl)-ethylthio)nicotinamid

Eine Lösung von 500 mg (1,55 mmol) 2-(2-(Phenylsulfonyl)-ethylthio)nicotinsäure in DCM (12 ml) wurde mit 264 mg (1,62 mmol) CDI versetzt und 1 h bei RT gerührt. Anschließend wurde eine Lösung von 200 $\mu$l (1,55 mmol) Cyclohexanmethylamin in DCM (12 ml) zugegeben und es wurde weitere 16 h bei RT gerührt. Dann wurde die Reaktionslösung jeweils dreimal mit eine ges. aq. Ammoniumchloridlösung und gesättigter, wässriger $NaHCO_3$ -Lösung gewaschen. Die organische Phase wurde über $MgSO_4$ getrocknet, filtriert und im Vakuum eingeengt. Als Rückstand wurden 624 mg (1,49 mmol, 96%) N-(Cyclohexylmethyl)-2-(2-(phenylsulfonyl)-ethylthio)nicotinamid erhalten.
MS: m/z 419,1 $[M+H]^+$.

**Beispiel 57:**

**Synthese von 2-(2-(Phenylsulfonyl)ethylthio)-N-(1-(3-(trifluormethyl)phenyl)-ethyl)nicotinamid**

Eine Lösung von 500 mg (1,55 mmol) 2-(2-(Phenylsulfonyl)-ethylthio)nicotinsäure in DCM (12 ml) wurde mit 264 mg (1,62 mmol) CDI versetzt und 1 h bei RT gerührt. Anschließend wurde eine Lösung von 292 mg (1,55 mmol) 1-(3-(Trifluormethyl)-phenyl)ethylamin in DCM (12 ml) zugegeben und es wurde weitere 16 h bei RT gerührt. Dann wurde die Reaktionslösung jeweils dreimal mit eine ges. aq. Ammoniumchloridlösung und gesättigter, wässriger NaCl-Lösung gewaschen. Die organische Phase wurde über $MgSO_4$ getrocknet, filtriert und im Vakuum eingeengt. Durch SC mit dem Rückstand (Ethylacetat /Hexan 1: 1) wurden 462 mg (0,93 mmol, 60%) 2-(2-(Phenylsulfonyl) ethylthio)-N-(1-(3-(trifluormethyl)phenyl)-ethyl)nicotinamid erhalten. MS: m/z 495,1 $[M+H]^+$.

**Beispiel 58:**

**Synthese von N-(2-Cyclohexyl-ethyl)-2-(2-(phenylsulfonyl)ethylthio)nicotinamid**

Eine Lösung von 300 mg (0,93 mmol) 2-(2-(Phenylsulfonyl)-ethylthio)nicotinsäure in DCM (8 ml) wurde mit 158 mg (0,93 mmol) CDI versetzt und 1 h bei RT gerührt. Anschließend wurde eine Lösung von 151 mg (0,93 mmol) 2-Cyclohexyl-ethylamin Hydrochlorid und 157 $\mu$l (0,93 mmol) Diisopropylethylamin in DCM (8 ml) zugegeben und es wurde weitere 16 h bei RT gerührt. Dann wurde die Reaktionslösung jeweils dreimal mit eine ges. aq. Ammoniumchloridlösung und gesättigter, wässriger $NaHCO_3$ -Lösung gewaschen. Die organische Phase wurde über $MgSO_4$ getrocknet, filtriert und im Vakuum eingeengt. Als

Rückstand wurden 390 mg (0,90 mmol, 97%) N-(2-Cyclohexyl-ethyl)-2-(2-(phenylsulfonyl)-ethylthio)nicotinamid erhalten. MS: m/z 433,2 $[M+H]^+$

**Beispiel 59:**

**Synthese von 2-(2-(Cyclohexylthio)ethylthio)-N-(thiophen-2-ylmethyl)nicotin-amid**

Eine Lösung von 500 mg (2,0 mmol) 2-Mercapto-N-(thiophen-2-ylmethyl)nicotinamid (**V1**) in DMF (5 ml) wurde mit 607 mg (4,4 mmol) $K_2CO_3$ versetzt und 1 h bei RT gerührt. Anschließend wurden 446 mg (2-Bromethyl)(cyclo-hexyl) sulfan (Rohprodukt **V2**) zugegeben und es wurde weitere 18 h bei RT gerührt. Danach wurde mit Ethylacetat verdünnt und mit Wasser versetzt. Die Phasen wurden getrennt und die wässrige Phase wurde mit Ethylacetat extrahiert. Die vereinten organischen Phasen wurden über $MgSO_4$ getrocknet, filtriert und im Vakuum eingeengt. Durch SC (Ethylacetat /n-Hexan 1:1) mit dem Rückstand wurden 268 mg (0,68 mmol, 34%) 2-(2-(Cyclohexylthio)-ethylthio)-N-(thiophen-2-ylmethyl)nicotin-amid erhalten. MS: m/z 393,1 $[M+H]^+$.

**Beispiel 100:**

**Synthese von 2-[2-(4-Fluorphenyl)sulfonylethylthio]-N-(2-thienylmethyl)-nicotinamid**

Eine Lösung von 500 mg (2,0 mmol) 2-Mercapto-N-(thiophen-2-ylmethyl)nicotinamid in DMF (5 ml) wurde mit 303 mg (2,2 mmol) $K_2CO_3$ versetzt und 30 min bei RT gerührt. Anschließend wurden 445 mg (2,0 mmol) 1-(2-Chlorethylsulfonyl)-4-fluorobenzol zugegeben und es wurde weitere 2 d bei RT gerührt. Dann wurde im Vakuum eingeengt und der Rückstand wurde mit EE aufgenommen und mit einer 1 M wässrigen $NaHCO_3$-Lösung versetzt. Die Phasen wurden getrennt und die wässrige Phase wurde mit EE extrahiert. Die vereinten organischen Phasen wurden über $MgSO_4$ getrocknet, durch Kieselgel filtriert und im Vakuum eingeengt. Nach SC (DCM / EE 4:1) des Rückstands wurden 365 mg (0,84 mmol, 48%) 2-[2-(4-Fluorphenyl)sulfonylethylthio]-N-(2-thienylmethyl)-nicotinamid erhalten. MS: m/z 437,0 $[M+H]^+$

**Beispiel 106:**

**Synthese von 2-[2-(3-Fluorphenyl)sulfonylethylthio]-N-(2-thienylmethyl)-nicotinamid**

Eine Lösung von 408 mg (2,0 mmol) 2-(3-Fluorphenylsulfonyl)ethanol in DCM (5 ml) wurde auf 0 °C abgekühlt und mit 304 μl (2,2 mmol) $NEt_3$ und 154 μl (2,0 mmol) Methansulfonsäurechlorid versetzt und 16 h bei RT gerührt. In einem anderen Gefäß wurde eine Lösung aus 500 mg (2,0 mmol) 2-Mercapto-N-(thiophen-2-ylmethyl)nicotinamid in DMF (6 ml) mit 275 mg (2,0 mmol) $K_2CO_3$ versetzt und 30 min bei RT gerührt. Die DCM-Reaktionslösung wurde zu dieser Lösung gegeben und es wurde 72 h bei RT weitergerührt. Anschließend wurde mit EE verdünnt und mit einer 1 N aq. $NaHCO_3$-Lösung gewaschen. Die organische Phase wurde über $MgSO_4$ getrocknet, filtriert und im Vakuum eingeengt. Nach SC (DCM / EE 4:1) des Rückstands wurden 194 mg (0,44 mmol, 22%) 2-[2-(3-Fluorphenyl)sulfonylethylthio]-N-(2-thienylmethyl)-nicotinamid erhalten. MS: m/z 437,0 $[M+H]^+$

**Beispiel 112:**

**Synthese von N-(2-Thienylmethyl)-2-[2-[[2-(trifluormethyl)phenyl]thio]ethylthiol-nicotinamid**

Eine Lösung von 375 mg (1,5 mmol) 2-Mercapto-N-(thiophen-2-ylmethyl)nicotinamid in DMF (6 ml) wurde mit 227 mg (1,65 mmol) $K_2CO_3$ versetzt und 60 min bei RT gerührt. Anschließend wurden 361 mg (1,5 mmol) (2-Chlorethyl) (3-(trifluormethyl)phenyl)sulfan zugegeben und es wurde weitere 16 h bei RT gerührt. Dann wurde mit EE verdünnt und mit Wasser extrahiert. Die organische Phase wurde über $MgSO_4$ getrocknet, filtriert und im Vakuum eingeengt. Nach SC (Hexan / EE 1:1) des Rückstands gefolgt von einer weiteren SC (DCM / EE 19:1) des erhaltenen Rückstands wurden 274 mg (0,60 mmol, 40%) N-(2-Thienylmethyl)-2-[2-[[2-(trifluormethyl)phenyl]thio]ethylthio]-nicotinamid erhalten. MS: m/z 455,0 $[M+H]^+$

**Beispiel 141:**

**Synthese von 2-[2-(Benzolsulfonyl)ethylthio]-6-methyl-N-(2-thienylmethyl)-nicotinamid**

Eine Lösung von 607 mg (3,0 mmol) 2-(Phenylsulfonyl)ethanthiol in DMF (10 ml) wurde bei 0 °C mit 336 mg (3,0 mmol) Kalium-tert.-butylat versetzt. Nach 10 min Rühren bei 0 °C wurden 534 mg (2,0 mmol) 2-Cloro-6-methyl-N-(thiophen-2-yl-methyl)nicotinamid zugegeben und anschließend wurde 16 h auf 50 °C erhitzt. Dann wurde mit EE verdünnt und mit Brine gewaschen. Die organische Phase wurde über $Na_2SO_4$ getrocknet, filtriert und im Vakuum

eingeengt. Nach SC (Hexan / EE 7:3) des Rückstands wurden 556 mg (1,28 mmol, 64%) 2-[2-(Benzolsulfonyl)ethylthio]-6-methyl-N-(2-thienylmethyl)-nicotinamid erhalten. MS: m/z 433,1 $[M+H]^+$

**Beispiel 143:**

**Synthese von 2-[2-(Benzolsulfonyl)ethylthio]-6-fluor-N-(2-thienylmethyl)-nicotinamid**

Eine Lösung von 465 mg (2,3 mmol) 2-(Phenylsulfonyl)ethanthiol in DMF (7 ml) wurde bei 0 °C mit 258 mg (2,3 mmol) Kalium-tert.-butylat versetzt. Nach 10 min Rühren bei 0 °C wurden 381 mg (1,5 mmol) 2,6-Difluor-N-(thiophen-2-ylmethyl)-nicotinamid zugegeben und anschließend wurde 16 h auf 50°C erhitzt. Dann wurde mit EE verdünnt und mit Brine gewaschen. Die organische Phase wurde über $Na_2SO_4$ getrocknet, filtriert und im Vakuum eingeengt. Nach SC (Hexan / EE 7:3) des Rückstands wurden 298 mg (0,68 mmol, 45%) 2-[2-(Benzolsulfonyl)ethylthio]-6-fluor-N-(2-thienylmethyl)-nicotinamid erhalten. MS: m/z 437,0 $[M+H]^+$

**Beispiel 149:**

**Synthese von 2-(2-(Benzolsulfonyl)ethylthio]-5-methyl-N-(2-thienylmethyl)-nicotinamid**

Eine Lösung von 337 mg (1,0 mmol) 5-Methyl-2-(2-(phenylsulfonyl)ethylthio)-nicotinsäure in DMF (3 ml) wurde bei 0 °C mit 456 mg (1,2 mmol) HATU und 680 µl (4,0 mmol) DIPEA versetzt. Nach weiteren 15 min Rühren bei 0 °C wurden 113 mg (1, 0 mmol) Thiophen-2-yl-methylamin zugegeben und es wurde 16 h bei RT gerührt. Dann wurde mit EE verdünnt und nacheinander mit einer ges. aq. Zitronensäurelösung, einer ges. aq. $Na_2CO_3$-Lösung und Brine gewaschen. Die organische Phase wurde über $Na_2SO_4$ getrocknet, filtriert und im Vakuum eingeengt. Nach SC(Hexan / EE 7:3) des Rückstands wurden 418 mg (0,97 mmol, 97%) 2-[2-(Benzolsulfonyl)-ethylthio]-5-methyl-N-(2-thienylmethyl)-nicotinamid erhalten. MS: m/z 433,1 $[M+H]^+$

**Beispiele 60-88 und 94-150**

Die Synthese der Beispiele **60 - 88** und **94 -150** erfolgte nach den für die Beispiele **56 - 59, 100, 106, 112, 141, 143** und **149** beschriebenen Verfahren. Welche Beispiele danach nach welchem Verfahren hergestellt wurden, ist in **Tabellen T4** und **T5** zusammengefaßt.

**Tabelle T4**

| Beispiel | Name | Synthese analog Beispiel Nummer | Ausbeute [%] | MS m/z $[M+H]^+$ |
|---|---|---|---|---|
| **60** | N-(Neopentyl)-2-(2-(phenylsulfonyl) ethylthio)nicotinamid | **56** | 71 | 393,1 |
| **61** | N-(5-Methylfuran-2-yl-methyl)-2-(2-(phenylsulfonyl)ethylthio)nicotinamid | **56** | 83 | 417,1 |
| **62** | N-(Furan-2-yl-methyl)-2-(2-(phenylsulfonyl)ethylthio)nicotinamid | **56** | 75 | 403,1 |
| **63** | 2-(2-(Phenylsulfonyl)ethylthio)-N-(tetrahydro-2H-pyran-4-yl-methyl) nicotinamid | **56** | 90 | 421,1 |
| **64** | 2-(2-(Phenylsulfonyl)ethylthio)-N-(4-(trifluormethylthio)benzyl)nicotinamid | **57** | 50 | 513,1 |
| **65** | 2-(2-(Phenylsulfonyl)ethylthio)-N-(3-tolylmethyl)nicotinamid | **57** | 28 | 427,1 |
| **66** | (*R*)-N-(1-Cyclohexyl-ethyl)-2-(2-(phenylsulfonyl)ethylthio)nicotinamid | **57** | 31 | 433,2 |

(fortgesetzt)

| Beispiel | Name | Synthese analog Beispiel Nummer | Ausbeute [%] | MS m/z $[M+H]^+$ |
|---|---|---|---|---|
| **67** | N-(1-(3,4-Dimethylphenyl)ethyl)-2-(2-(phenylsulfonyl)ethylthio)nicotinamid | **57** | 54 | 455,1 |
| **68** | N-(1-Thiophen-2-ylethyl)-2-(2-(phenylsulfonyl)ethylthio)nicotinamid | **57** | 57 | 433,1 |
| **69** | N-(1-(3,5-Dimethylphenyl)methyl)-2-(2-(phenylsulfonyl)ethylthio) nicotinamid | **57** | 52 | 441,1 |
| **70** | N-(Cyclohexylmethyl)-2-(2-(3-trifluormethyl-phenylsulfonyl)ethylthio) nicotinamid | **57** | 40 | 487,1 |
| **71** | (S)-N-(1-Cyclohexyl-ethyl)-2-(2-(phenylsulfonyl)ethylthio)nicotinamid | **57** | 44 | 433,2 |
| **72** | N-(1-(3,5-Dimethylphenyl)ethyl)-2-(2-(phenylsulfonyl)ethylthio)nicotinamid | **57** | 46 | 455,1 |
| **73** | N-(Thiophen-2-ylmethyl)-2-(2-(3-(trifluormethyl)phenylthio)ethylthio) nicoti namid | **59** | 25 | 455,0 |
| **74** | N-(Cyclopentylmethyl)-2-(2-(3-trifluormethyl-phenylsulfonyl)ethylthio) nicotinamid | **57** | 73 | 405,1 |
| **75** | N-(Cyclobutylmethyl)-2-(2-(3-trifluormethyl-phenylsulfonyl)ethylthio) nicotinamid | **58** | 62 | 391,1 |
| **76** | N-((1,4-dioxan-2-yl)methyl)-2-(2-(phenylsulfonyl)ethylthio)nicotinamid | **57** | 57 | 423,1 |
| **77** | 2-(2-(Phenylsulfonyl)ethylthio)-N-(4-(pyridin-2-yloxy)benzyl)nicotinamid | **58** | 52 | 506,1 |
| **78** | N-(2-Methylbutyl)-2-(2-(phenyl-sulfonyl)ethylthio)nicotinamid | **56** | 59 | 393,1 |
| **79** | N-(2-Ethylbutyl)-2-(2-(phenylsulfonyl) ethylthio)nicotinamid | **56** | 60 | 407,1 |
| **80** | N-(Cyclopropylmethyl)-2-(2-(3-trifluormethyl-phenylsulfonyl)ethylthio) nicotinamid | **56** | 61 | 377,1 |
| **81** | N-(3-(2-Methoxyethoxy)propyl)-2-(2-(phenylsulfonyl)ethylthio)nicotinamid | **56** | 58 | 439,1 |
| **82** | 2-(2-(Phenylsulfonyl)ethylthio)-N-(1-(4-(trifluormethylthio)phenyl)ethyl) nicotina mid | **56** | 38 | 527,1 |

(fortgesetzt)

| Beispiel | Name | Synthese analog Beispiel Nummer | Ausbeute [%] | MS m/z $[M+H]^+$ |
|---|---|---|---|---|
| **83** | N-(3-(1H-Pyrazol-1-yl)benzyl)-2-(2-(phenylsulfonyl)ethylthio)nicotinamid | **57** | 57 | 479,1 |
| **84** | N-((2,3-Dihydrobenzofuran-5-yl) methyl)-2-(2-(phenylsulfonyl)ethylthio) nicotinamid | **58** | 57 | 455,1 |
| **85** | N-(4-Phenoxybenzyl)-2-(2-(phenyl-sulfonyl)ethylthio)nicotinamid | **57** | 52 | 505,1 |
| **86** | N-(((1R,2S,5R)-6,6-dimethylbicyclo [3.1.1]heptan-2-yl)methyl)-2-(2-(phenylsulfonyl)ethylthio)nicotinamid | **57** | 75 | 459,2 |
| **87** | N-(Thiophen-2-ylmethyl)-2-(2-(3-(trifluormethyl)phenylsulfonyl) ethylthio)n icotinamid | **57** | 64 | 487,0 * |
| **88** | 2-(2-(Phenylsulfonyl)ethylthio)-N-(3-(trifluormethyl)benzyl)nicotinamid | **57** | 71 | 481,1 |
| Weitere getestete Verbindungen: | | | | |
| **89** | 2-(2-(Phenylsulfonyl)ethylthio)-N-(thiophe en-2-ylmethyl)nic otinamid | | | |
| **90** | N-(Pyridin-2-ylmethyl)-2-(2-(3-(trifluoromethyl)phenylsulfonyl)ethylthio)nicotinamid | | | |
| **91** | N-(Pyridin-2-ylmethyl)-2-(2-(5-(trifluoromethyl) pyridin-2-ylsulfonyl)ethylthio)nicotinamid | | | |
| **92** | N-(Thiophen-2-ylmethyl)-2-(2-(5-(trifluoro ethyl)pyridin-2-ylsulfonyl)ethylthio)nicotinamid | | | |

**Beispiel 93:**

**Synthese von N-(Isobutyl)-2-(2-(phenylsulfonyl)ethylthio)nicotinamid**

Eine Lösung von 323 mg (1,00 mmol) 2-(2-(phenylsulfonyl)-ethylthio)nicotinsäure in DCM (16 ml) wurde mit 171 mg (1,05 mmol) CDI versetzt und 1 h bei RT gerührt. Anschließend wurden 99 μl (1,00 mmol) Isobutylamin zugegeben und es wurde weitere 5 d bei RT gerührt. Dann wurde die Reaktionslösung jeweils zweimal mit eine 4M aq. Ammoniumchloridlösung und einer 1 M aq. Natriumhydrogencarbonat-lösung gewaschen. Die organische Phase wurde über $MgSO_4$ getrocknet, filtriert und im Vakuum eingeengt. Der erhaltene Rückstand wurde mit Ethylacetat (30 ml) aufgenommen und mit einer 0,4 M Salzsäure (5 ml) gewaschen. Erneut wurde organische Phase über $MgSO_4$ getrocknet, filtriert und im Vakuum eingeengt. Als Rückstand wurden 160 mg (0,42 mmol, 42 N-(Isobutyl)-2-(2-(phenylsulfonyl) ethylthio)nicotinamid erhalten. MS: m/z 379,1 $[M+H]^+$

**Tabelle T5**

| Beispiel | Name | Synthese analog Beispiel Nummer | Ausbeute [%] | MS m/z $[M+H]^+$ |
|---|---|---|---|---|
| **94** | 2-[2-(Benzolsulfonyl)ethylthio]-N-(2-tetrahydropyranylmethyl)-nicotinamid | 57 | 53 | 421,1 |
| **95** | 2-[2-(Benzolsulfonyl)ethylthio]-N-[(5-methyl-2-thienyl)methyl]-nicotinamid | 57 | 80 | 433,1 |
| **96** | 2-[2-(Benzolsulfonyl)ethylthio]-N-[(4-methyl-2-thienyl)methyl]-nicotinamid | 57 | 84 | 433,1 |

(fortgesetzt)

| Beispiel | Name | Synthese analog Beispiel Nummer | Ausbeute [%] | MS m/z $[M+H]^+$ |
|---|---|---|---|---|
| **97** | N-(1-Adamantylmethyl)-2-[2-(benzolsulfonyl) ethylthiol-nicotinamid | 57 | 39 | 471,2 |
| **98** | 2-[2-(Benzolsulfonyl)ethylthio]-N-[(3-morpholinophenyl)methyl]-nicotinamid | 57 | 67 | 498,1 |
| **99** | 2-[2-(4-Chlorphenyl)sulfonylethylthio]-N-(2-thienylmethyl)-nicotinamid | 100 | 27 | 453,0 |
| **101** | N-(2-Thienylmethyl)-2-[2-[3-(trifluormethoxy) phenyl]sulfonylethylthio ]-nicotinamid | 100 | 42 | 503,0 |
| **102** | N-(2-Thienylmethyl)-2-[2-[4-(trifluormethyl) phenyl]sulfonylethylthio]-nicotinamid | 100 | 76 | 487,0 |
| **103** | N-(2-Thienylmethyl)-2-[2-[4-(trifluormethoxy) phenyl]sulfonylethylthio ]-nicotinamid | 100 | 24 | 503,0 |
| **104** | 2-[2-(m-Tolylsulfonyl)ethylthio]-N-(2-thienylmethyl)-nicotinamid | 100 | 19 | 433,1 |
| **105** | 2-[2-(m-Tolylthio)ethylthio]-N-(2-thienylmethyl)-nicotinamid | 112 | 38 | 401,1 |
| **107** | 2-[2-(Benzolsulfonyl)ethylthio]-N-(3,3-dimethylbutyl)-nicotinamid | 57 | 80 | 407,1 |
| **108** | 2-[2-(Benzolsulfonyl)ethylthio]-N-(2-benzothiophenylmethyl)-nicotinamid | 57 | 60 | 469,1 |
| **109** | 2-[2-(Phenylthio)ethylthio]-N-(2-thienylmethyl)-nicotinamid | 112 | 26 | 387,1 |
| **110** | 2-[2-(Benzolsulfinyl)ethylthio]-N-(2-thienylmethyl)-nicotinamid | 112 | 26 | 403,1 |
| **111** | 2-(2-Cyclohexylsulfonylethylthio)-N-(2-thienylmethyl)-nicotinamid | 112 | 11 | 425,1 |
| **113** | N-(2-Thienylmethyl)-2-[2-[2-(trifluormethyl) phenyl]sulfinylethylthio]-nicotinamid | 112 | 41 | 471,0 |
| **114** | N-(2-Thienylmethyl)-2-[2-[2-(trifluormethyl) phenyl]sulfonylethylthio]-nicotinamid | 100 | 39 | 487,0 |
| **115** | 2-[2-(Benzolsulfonyl)ethylthio]-N-[(5-chlor-2-thienyl)methyl]-nicotinamid | 57 | 61 | 453,0 |
| **116** | 2-[2-(2-Fluorphenyl)sulfonylethylthio]-N-(2-thienylmethyl)-nicotinamid | 100 | 17 | 437,0 |
| **117** | 2-[2-[3,5-bis(Trifluormethyl)phenyl] sulfonylethylth io]-N-(2-thienylmethyl)-nicotinamid | 100 | 12 | 555,0 |
| **118** | 2-[2-(3-Methoxyphenyl)sulfonylethylthio]-N-(2-thienylmethyl)-nicotinamid | 100 | 28 | 449,1 |
| **119** | 2-[2-(4-Methoxyphenyl)sulfonylethylthio]-N-(2-thienylmethyl)-nicotinamid | 100 | 33 | 449,1 |

(fortgesetzt)

| Beispiel | Name | Synthese analog Beispiel Nummer | Ausbeute [%] | MS m/z $[M+H]^+$ |
|---|---|---|---|---|
| **120** | 2-[2-(Benzolsulfonyl)ethylthio]-N-(4-tetrahydrothiopyranylmethyl)-nicotinamid | 57 | 60 | 437,1 |
| **121** | 2-[2-(4-Ethylphenyl)sulfonylethylthio]-N-(2-thienylmethyl)-nicotinamid | 57 | 16 | 447,1 |
| **122** | N-(2-Thienylmethyl)-2-[2-[[4-(trifluormethyl) phenyl]thio]ethylthio]-nicotinamid | 112 | 41 | 455,0 |
| **123** | 2-[2-(o-Tolylthio)ethylthio]-N-(2-thienylmethyl)-nicotinamid | 112 | 25 | 401,1 |
| **124** | 2-[2-[(3-Fluorphenyl)thio]ethylthio]-N-(2-thienylmethyl)-nicotinamid | 112 | 39 | 405,0 |
| **125** | 2-[2-[(3,4-Difluorphenyl)thio]ethylthio]-N-(2-thienylmethyl)-nicotinamid | 112 | 42 | 423,0 |
| **126** | 2-[2-[(2,4-Difluorphenyl)thio]ethylthio]-N-(2-thienylmethyl)-nicotinamid | 112 | 41 | 423,0 |
| **127** | 2-[2-(Benzolsulfonyl)ethylthio]-N-[2-(2-thienyl) ethyl]-nicotinamid | 57 | 60 | 433,1 |
| **128** | 2-[2-(Benzolsulfonyl)ethylthio]-N-phenthyl-nicotinamid | 57 | 63 | 427,1 |
| **129** | 2-[2-(Benzolsulfonyl)ethylthio]-N-(3-phenylpropyl)-nicotinamid | 57 | 60 | 441,1 |
| **130** | 2-[2-(3,4-Difluorphenyl)sulfonylethylthiol-N-(2-thienylmethyl)-nicotinamid | 100 | 50 | 455,0 |
| **131** | 2-[2-(2,4-Difluorphenyl)sulfonylethylthio]-N-(2-thienylmethyl)-nicotinamid | 100 | 67 | 455,0 |
| **132** | 2-[2-[(2-Fluorphenyl)thio]ethylthio]-N-(2-thienylmethyl)-nicotinamid | 112 | 40 | 405,0 |
| **133** | 2-[2-[(4-Fluorphenyl)thio]ethylthio]-N-(2-thienylmethyl)-nicotinamid | 112 | 43 | 405,0 |
| **134** | 2-[2-[(4-Chlorphenyl)thio]ethylthio]-N-(2-thienylmethyl)-nicotinamid | 112 | 49 | 421,0 |
| **135** | 2-[2-(p-Tolylthio)ethylthio]-N-(2-thienylmethyl)-nicotinamid | 112 | 52 | 401,1 |
| **136** | 2-[2-(Benzolsulfonyl)ethylthio]-N-isopentyl-nicotinamid | 57 | 53 | 393,1 |
| **137** | 2-[2-(Benzolsulfonyl)ethylthio]-N-(2-cyclopropylethyl)-nicotinamid | 57 | 57 | 391,1 |
| **138** | 2-[2-(Benzolsulfonyl)ethylthio]-N-(2-cyclopentylethyl)-nicotinamid | 57 | 40 | 419,1 |
| **139** | N-(3,3-Dimethylbutyl)-2-[2-[3-(trifluormethyl) phenyl]sulfonylethylthio]-nicotinamid | 57 | 17 | 475,1 |
| **140** | N-(Cyclopentylmethyl)-2-[2-[3-(trifluormethyl) phenyl]sulfonylethylthio]-nicotinamid | 57 | 26 | 473,1 |

(fortgesetzt)

| Beispiel | Name | Synthese analog Beispiel Nummer | Ausbeute [%] | MS m/z [M+H]+ |
|---|---|---|---|---|
| **142** | 2-[2-(Benzolsulfonyl)ethylthio]-N-(2-thienylmethyl)-6-(trifluormethyl)-nicotinamid | 141 | 41 | 487,0 |
| **144** | 2-[2-(Benzolsulfonyl)ethylthio]-N-[(3-methylcyclohexyl)methyl]-nicotinamid | 57 | | 433,2 |
| **145** | 2-[2-(Benzolsulfonyl)ethylthio]-N-(cycloheptylmethyl)-nicotinamid | 57 | | 433,2 |
| **146** | 2-[2-(Benzolsulfonyl)ethylthio]-N-[(2-methylcyclohexyl)methyl]-nicotinamid | 57 | | 433,2 |
| **147** | 2-[2-(Benzolsulfonyl)ethylthio]-N-[(4-methylcyclohexyl)methyl]-nicotinamid | 57 | | 433,2 |
| **148** | 2-[2-(Benzolsulfonyl)ethylthio]-5-fluor-N-(2-thienylmethyl)-nicotinamid | 143 | | 437,0 |
| **150** | 2-[2-(Benzolsulfonyl)ethylthio]-N-(2-thienylmethyl)-5-(trifluormethyl)-nicotinamid | 141 | | 487,0 |

**Biologische Daten**

**Fluoreszenzassay unter Verwendung eines ’voltage sensitive dyes’**

Humane, KCNQ2/3-Kanäle exprimierende CHO-K1-Zellen werden in Zellkulturflaschen (z.B. 80 $cm^2$ TC flasks, Nunc) mit DMEM-high glucose (Sigma Aldrich, D7777) inklusive 10% FCS (PAN Biotech, z.B. 3302-P270521) oder alternativ MEM Alpha Medium (1x, flüssig, Invitrogen, #22571), 10 % Fetal Calf Serum (FCS) (Invitrogen, #10270-106, hitzeinaktiviert) und den notwendigen Selektionsantibiotika bei 37°C, 5 % $CO_2$ und 95 % Luftfeuchtigkeit adhärent kultiviert.

Vor Aussaat für die Messungen werden die Zellen mit einem 1 x DPBS-Puffer ohne $Ca^{2+/}$ $Mg^{2+}$ (z.B. Invitrogen, #14190-094) gewaschen und mittels Accutase (PAA Laboratories, #L11-007) vom Boden des Kulturgefäßes abgelöst (Inkubation mit Accutase für 15 min bei 37°C). Die Bestimmung der dann vorliegenden Zellzahl wird mit einem CASY™ cell counter (Modell TCC, Schärfe System) durchgeführt, um anschließend je nach Dichteoptimierung für die individuelle Zelllinie 20.000 - 30.000 Zellen/well/100 µl des beschriebenen Nährmediums auf die 96 well-Messplatten des Typs Corning™ CellBIND™ (Flat Clear Bottom Black Polystyrene Microplates, #3340) auszubringen. Danach erfolgt eine einstündige Inkubation bei Raumtemperatur ohne Begasung oder Regelung der Luftfeuchtigkeit, gefolgt von einer 24stündigen Inkubation bei 37°C, 5 % $CO_2$ und 95 % Luftfeuchtigkeit.

Der spannungssensitive Fluoreszenzfarbstoff aus dem Membrane Potential Assay Kit (Red™ Bulk format part R8123 for FLIPR, MDS Analytical Technologies™) wird vorbereitet, indem der Inhalt eines Gefäßes *Membrane Potential Assay Kit Red Component A* in 200 ml Extrazellulären Puffers (ES-Puffer, 120 mM NaCl, 1 mM KCl, 10 mM HEPES, 2 mM $CaCl_2$, 2 mM $MgCl_2$, 10 mM Glucose; pH 7,4) gelöst wird. Nach Abnahme des Nährmediums werden die Zellen mit 200 µl ES-Puffer gewaschen, anschließend mit 100 µl der oben angesetzten Farbstofflösung überschichtet und 45 min bei Raumtemperatur unter Lichtverschluss inkubiert.

Die Fluoreszenzmessungen werden mit einem BMG Labtech FLUOstar™-, BMG Labtech NOVOstar™- oder BMG Labtech POLARstar™-Instrument durchgeführt (525 nm Exication, 560 nm Emission, Bottom Read mode). Nach der Farbstoff-Inkubation werden 50 µl der zu testenden Substanzen in den gewünschten Konzentrationen oder 50 µl ES-Puffer zwecks Kontrolle in separate Cavitäten der Messplatte gegeben und 30 min bei Raumtemperatur unter Abschirmung von Licht inkubiert. Anschließend misst man für 5 min die Fluoreszenzintensität des Farbstoffs und ermittelt so zu einem festgelegten und gleichbleibenden Zeitpunkt den Fluoreszenzwert $F_1$ eines jeden wells. Daraufhin erfolgt Zugabe von 15 µl einer 100 mM KCl-Lösung (Endkonzentration 92 mM) in jedes well. Die Veränderung der Fluoreszenz wird anschließend so lange gemessen, bis alle relevanten Messwerte erhalten sind (vornehmlich 5-30 min). Zu einem festgelegten Zeitpunkt nach KCl-Applikation wird ein Fluoreszenzwert $F_2$ ermittelt, in diesem Falle zum Zeitpunkt des Fluoreszenzpeaks.

Zur Berechnung wird die Fluoreszenzintensität $F_2$ mit der Fluoreszenzintensität $F_1$ verglichen und daraus die agonistische Aktivität der Zielverbindung auf den Kaliumkanal ermittelt. $F_2$ und $F_1$ werden hierfür wie folgt verrechnet:

$$\left(\frac{F_2 - F_1}{F_1}\right) \times 100 = \frac{\Delta F}{F} (\%)$$

Um zu ermitteln, ob eine Substanz eine agonistische Aktivität besitzt, kann z.B. $\frac{\Delta F}{F}$ mit $\left(\frac{\Delta F}{F}\right)_K$ von Kontrollzellen verglichen werden. $\left(\frac{\Delta F}{F}\right)_K$ wird ermittelt, indem man dem Reaktionsansatz anstatt der zu testenden Substanz lediglich die Pufferlösung zusetzt, den Wert $F_{1K}$ der Fluoreszenzintensität bestimmt, die Kaliumionen wie oben beschrieben zugibt und einen Wert $F_{2K}$ der Fluoreszenzintensität misst. Dann werden $F_{2K}$ und $F_{1K}$ wie folgt verrechnet:

$$\left(\frac{F_{2K} - F_{1K}}{F_{1K}}\right) \times 100 = \left(\frac{\Delta F}{F}\right)_K (\%)$$

Eine Substanz besitzt eine agonistische Aktivität auf den Kaliumkanal, wenn $\frac{\Delta F}{F}$ größer als $\left(\frac{\Delta F}{F}\right)_K$ ist:

$$\frac{\Delta F}{F} \rangle \left(\frac{\Delta F}{F}\right)_K$$

Unabhängig vom Vergleich von $\frac{\Delta F}{F}$ mit $\left(\frac{\Delta F}{F}\right)_K$ lässt sich auch auf eine agonistische Aktivität einer Zielverbindung schließen, wenn mit steigender Dosierung der Zielverbindung eine Zunahme von $\frac{\Delta F}{F}$ zu beobachten ist. Kalkulationen von $EC_{50}$- und $IC_{50}$-Werten werden mit Hilfe der Software 'Prism v4.0' (GraphPad Software™) durchgeführt.

Folgende Werte wurden exemplarisch bestimmt:

**Tabelle T6**

| Beispiel | % Hemmung [10μM] oder $EC_{50}$ |
|---|---|
| 1 | 14.2 |
| 2 | - |
| 3 | 20.8 |
| 4 | - |
| 5 | 1.86 ($EC_{50}$) |
| 6 | - |
| 7 | - |
| 8 | 1.28 ($EC_{50}$) |
| 9 | - |
| 10 | - |

(fortgesetzt)

| Beispiel | % Hemmung [10μM] oder $EC_{50}$ |
|---|---|
| 11 | 1.17 ($EC_{50}$) |
| 12 | 1.8 ($EC_{50}$) |
| 13 | 1.4 ($EC_{50}$) |
| 14 | 19.1 |
| 15 | 2.48 ($EC_{50}$) |
| 16 | - |
| 17 | 6.52 ($EC_{50}$) |
| 18 | 15.3 |
| 19 | 1.27 ($EC_{50}$) |
| 20 | 1.28 ($EC_{50}$) |
| 21 | 7.9 |
| 22 | 24.7 |
| 23 | - |
| 24 | 19.1 |
| 25 | 1.28 ($EC_{50}$) |
| 26 | - |
| 29 | 90 |
| 33 | - |
| 34 | 9.4 |
| 35 | - |
| 36 | 4.11 ($EC_{50}$) |
| 37 | 15.5 |
| 38 | 1.4 |
| 39 | 6.09 ($EC_{50}$) |
| 40 | 7.9 |
| 41 | 2.39 ($EC_{50}$) |
| 42 | - |
| 43 | 1.97 ($EC_{50}$) |
| 44 | 2.23 ($EC_{50}$) |
| 45 | 2.14 ($EC_{50}$) |
| 46 | 7.4 |
| 47 | 17.5 |
| 48 | 7.7 |
| 49 | 7.6 ($EC_{50}$) |
| 50 | 6 |
| 51 | 4.12 ($EC_{50}$) |
| 52 | 1.82 ($EC_{50}$) |

(fortgesetzt)

| Beispiel | % Hemmung [10 μM] oder $EC_{50}$ |
|---|---|
| 54 | 2.34 ($EC_{50}$) |
| 55 | 16.52 ($EC_{50}$) |
| 56 | 1.01 ($EC_{50}$) |
| 57 | 5.7 ($EC_{50}$) |
| 58 | 1.18 ($EC_{50}$) |
| 59 | 91 |
| 60 | 9.59 ($EC_{50}$) |
| 61 | 3.47 ($EC_{50}$) |
| 62 | 4.39 ($EC_{50}$) |
| 64 | 0.88 ($EC_{50}$) |
| 65 | 0.58 ($EC_{50}$) |
| 66 | 40 |
| 67 | 32 |
| 70 | 106 |
| 71 | 20 |
| 72 | 45 |
| 73 | 107 |
| 74 | 130 |
| 75 | 64 |
| 76 | 52 |
| 77 | 83 |
| 78 | 112 |
| 79 | 76 |
| 80 | 38 |
| 83 | 32 |
| 84 | 81 |
| 85 | 88 |
| 86 | 88 |
| 87 | 103 |
| 89 | 2.28 ($EC_{50}$) |
| 90 | 2.49 ($EC_{50}$) |
| 92 | 2.74 ($EC_{50}$) |
| 99 | 0.234 ($EC_{50}$) |
| 100 | 0.503 ($EC_{50}$) |
| 101 | 156 |
| 102 | 0.197($EC_{50}$) |
| 103 | 83 |

(fortgesetzt)

| Beispiel | % Hemmung [10μM] oder $EC_{50}$ |
|---|---|
| 104 | 151 |
| 105 | 0.168 ($EC_{50}$) |
| 106 | 0.187($EC_{50}$) |
| 107 | 1.418($EC_{50}$) |
| 108 | 57 |
| 109 | 0.075($EC_{50}$) |
| 110 | 64 |
| 111 | 63 |
| 112 | 141 |
| 113 | 52 |
| 114 | 77 |
| 115 | 131 |
| 116 | 107 |
| 117 | 93 |
| 118 | 145 |
| 119 | 57 |
| 120 | 72 |
| 121 | 73 |
| 122 | 108 |
| 123 | 137 |
| 124 | 151 |
| 125 | 147 |
| 126 | 150 |
| 127 | 75 |
| 128 | 48 |
| 129 | 132 |
| 130 | 147 |
| 131 | 116 |
| 132 | 133 |
| 133 | 142 |
| 134 | 150 |
| 135 | 125 |
| 141 | 115 |
| 142 | 62 |
| 143 | 114 |
| 144 | 147 |
| 145 | 164 |

(fortgesetzt)

| Beispiel | % Hemmung [10μM] oder $EC_{50}$ |
|---|---|
| 146 | 101 |
| 147 | 131 |
| 148 | 117 |
| 149 | 46 |

**Voltage-clamp Messungen**

Um eine KCNQ2/3 agonistische Wirkung der Substanzen elektrophysiologisch zu konfirmieren wurden patch-clamp Messungen (Hamill et al., 1981) im voltage-clamp Modus an einer stabil transfizierten hKCNQ2/3 CHO-K1 Zelllinie durchgeführt. Nach Ausbildung des Gigaseals wurden die Zellen zunächst auf ein Haltepotential von -60 mV geklemmt. Im Anschluss wurden depolarisierende Spannungssprünge bis zu einem Potential von +20 mV appliziert (Inkrement: 20 mV, Dauer: 1 Sekunde), um die funktionelle Expression von KCNQ2/3 typischen Strömen zu bestätigen. Die Substanztestung erfolgte bei einem Potential von -40 mV. An jeder Zelle wurde zunächst die durch Retigabin (10 μM) induzierte Stromzunahme bei -40 mV als Positivkontrolle registriert. Nach komplettem Auswaschen des Retigabineffektes (Dauer: 80 s) wurde die Testsubstanz (10 μM) appliziert. Die durch die Testsubstanz induzierte Stromzunahme wurde auf den Retigabineffekt normiert und als relative Efficacy angegeben (s. u.).

Hamill OP, Marty A, Neher E, Sakmann B, Sigworth FJ.: Improved patch-clamp techniques for high-resolution current recording from cells and cell-free membrane patches. Pflugers Arch. 1981 Aug; 391(2):85-100.

Voltage clamp-Messungen wurden nur für ausgesuchte Verbindungen durchgeführt:

**Tabelle T7**

| Beispiel | **MAN** rel Eff @10μM[RG=1] | **MAN** $EC_{50}$ [μM] |
|---|---|---|
| 56 | 1.06 | |
| 58 | 0.47 | |
| 61 | 0.81 | |
| 65 | 0.4 | |
| 89 | | 1.01 |
| 90 | 0.79 | |

**Formalin-Test, Ratte**

Die Untersuchungen zur Bestimmung der antinozizeptiven Wirkung der Verbindungen wurden im Formalin-Test an männlichen Ratten (Sprague-Dawley, 150 - 170 g) durchgeführt. Im Formalin-Test werden die erste (frühe) Phase (0 - 15 min nach Formalin-Injektion) und die zweite (späte) Phase (15 - 60 min nach Formalin-Injektion) unterschieden (D. Dubuisson, S.G. Dennis, Pain 4, 161 - 174 (1977)). Die frühe Phase stellt als direkte Reaktion auf die Formalin-Injektion ein Modell für Akutschmerz dar, während die späte Phase als Modell für persistierenden (chronischen) Schmerz angesehen wird (T.J. Coderre, J. Katz, A.L. Vaccarino, R. Melzack, Pain, Vol. 52, S. 259, 1993).

Die erfindungsgemäßen Verbindungen wurden in der zweiten Phase des Formalin-Tests untersucht, um Aussagen über Substanzwirkungen im chronisch/entzündlichen Schmerz zu erhalten.

Durch eine einmalige subkutane Formalin-Injektion (50 μl, 5 %ig) in die dorsale Seite der rechten Hinterpfote wurde bei freibeweglichen Versuchstieren eine nozizeptive Reaktion induziert, die sich in folgenden Verhaltensparametern darstellt: Heben und Halten der betroffenen Pfote (Score 1), Schütteln bzw. Zucken (Score 2), Lecken und Beißen (Score 3). Die aufgrund der Formalininjektion ausgelösten differierenden Verhaltensweisen wurden durch Beobachtung der Tiere in der späten Phase des Formalin-Tests kontinuierlich erfaßt und in einer Bewertung unterschiedlich gewichtet. Normalverhalten, bei dem das Tier alle vier Pfoten gleichmäßig belastet, wurde als Score 0 registriert. Abhängig von der Applikationsart der erfindungsgemäßen Verbindungen wurde der Applikationszeitpunkt vor der Formalin-Injektion gewählt (intraperitoneal: 15 min, intravenös: 5 min). Nach Injektion von Substanzen, die im Formalin-Test antinozizeptiv wirksam sind, sind die beschriebenen Verhaltensweisen (Score 1 - 3) der Tiere reduziert, evtl. sogar aufgehoben. Der Vergleich erfolgte mit Kontrolltieren, die Vehikel (Lösungsmittel) vor Formalinapplikation erhalten hatten. Das nozizeptive

Verhalten wurde als sogenannte Schmerz-Rate (Pain-Rate, PR) berechnet. Die verschiedenen Verhaltensparameter erhielten eine unterschiedliche Gewichtung (Faktor 0, 1, 2, 3). Die Kalkulation erfolgte in Teilintervallen von 3 min nach folgender Formel:

$$PR = [(T_0 \times 0) + (T_1 \times 1) + (T_2 \times 2) + (T_3 \times 3)] / 180,$$

wobei $T_0$, $T_1$, $T_2$, und $T_3$ jeweils der Zeit in Sekunden entspricht, in der das Tier die Verhaltensweisen 0, 1, 2 oder 3 zeigte. Substanz- und Vehikelgruppen umfassen jeweils n = 10 Tiere. Basierend auf den PR-Berechnungen wurde die Substanzwirkung als Änderung gegen Kontrolle in Prozent ermittelt

**Tabelle T8**

| Beispiel | Applikationsart | Anderung in % |
|---|---|---|
| **56** | 2,15 mg/kg i.v. | -34,1 |
| **73** | 1 mg/kg i.v. | -29,9 |
| **89** | 6,81 mg/kg i.v. | -74,2 |

Darüber hinaus wurde Beispiel 89 im Chung-Test an der Ratte untersucht. Bei i. v.-Applikation wurde ein $ED_{50}$ von 6,3 mg/kg bestimmt (Kim,S.H. and Chung,J.M., An experimental model for peripheral neuropathy produced by segmental spinal nerve ligation in the rat, Pain, 50 (1992) 355-363).

**Abkürzungen**

| | |
|---|---|
| aq. | wässrig |
| Brine | ges. aq. NaCl-Lösung |
| CDI | 1,1'-Carbonyldiimidazol |
| d | Tage |
| DCC | N,N'-Dicyclohexylcarbodiimid |
| DCM | Dichlormethan |
| DIPEA | Diisopropylethylamin |
| DMAP | 4-(Dimethylamino)-pyridin |
| DMF | N,N-Dimethylformamid |
| DMSO | Dimethylsulfoxid |
| EDCI | N'(3-Dimethylaminopropyl)-N'-ethyl-carbodiimid |
| EE | Ethylacetat |
| ges. | gesättigt |
| h | Stunde(n) |
| HATU | O-(7-Aza-Benzotriazol-1-yl)-N,N,N',N'-tetramethyluroniumhexafluorphosphat |
| HBTU | O-(Benzotriazol-1-yl)-N,N,N',N'-tetramethyluroniumhexafluorphosphat |
| HMPT | Hexamethylphosphorsäuretriamid |
| HOBt | 1-hydroxyl-1H-benzotriazol |
| M | molar |
| m/z | Verhältnis Massung zur Ladung |
| MeOH | Methanol |
| min | Minuten |
| MS | Massenspektrometrie |
| RT | Raumtemperatur 23 ± 7 °C |
| SC | Säulenchromatographie auf Kieselgel |
| THF | Tetrahydrofuran |
| vv | Volumenverhältnis |

**Patentansprüche**

1. Substituierte Nicotinamid-Verbindungen der allgemeinen Formel I,

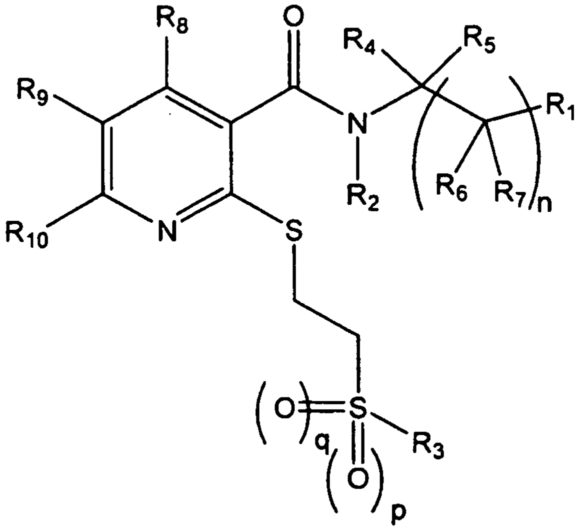

worin

n = 0, 1 oder 2
p = 0 oder 1
q = 0 oder 1
$R^1$ Aryl oder Heteroaryl, unsubstituiert oder einfach oder mehrfach substituiert; $C_{1-6}$-Alkyl, $C_{3-10}$-Cycloalkyl oder Heterocyclyl, unsubstituiert oder einfach oder mehrfach substituiert;
$R^2$ H; $C_1$-$_6$-Alkyl, unsubstituiert oder einfach oder mehrfach substituiert;
$R^3$ Aryl oder Heteroaryl, unsubstituiert oder einfach oder mehrfach substituiert; $C_{1-6}$-Alkyl oder $C_{3-10}$-Cycloalkyl, jeweils unsubstituiert oder einfach oder mehrfach substituiert;
$R^4$, $R^5$, $R^6$ und $R^7$ unabhängig voneinander H; $C_{1-6}$-Alkyl, unsubstituiert oder einfach oder mehrfach substituiert,
$R^8$, $R^9$ und $R^{10}$ unabhängig voneinander H, F, Cl, Br, O-$C_{1-6}$-Alkyl, $CF_3$, $OCF_3$, $SCF_3$, Cis-Alkyl; bedeutet;
mit der Maßgabe, dass wenn $R^3$ 3-Trifluormethylphenyl oder 4-Trifluormethyl-2-pyridyl, $R^2$, $R^4$ und $R^5$ H sowie n 0 bedeuten, $R^1$ nicht 2-Pyridyl oder 2-Thienyl bedeutet
und
wenn $R^3$ Phenyl oder Methyl, $R^2$, $R^4$ und $R^5$ H sowie n 0 bedeuten, $R^1$ nicht 2-Thienyl bedeutet; wobei $C_{1-6}$-Alkyl acyclische gesättigte oder ungesättigte Kohlenwasserstoffreste umfasst und wobei Cycloalkyl und $C_{3-10}$-Cycloalkyl gesättigte oder ungesättigte aber nicht aromatische cyclische Kohlenwasserstoffe umfasst
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

2. Substituierte Nicotinamid-Derivate gemäß Anspruch 1, worin
"Alkyl substituiert", "Heterocyclyl substituiert" und Cycloalkyl substituiert" für die Substitution eines Wasserstoffrestes durch F, Cl, Br, I, -CN, $NH_2$, NH-$C_{1-6}$-Alkyl, NH-$C_{1-6}$-Alkyl-OH, $C_{1-6}$-Alkyl, N($C_{1-6}$-Alkyl)$_2$, N($C_{1-6}$-Alkyl-OH)$_2$, $NO_2$ SH, S-$C_{1-6}$-Alkyl, S-Benzyl, O-$C_{1-6}$-Alkyl, OH, O-$C_{1-6}$-Alkyl-OH, =O, O-Benzyl, C(=O)$C_{1-6}$-Alkyl, $CO_2H$, $CO_2$-$C_{1-6}$-Alkyl, Phenyl, Phenoxy, Morpholinyl, Piperidinyl, Pyrolidinyl oder Benzyl, steht;
und "Aryl substituiert" und "Heteroaryl substituiert" für die ein- oder mehrfache, z.B. zwei-, drei- oder vierfache, Substitution eines oder mehrerer Wasserstoffatome des Ringsystems durch F, Cl, Br, I, CN, $NH_2$, NH-$C_{1-6}$-Alkyl, NH- $C_{1-6}$-Alkyl-OH, N($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alkyl, O-$C_{1-6}$Alkyl-OH, C (=O)$C_{1-6}$-Alkyl, C(=O)NH$C_{1-6}$-Alkyl; o-Pyridyl; C(=O)-Aryl; C(=O)-N-Morpholin; C(=O)-Piperidin; (C=O)-Pyrrolidin; (C=O)-Piperazin; $NHSO_2C_{1-6}$-Alkyl, $NHCOC_{1-6}$-Alkyl, $CO_2H$, $CH_2SO_2$-Phenyl, $CO_2$-$C_{1-6}$-Alkyl, $OCF_3$, $SCF_3$, $CF_3$,

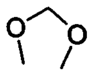, 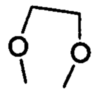,

$C_{1-6}$-Alkyl, Pyrolidinyl, Piperidinyl, Morpholinyl, Benzyloxy, Phenoxy, Phenyl, Pyridyl, Alkylaryl, Imidazolyl, Pyrazolyl, Thienyl oder Furyl; bedeutet.

**3.** Substituierte Nicotinamid-Derivate gemäß einem der Ansprüche 1 oder 2, worin p und q 1 bedeuten.

**4.** Substituierte Nicotinamid-Derivate gemäß einem der Ansprüche 1 oder 2, worin $R^1$ für tert-Butyl; Phenyl, Pyridyl, Thienyl, Furyl oder Cyclohexyl, unsubstituiert oder einfach oder mehrfach substituiert, steht.

**5.** Substituierte Nicotinamid-Derivate gemäß Anspruch 4, worin $R^1$ Cyclohexyl, oder Phenyl, unsubstituiert oder einfach oder mehrfach substituiert mit F, $CH_3$, Cl, Br, $CF_3$, $OCH_3$, $SCF_3$ oder $OCF_3$; Pyridyl, Thienyl oder Furyl, unsubstituiert oder einfach oder mehrfach substituiert mit $CH_3$; steht.

**6.** Substituierte Nicotinamid-Derivate gemäß einem der Ansprüche 1 oder 2, worin $R^2$ für $CH_3$ oder H steht, insbesondere für H.

**7.** Substituierte Nicotinamid-Derivate gemäß einem der Ansprüche 1 oder 2, worin $R^4$, $R^5$, $R^6$ und $R^7$ unabhängig voneinander für H oder $CH_3$ stehen, insbesondere für H.

**8.** Substituierte Nicotinamid-Derivate gemäß einem der Ansprüche 1 oder 2, worin n 0 oder 1, besonders bevorzugt 0 bedeutet.

**9.** Substituierte Nicotinamid-Derivate gemäß einem der Ansprüche 1 oder 2, worin $R^3$ Aryl oder Heteroaryl, unsubstituiert oder einfach oder mehrfach substituiert,
vorzugsweise
$R^3$ Phenyl unsubstituiert oder substituiert mit $CF_3$ oder $CH_3$ bedeutet.

**10.** Substituierte Nicotinamid-Derivate gemäß Anspruch 1 aus der Gruppe

| | |
|---|---|
| 1 | 2-(2-(Phenylsulfonyl)ethylthio)-N-(pyridin-2-ylmethyl)nicotinamid |
| 2 | 2-(2-(Phenylsulfonyl)ethylthio)-N-(pyridin-4-ylmethyl)nicotinamid |
| 3 | N-(3-Fluorophenethyl)-2-(2-(phenylsulfonyl)ethylthio)nicotinamid |
| 4 | N-Methyl-N-(3-methylbenzyl)-2-(2-(phenylsulfonyl)ethylthio)nicotinamid |
| 5 | N-(4-methylbenzyl)-2-(2-(phenylsulfonyl)ethylthio)nicotinamid |
| 6 | 2-(2-(Phenylsulfonyl)ethylthio)-N-(2-(trifluoromethyl)benzyl)nicotinamid |
| 7 | 2-(2-(Phenylsulfonyl)ethylthio)-N-(pyridin-3-ylmethyl)nicotinamid |
| 8 | N-(3,5-Difluorobenzyl)-2-(2-(phenylsulfonyl)ethylthio)nicotinamid |
| 9 | N-Methyl-N-phenethyl-2-(2-(phenylsulfonyl)ethylthio)nicotinamid |
| 10 | N-(3-Methoxybenzyl)-N-methyl-2-(2-(phenylsulfonyl)ethylthio)nicotinamid |
| 11 | N-(2-Fluorobenzyl)-2-(2-(phenylsulfonyl)ethylthio)nicotinamid |
| 12 | N-(3,4-Difluorobenzyl)-2-(2-(phenylsulfonyl)ethylthio)nicotinamid |
| 13 | N-(3-Bromobenzyl)-N-methyl-2-(2-(phenylsulfonyl)ethylthio)nicotinamid |
| 14 | N-(2-Methoxybenzyl)-2-(2-(phenylsulfonyt)ethylthio)nicotinamid |
| 15 | N-(3-Fluorobenzyl)-2-(2-(phenylsulfonyl)ethylthio)nicotinamid |
| 16 | N-(Furan-2-ylmethyl)-N-methyl-2-(2-(phenylsulfonyl)ethylthio)nicotinamid |
| 17 | N-(4-Methoxybenzyl)-2-(2-(phenylsulfonyl)ethylthio)nicotinamid |
| 18 | N-(2-Chlorobenzyl)-2-(2-(phenylsulfonyl)ethylthio)nicotinamid |
| 19 | N-(3,4-Dichlorobenzyl)-2-(2-(phenylsulfonyl)ethylthio)nicotinamid |
| 20 | N-(4-Fluorobenzyl)-2-(2-(phenylsulfonyl)ethylthio)nicotinamid |
| 21 | N-(2-Methoxyphenethyl)-2-(2-(phenylsulfonyl)ethylthio)nicotinamid |

(fortgesetzt)

| | |
|---|---|
| 22 | N-(2,6-Difluorobenzyl)-2-(2-(phenylsulfonyl)ethylthio)nicotinamid |
| 23 | N-(2-Methylbenzyl)-2-(2-(phenylsulfonyl)ethylthio)nicotinamid |
| 24 | N-(3,5-Dimethoxybenzyl)-2-(2-(phenylsulfonyl)ethylthio)nicotinamid |
| 25 | N-(3-Chlorobenzyl)-2-(2-(phenylsulfonyl)ethylthio)nicotinamid |
| 26 | N-(2,4-Dichlorobenzyl)-2-(2-(phenylsulfonyl)ethylthio)nicotinamid |
| 29 | N-(4-Chlorobenzyl)-2-(2-(phenylsulfonyl)ethylthio)nicotinamid |
| 30 | N-(2,3-Dichlorobenzyl)-2-(2-(phenylsulfonyl)ethylthio)nicotinamid |
| 31 | N-(4-Bromobenzyl)-N-methyl-2-(2-(phenylsulfonyl)ethylthio)nicotinamid |
| 32 | N-((1,3-Dioxolan-2-yl)methyl)-N-methyl-2-(2-(phenylsulfonyl)ethylthio)nicotinamid |
| 33 | N-Benzyl-N-methyl-2-(2-tosylethylthio)nicotinamid |
| 34 | N-(Pyridin-2-ylmethyl)-2-(2-tosylethylthio)nicotinamid |
| 35 | N-(Pyridin-4-ylmethyl)-2-(2-tosylethylthio)nicotinamid |
| 36 | N-(Thiophen-2-ylmethyl)-2-(2-tosylethylthio)nicotinamid |
| 37 | N-(3-Fluorophenethyl)-2-(2-tosylethylthio)nicotinamid |
| 38 | N-Methyl-N-(3-methylbenzyl)-2-(2-tosylethylthio)nicotinamid |
| 39 | N-(Furan-2-ylmethyl)-2-(2-tosylethylthio)nicotinamid |
| 40 | N-(Pyridin-3-ylmethyl)-2-(2-tosylethylthio)nicotinamid |
| 41 | N-(3.5-Difluorobenzyl)-2-(2-tosylethylthio)nicotinamid |
| 42 | N-(3-Methoxybenzyl)-N-methyl-2-(2-tosylethylthio)nicotinamid |
| 43 | N-(2-Fluorobenzyl)-2-(2-tosylethylthio)nicotinamid |
| 44 | N-(3-Methylbenzyl)-2-(2-tosylethylthio)nicotinamid |
| 45 | N-(3.4-Difluorobenzyl)-2-(2-tosylethylthio)nicotinamid |
| 46 | N-(3-Bromobenzyl)-N-methyl-2-(2-tosylethylthio)nicotinamid |
| 47 | N-(4-Methoxybenzyl)-2-(2-tosylethylthio)nicotinamid |
| 48 | N-(2-Chlorobenzyl)-2-(2-tosylethylthio)nicotinamid |
| 49 | N-(4-Fluorobenzyl)-2-(2-tosylethylthio)nicotinamid |
| 50 | N-(3, 5-Dimethoxybenzyl)-2-(2-tosylethylthio)nicotinamid |
| 51 | N-(3-Chlorobenzyl)-2-(2-tosylethylthio)nicotinamid |
| 52 | 2-(2-Tosylethylthio)-N-(3-(trifluoromethyl)benzyl)nicotinamid |
| 54 | N-Benzyl-2-(2-(phenylsulfonyl)ethylthio)nicotinamid |
| 55 | N-Benzyl-N-methyl-2-(2-(phenylsulfonyl)ethylthio)nicotinamid |
| 56 | N-(Cyclohexylmethyl)-2-(2-(phenylsulfonyl)-ethylthio)nicotinamid |
| 57 | 2-(2-(Phenylsulfonyl)ethylthio)-N-(1-(3-(trifluormethyl)phenyl)-ethyl)nicotinamid |
| 58 | N-(2-Cyclohexyl-ethyl)-2-(2-(phenylsulfonyl)ethylthio)nicotinamid |
| 59 | 2-(2-(Cyclohexylthio)ethylthio)-N-(thiophen-2-ylmethyl)nicotinamid |
| 60 | N-(Neopentyl)-2-(2-(phenylsulfonyl)ethylthio)nicotinamid |
| 61 | N-(5-Methylfuran-2-yl-methyl)-2-(2-(phenylsulfonyl)ethylthio)nicotinamid |
| 62 | N-(Furan-2-yl-methyl)-2-(2-(phenylsulfonyl)ethylthio)nicotinamid |
| 63 | 2-(2-(Phenylsulfonyl)ethylthio)-N-(tetrahydro-2H-pyran-4-yl-methyl)nicotinamid |
| 64 | 2-(2-(Phenylsulfonyl)ethylthio)-N-(4-(trifluormethylthio)benzyl)nicotinamid |
| 65 | 2-(2-(Phenylsulfonyl)ethylthio)-N-(3-tolylmethyl)nicotinamid |
| 66 | R)-N-(1-Cyclohexyl-ethyl)-2-(2-(phenylsulfonyl)ethylthio)nicotinamid |
| 67 | N-(1-(3,4-Dimethylphenyl)ethyl)-2-(2-(phenylsulfonyl)ethylthio)nicotinamid |
| 68 | N-(1-Thiophen-2-ylethyl)-2-(2-(phenylsulfonyl)ethylthio)nicotinamid |
| 69 | N-(1-(3,5-Dimethylphenyl)methyl)-2-(2-(phenylsulfonyl)ethylthio)nicotinamid |
| 70 | N-(Cyclohexylmethyl)-2-(2-(3-trifluormethyl-phenylsulfonyl)ethylthio)nicotinamid |
| 71 | (S)-N-(1-Cyclohexyl-ethyl)-2-(2-(phenylsulfonyl)ethylthio)nicotinamid |
| 72 | N-(1-(3,5-Dimethylphenyl)ethyl)-2-(2-(phenylsulfonyl)ethylthio)nicotinamid |
| 73 | N-(Thiophen-2-ylmethyl)-2-(2-(3-(trifluormethyl)phenylthio)ethylthio)nicotinamid |
| 74 | N-(Cyclopentylmethyl)-2-(2-(phenylsulfonyl)ethylthio)nicotinamid |
| 75 | N-(Cyclobutylmethyl)-2-(2-(phenylsulfonyl)ethylthio)nicotinamid |

(fortgesetzt)

| | |
|---|---|
| 76 | N-((1,4-Dioxan-2-yl)methyl)-2-(2-(phenylsulfonyl)ethylthio)nicotinamid |
| 77 | 2-(2-(Phenylsulfonyl)ethylthio)-N-(4-(pyridin-2-yloxy)benzyl)nicotinamid |
| 78 | N-(2-Methylbutyl)-2-(2-(phenylsulfonyl)ethylthio)nicotinamid |
| 79 | N-(2-ethylbutyl)-2-(2-(phenylsulfonyl)ethylthio)nicotinamid |
| 80 | N-(Cyclopropylmethyl)-2-(2-(phenylsulfonyl)ethylthio)nicotinamid |
| 81 | N-(3-(2-Methoxyethoxy)propyl)-2-(2-(phenylsulfonyl)ethylthio)nicotinamid |
| 82 | 2-(2-(Phenylsulfonyl)ethylthio)-N-(1-(4-(trifluormethylthio)phenyl)ethyl)nicotinamid |
| 83 | N-(3-(1H-Pyrazol-1-yl)benzyl)-2-(2-(phenylsulfonyl)ethylthio)nicotinamid |
| 84 | N-((2,3-Dihydrobenzofuran-5-yl)methyl)-2-(2-(phenylsulfonyl)ethylthio)nicotinamid |
| 85 | N-(4-Phenoxybenzyl)-2-(2-(phenylsulfonyl)ethylthio)nicotinamid |
| 86 | N-(((1R,2S,5R)-6,6-dimethylbicyclo[3.1.1]heptan-2-yl)methyl)-2-(2-(phenylsulfonyl)-ethylthio)nicotinamid |
| 87 | N-(Thiophen-2-ylmethyl)-2-(2-(3-(trifluoromethyl)phenylsulfonyl)ethylthio)nicotinamid |
| 88 | 2-(2-(Phenylsulfonyl)ethylthio)-N-(3-(trifluoromethyl)benzyl)nicotinamid |
| 93 | N-isobutyl-2-(2-(phenylsulfonyl)ethylthio)nicotinamid |
| 94 | 2-[2-(Benzolsulfonyl)ethylthio]-N-(2-tetrahydropyranylmethyl)-nicotinamid |
| 95 | 2-[2-(Benzolsulfonyl)ethylthio]-N-[(5-methyl-2-thienyl)methyl]-nicotinamid |
| 96 | 2-[2-(Benzolsulfonyl)ethylthio]-N-[(4-methyl-2-thienyl)methyl]-nicotinamid |
| 97 | N-(1-Adamantylmethyl)-2-[2-(benzolsulfonyl)ethylthio]-nicotinamid |
| 98 | 2-[2-(Benzolsulfonyl)ethylthio]-N-[(3-morpholinophenyl)methyl]-nicotinamid |
| 99 | 2-[2-(4-Chlorphenyl)sulfonylethylthio]-N-(2-thienylmethyl)-nicotinamid |
| 100 | 2-[2-(4-Fluorphenyl)sulfonylethylthio]-N-(2-thienylmethyl)-nicotinamid |
| 101 | N-(2-Thienylmethyl)-2-[2-[3-(trifluormethoxy)phenyl]sulfonylethylthio]-nicotinamid |
| 102 | N-(2-Thienylmethyl)-2-[2-[4-(trifluormethyl)phenyl]sulfonylethylthio]-nicotinamid |
| 103 | N-(2-Thienylmethyl)-2-[2-[4-(trifluormethoxy)phenyl]sulfonylethylthio]-nicotinamid |
| 104 | 2-[2-(m-Tolylsulfonyl)ethylthio]-N-(2-thienylmethyl)-nicotinamid |
| 105 | 2-[2-(m-Tolylthio)ethylthio]-N-(2-thienylmethyl)-nicotinamid |
| 106 | 2-[2-(3-Fluorphenyl)sulfonylethylthio]-N-(2-thienylmethyl)-nicotinamid |
| 107 | 2-[2-(Benzolsulfonyl)ethylthio]-N-(3,3-dimethylbutyl)-nicotinamid |
| 108 | 2-[2-(Benzolsulfonyl)ethylthio]-N-(2-benzothiophenylmethyl)-nicotinamid |
| 109 | 2-[2-(Phenylthio)ethylthio]-N-(2-thienylmethyl)-nicotinamid |
| 110 | 2-[2-(Benzolsulfinyl)ethylthio]-N-(2-thienylmethyl)-nicotinamid |
| 111 | 2-(2-Cyclohexylsulfonylethylthio)-N-(2-thienylmethyl)-nicotinamid |
| 112 | N-(2-Thienylmethyl)-2-[2-[[2-(trifluormethyl)phenyl]thio]ethylthio]-nicotinamid |
| 113 | N-(2-Thienylmethyl)-2-[2-[2-(trifluormethyl)phenyl]sulfinylethylthio]-nicotinamid |
| 114 | N-(2-Thienylmethyl)-2-[2-[2-(trifluormethyl)phenyl]sulfonylethylthio]-nicotinamid |
| 115 | 2-[2-(Benzolsulfonyl)ethylthio]-N-[(5-chlor-2-thienyl)methyl]-nicotinamid |
| 116 | 2-[2-(2-Fluorphenyl)sulfonylethylthio]-N-(2-thienylmethyl)-nicotinamid |
| 117 | 2-[2-[3,5-bis(Trifluormethyl)phenyl]sulfonylethylthio]-N-(2-thienylmethyl)-nicotinamid |
| 118 | 2-[2-(3-Methoxyphenyl)sulfonylethylthio]-N-(2-thienylmethyl)-nicotinamid |
| 119 | 2-[2-(4-Methoxyphenyl)sulfonylethylthio]-N-(2-thienylmethyl)-nicotinamid |
| 120 | 2-[2-(Benzolsulfonyl)ethylthio]-N-(4-tetrahydrothiopyranylmethyl)-nicotinamid |
| 121 | 2-[2-(4-Ethylphenyl)sulfonylethylthio]-N-(2-thienylmethyl)-nicotinamid |
| 122 | N-(2-Thienylmethyl)-2-[2-[[4-(trifluormethyl)phenyl]thio]ethylthio]-nicotinamid |
| 123 | 2-[2-(o-Tolylthio)ethylthio]-N-(2-thienylmethyl)-nicotinamid |
| 124 | 2-[2-[(3-Fluorphenyl)thio]ethylthio]-N-(2-thienylmethyl)-nicotinamid |
| 125 | 2-[2-[(3,4-Difluorphenyl)thio]ethylthio]-N-(2-thienylmethyl)-nicotinamid |
| 126 | 2-[2-[(2,4-Difluorphenyl)thio]ethylthio]-N-(2-thienylmethyl)-nicotinamid |
| 127 | 2-[2-(Benzolsulfonyl)ethylthio]-N-[2-(2-thienyl)ethyl]-nicotinamid |
| 128 | 2-[2-(Benzolsulfonyl)ethylthio]-N-phenthyl-nicotinamid |
| 129 | 2-[2-(Benzolsulfonyl)ethylthio]-N-(3-phenylpropyl)-nicotinamid |
| 130 | 2-[2-(3,4-Difluorphenyl)sulfonylethylthio]-N-(2-thienylmethyl)-nicotinamid |

(fortgesetzt)

| | |
|---|---|
| 131 | 2-[2-(2,4-Difluorphenyl)sulfonylethylthio]-N-(2-thienylmethyl)-nicotinamid |
| 132 | 2-[2-[(2-Fluorphenyl)thio]ethylthio]-N-(2-thienylmethyl)-nicotinamid |
| 133 | 2-[2-[(4-Fluorphenyl)thio]ethylthio]-N-(2-thienylmethyl)-nicotinamid |
| 134 | 2-[2-[(4-Chlorphenyl)thio]ethylthioj-N-(2-thienylmethyl)-nicotinamid |
| 135 | 2-[2-(p-Tolylthio)ethylthio]-N-(2-thienylmethyl)-nicotinamid |
| 136 | 2-[2-(Benzolsulfonyl)ethylthio]-N-isopentyl-nicotinamid |
| 137 | 2-[2-(Benzolsulfonyl)ethylthio]-N-(2-cyclopropylethyl)-nicotinamid |
| 138 | 2-[2-(Benzolsulfonyl)ethylthio]-N-(2-cyclopentylethyl)-nicotinamid |
| 139 | N-(3,3-Dimethylbutyl)-2-[2-[3-(trifluormethyl)phenyl]sulfonylethylthio]-nicotinamid |
| 140 | N-(Cyclopentylmethyl)-2-[2-[3-(trifluormethyl)phenyl]sulfonylethylthio]-nicotinamid |
| 141 | 2-[2-(Benzolsulfonyl)ethylthio]-6-methyl-N-(2-thienylmethyl)-nicotinamid |
| 142 | 2-[2-(Benzolsulfonyl)ethylthio]-N-(2-thienylmethyl)-6-(trifluormethyl)-nicotinamid |
| 143 | 2-[2-(Benzolsulfonyl)ethylthio]-6-fluor-N-(2-thienylmethyl)-nicotinamid |
| 144 | 2-[2-(Benzolsulfonyl)ethylthio]-N-[(3-methylcyclohexyl)methyl]-nicotinamid |
| 145 | 2-[2-(Benzolsulfonyl)ethylthio]-N-(cycloheptylmethyl)-nicotinamid |
| 146 | 2-[2-(Benzolsulfonyl)ethylthio]-N-[(2-methylcyclohexyl)methyl]-nicotinamid |
| 147 | 2-[2-(Benzolsulfonyl)ethylthio]-N-[(4-methylcyclohexyl)methyl]-nicotinamid |
| 148 | 2-[2-(Benzolsulfonyl)ethylthio]-5-fluor-N-(2-thienylmethyl)-nicotinamid |
| 149 | 2-[2-(Benzolsulfonyl)ethylthio]-5-methyl-N-(2-thienylmethyl)-nicotinamid |
| 150 | 2-[2-(Benzolsulfonyl)ethylthio]-N-(2-thienylmethyl)-5-(trifluormethyl)-nicotinamid. |

**11.** Arzneimittel enthaltend wenigstens eine erfindungsgemäße substituierte Nicotinamid-Verbindung der allgemeinen Formel I gemäß Anspruch 1, worin
n = 0, 1 oder 2
p = 0 oder 1
q = 0 oder 1
$R^1$ Aryl oder Heteroaryl, unsubstituiert oder einfach oder mehrfach substituiert; $C_{1-6}$-Alkyl, $C_{3-10}$-Cycloalkyl oder Heterocyclyl, unsubstituiert oder einfach oder mehrfach substituiert;
$R^2$ H; $C_{1-6}$-Alkyl, unsubstituiert oder einfach oder mehrfach substituiert;
$R^3$ Aryl oder Heteroaryl, unsubstituiert oder einfach oder mehrfach substituiert; $C_{1-6}$-Alkyl oder $C_{3-10}$-Cycloalkyl, jeweils unsubstituiert oder einfach oder mehrfach substituiert;
$R^4$, $R^5$, $R^6$ und $R^7$ unabhängig voneinander H; $C_{1-6}$-Alkyl, unsubstituiert oder einfach oder mehrfach substituiert,
$R^8$, $R^9$ und $R^{10}$ unabhängig voneinander H, F, Cl, Br, O-$C_{1-6}$-Alkyl, $CF_3$, $OCF_3$, $SCF_3$, $C_{1-6}$-Alkyl;
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren sowie ggf. einen oder mehrere pharmazeutisch verträgliche Hilfsstoffe.

**12.** Arzneimittel gemäß Anspruch 11 aus der Gruppe

| | |
|---|---|
| 89 | 2-(2-(Phenylsulfonyl)ethylthio)-N-(thiophen-2-ylmethyl)nicotinamid |
| 90 | N-(Pyridin-2-ylmethyl)-2-(2-(3-(trifluoromethyl)phenylsulfonyl)ethylthio)nicotinamid |
| 91 | N-(Pyridin-2-ylmethyl)-2-(2-(5-(trifluoromethyl)pyridin-2-ylsulfonyl)ethylthio)-nicotinamid |
| 92 | N-(Thiophen-2-ylmethyl)-2-(2-(5-(trifluoromethyl)pyridin-2-ylsulfonyl)ethylthio)-nicotinamid. |

**13.** Verwendung von substituierten Nicotinamid-Verbindungen gemäß Anspruch 1 oder 11 zur Herstellung eines Arzneimittels zur Behandlung von Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz.

**14.** Verwendung von Verbindungen gemäß Anspruch 1 oder 11 zur Herstellung eines Arzneimittels zur Behandlung von Epilepsie, Migräne, Angstzuständen und Harninkontinenz.

## Claims

1. Substituted nicotinamide compounds of the general formula I

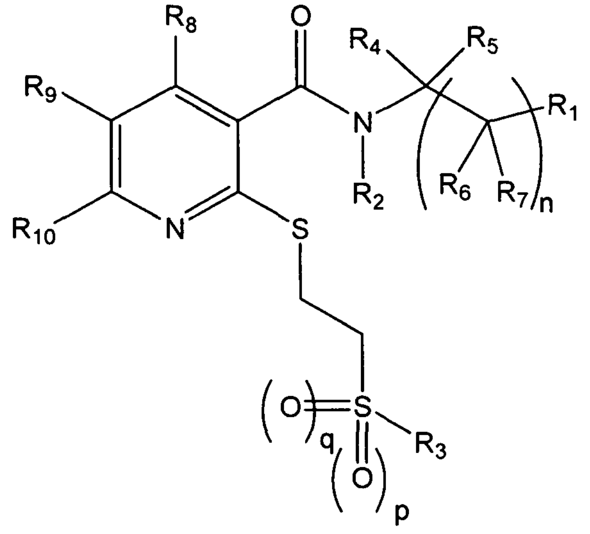

wherein

n = 0, 1 or 2
p = 0 or 1
q=0or1,
$R^1$ denotes aryl or heteroaryl, unsubstituted or mono- or poly-substituted; $C_{1-6}$-alkyl, $C_{3-10}$-cycloalkyl or heterocyclyl, unsubstituted or mono- or poly-substituted;
$R^2$ denotes H; $C_{1-6}$-alkyl, unsubstituted or mono- or poly-substituted;
$R^3$ denotes aryl or heteroaryl, unsubstituted or mono- or poly-substituted; $C_{1-6}$-alkyl or $C_{3-10}$-cycloalkyl, in each case unsubstituted or mono- or poly-substituted;
$R^4$, $R^5$, $R^6$ and $R^7$ independently of one another denote H; $C_{1-6}$-alkyl, unsubstituted or mono- or poly-substituted;
$R^8$, $R^9$ and $R^{10}$ independently of one another denote H, F, Cl, Br, O-$C_{1-6}$-alkyl, $CF_3$, $OCF_3$, $SCF_3$, $C_{1-6}$-alkyl;
with the proviso that when $R^3$ denotes 3-trifluoromethylphenyl or 4-trifluoromethyl-2-pyridyl, $R^2$, $R^4$ and $R^5$ denote H and n denotes 0, then $R^1$ does not denote 2-pyridyl or 2-thienyl
and
when $R^3$ denotes phenyl or methyl, $R^2$, $R^4$ and $R^5$ denote H and n denotes 0, then $R^1$ does not denote 2-thienyl;
wherein $C_{1-6}$-alkyl includes acyclic saturated or unsaturated hydrocarbon radicals and wherein cycloalkyl and $C_{3-10}$-cycloalkyl include saturated or unsaturated but not aromatic cyclic hydrocarbons;
in the form of the racemate; of the enantiomers, diastereoisomers, mixtures of the enantiomers or diastereoisomers or of an individual enantiomer or diastereoisomer; of the bases and/or salts of physiologically acceptable acids.

2. Substituted nicotinamide derivatives according to claim 1, wherein
"alkyl substituted", "heterocyclyl substituted" and "cycloalkyl substituted" represents the replacement of a hydrogen radical by F, Cl, Br, I, -CN, $NH_2$, NH-$C_{1-6}$-alkyl, NH-$C_{1-6}$-alkyl-OH, $C_{1-6}$-alkyl, N($C_{1-6}$-alkyl)$_2$, N($C_{1-6}$-alkyl-OH)$_2$, $NO_2$, SH, S-$C_{1-6}$-alkyl, S-benzyl, O-$C_{1-6}$-alkyl, OH, O-$C_{1-6}$-alkyl-OH, =O, O-benzyl, C(=O)$C_{1-6}$-alkyl, $CO_2H$, $CO_2$-$C_{1-6}$-alkyl, phenyl, phenoxy, morpholinyl, piperidinyl, pyrrolidinyl or benzyl;
and "aryl substituted" and "heteroaryl substituted" represents the replacement one or more times, e.g. two, three or four times, of one or more hydrogen atoms of the ring system by F, Cl, Br, I, CN, $NH_2$, NH-$C_{1-6}$-alkyl, NH-$C_{1-6}$-alkyl-OH, N($C_{1-6}$-alkyl)$_2$, N($C_{1-6}$-alkyl-OH)$_2$, $NO_2$, SH, S-$C_{1-6}$-alkyl, OH, O-$C_{1-6}$-alkyl, O-$C_{1-6}$alkyl-OH, C(=O)$C_{1-6}$-alkyl, C (=O)NH$C_{1-6}$-alkyl; o-pyridyl; C(=O)-aryl; C(=O)-N-morpholine; C(=O)-piperidine; (C=O)-pyrrolidine; (C=O)-piperazine; $NHSO_2C_{1-6}$-alkyl, $NHCOC_{1-6}$-alkyl, $CO_2H$, $CH_2SO_2$-phenyl, $CO_2$-$C_{1-6}$-alkyl, $OCF_3$,
$SCF_3$, $CF_3$,

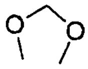, 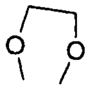,

$C_{1-6}$-alkyl, pyrrolidinyl, piperidinyl, morpholinyl, benzyloxy, phenoxy, phenyl, pyridyl, alkylaryl, imidazolyl, pyrazolyl, thienyl or furyl.

**3.** Substituted nicotinamide derivatives according to either claim 1 or claim 2, wherein p and q denote 1.

**4.** Substituted nicotinamide derivatives according to either claim 1 or claim 2, wherein $R^1$ represents tert-butyl; phenyl, pyridyl, thienyl, furyl or cyclohexyl, unsubstituted or mono- or poly-substituted.

**5.** Substituted nicotinamide derivatives according to claim 4, wherein $R^1$ represents cyclohexyl, or phenyl, unsubstituted or mono- or poly-substituted by F, $CH_3$, Cl, Br, $CF_3$, $OCH_3$, $SCF_3$ or $OCF_3$; pyridyl, thienyl or furyl, unsubstituted or mono- or poly-substituted by $CH_3$.

**6.** Substituted nicotinamide derivatives according to either claim 1 or claim 2, wherein $R^2$ represents $CH_3$ or H, in particular H.

**7.** Substituted nicotinamide derivatives according to either claim 1 or claim 2, wherein $R^4$, $R^5$, $R^6$ and $R^7$ independently of one another represent H or $CH_3$, in particular H.

**8.** Substituted nicotinamide derivatives according to either claim 1 or claim 2, wherein n denotes 0 or 1, particularly preferably 0.

**9.** Substituted nicotinamide derivatives according to either claim 1 or claim 2, wherein $R^3$ denotes aryl or heteroaryl, unsubstituted or mono- or poly-substituted,
preferably
$R^3$ denotes phenyl unsubstituted or substituted by $CF_3$ or $CH_3$.

**10.** Substituted nicotinamide derivatives according to claim 1 from the group

| | |
|---|---|
| 1 | 2-(2-(phenylsulfonyl)ethylthio)-N-(pyridin-2-ylmethyl)nicotinamide |
| 2 | 2-(2-(phenylsulfonyl)ethylthio)-N-(pyridin-4-ylmethyl)nicotinamide |
| 3 | N-(3-fluorophenethyl)-2-(2-(phenylsulfonyl)ethylthio)nicotinamide |
| 4 | N-methyl-N-(3-methylbenzyl)-2-(2-(phenylsulfonyl)ethylthio)nicotinamide |
| 5 | N-(4-methylbenzyl)-2-(2-(phenylsulfonyl)ethylthio)nicotinamide |
| 6 | 2-(2-(phenylsulfonyl)ethylthio)-N-(2-(trifluoromethyl)benzyl)nicotinamide |
| 7 | 2-(2-(phenylsulfonyl)ethylthio)-N-(pyridin-3-ylmethyl)nicotinamide |
| 8 | N-(3,5-difluorobenzyl)-2-(2-(phenylsulfonyl)ethylthio)nicotinamide |
| 9 | N-methyl-N-phenethyl-2-(2-(phenylsulfonyl)ethylthio)nicotinamide |
| 10 | N-(3-methoxybenzyl)-N-methyl-2-(2-(phenylsulfonyl)ethylthio)nicotinamide |
| 11 | N-(2-fluorobenzyl)-2-(2-(phenylsulfonyl)ethylthio)nicotinamide |
| 12 | N-(3,4-difluorobenzyl)-2-(2-(phenylsulfonyl)ethylthio)nicotinamide |
| 13 | N-(3-bromobenzyl)-N-methyl-2-(2-(phenylsulfonyl)ethylthio)nicotinamide |
| 14 | N-(2-methoxybenzyl)-2-(2-(phenylsulfonyl)ethylthio)nicotinamide |
| 15 | N-(3-fluorobenzyl)-2-(2-(phenylsulfonyl)ethylthio)nicotinamide |
| 16 | N-(furan-2-ylmethyl)-N-methyl-2-(2-(phenylsulfonyl)ethylthio)nicotinamide |
| 17 | N-(4-methoxybenzyl)-2-(2-(phenylsulfonyl)ethylthio)nicotinamide |
| 18 | N-(2-chlorobenzyl)-2-(2-(phenylsulfonyl)ethylthio)nicotinamide |
| 19 | N-(3,4-dichlorobenzyl)-2-(2-(phenylsulfonyl)ethylthio)nicotinamide |
| 20 | N-(4-fluorobenzyl)-2-(2-(phenylsulfonyl)ethylthio)nicotinamide |
| 21 | N-(2-methoxyphenethyl)-2-(2-(phenylsulfonyl)ethylthio)nicotinamide |

(continued)

| | |
|---|---|
| 22 | N-(2,6-difluorobenzyl)-2-(2-(phenylsulfonyl)ethylthio)nicotinamide |
| 23 | N-(2-methylbenzyl)-2-(2-(phenylsulfonyl)ethylthio)nicotinamide |
| 24 | N-(3,5-dimethoxybenzyl)-2-(2-(phenylsulfonyl)ethylthio)nicotinamide |
| 25 | N-(3-chlorobenzyl)-2-(2-(phenylsulfonyl)ethylthio)nicotinamide |
| 26 | N-(2,4-dichlorobenzyl)-2-(2-(phenylsulfonyl)ethylthio)nicotinamide |
| 29 | N-(4-chlorobenzyl)-2-(2-(phenylsulfonyl)ethylthio)nicotinamide |
| 30 | N-(2,3-dichlorobenzyl)-2-(2-(phenylsulfonyl)ethylthio)nicotinamide |
| 31 | N-(4-bromobenzyl)-N-methyl-2-(2-(phenylsulfonyl)ethylthio)nicotinamide |
| 32 | N-((1,3-dioxolan-2-yl)methyl)-N-methyl-2-(2-(phenylsulfonyl)ethylthio)-nicotinamide |
| 33 | N-benzyl-N-methyl-2-(2-tosylethylthio)nicotinamide |
| 34 | N-(pyridin-2-ylmethyl)-2-(2-tosylethylthio)nicotinamide |
| 35 | N-(pyridin-4-ylmethyl)-2-(2-tosylethylthio)nicotinamide |
| 36 | N-(thiophen-2-ylmethyl)-2-(2-tosylethylthio)nicotinamide |
| 37 | N-(3-fluorophenethyl)-2-(2-tosylethylthio)nicotinamide |
| 38 | N-methyl-N-(3-methylbenzyl)-2-(2-tosylethylthio)nicotinamide |
| 39 | N-(furan-2-ylmethyl)-2-(2-tosylethylthio)nicotinamide |
| 40 | N-(pyridin-3-ylmethyl)-2-(2-tosylethylthio)nicotinamide |
| 41 | N-(3,5-difluorobenzyl)-2-(2-tosylethylthio)nicotinamide |
| 42 | N-(3-methoxybenzyl)-N-methyl-2-(2-tosylethylthio)nicotinamide |
| 43 | N-(2-fluorobenzyl)-2-(2-tosylethylthio)nicotinamide |
| 44 | N-(3-methylbenzyl)-2-(2-tosylethylthio)nicotinamide |
| 45 | N-(3,4-difluorobenzyl)-2-(2-tosylethylthio)nicotinamide |
| 46 | N-(3-bromobenzyl)-N-methyl-2-(2-tosylethylthio)nicotinamide |
| 47 | N-(4-methoxybenzyl)-2-(2-tosylethylthio)nicotinamide |
| 48 | N-(2-chlorobenzyl)-2-(2-tosylethylthio)nicotinamide |
| 49 | N-(4-fluorobenzyl)-2-(2-tosylethylthio)nicotinamide |
| 50 | N-(3,5-dimethoxybenzyl)-2-(2-tosylethylthio)nicotinamide |
| 51 | N-(3-chlorobenzyl)-2-(2-tosylethylthio)nicotinamide |
| 52 | 2-(2-tosylethylthio)-N-(3-(trifluoromethyl)benzyl)nicotinamide |
| 54 | N-benzyl-2-(2-(phenylsulfonyl)ethylthio)nicotinamide |
| 55 | N-benzyl-N-methyl-2-(2-(phenylsulfonyl)ethylthio)nicotinamide |
| 56 | N-(cyclohexylmethyl)-2-(2-(phenylsulfonyl)ethylthio)nicotinamide |
| 57 | 2-(2-(phenylsulfonyl)ethylthio)-N-(1-(3-(trifluoromethyl)phenyl)ethyl)-nicotinamide |
| 58 | N-(2-cyclohexylethyl)-2-(2-(phenylsulfonyl)ethylthio)nicotinamide |
| 59 | 2-(2-(cyclohexylthio)ethylthio)-N-(thiophen-2-ylmethyl)nicotinamide |
| 60 | N-(neopentyl)-2-(2-(phenylsulfonyl)ethylthio)nicotinamide |
| 61 | N-(5-methylfuran-2-ylmethyl)-2-(2-(phenylsulfonyl)ethylthio)nicotinamide |
| 62 | N-(furan-2-ylmethyl)-2-(2-(phenylsulfonyl)ethylthio)nicotinamide |
| 63 | 2-(2-(phenylsulfonyl)ethylthio)-N-(tetrahydro-2H-pyran-4-ylmethyl)-nicotinamide |
| 64 | 2-(2-(phenylsulfonyl)ethylthio)-N-(4-(trifluoromethylthio)benzyl)nicotinamide |
| 65 | 2-(2-(phenylsulfonyl)ethylthio)-N-(3-tolylmethyl)nicotinamide |
| 66 | (R)-N-(1-cyclohexylethyl)-2-(2-(phenylsulfonyl)ethylthio)nicotinamide |
| 67 | N-(1-(3,4-dimethylphenyl)ethyl)-2-(2-(phenylsulfonyl)ethylthio)nicotinamide |
| 68 | N-(1-thiophen-2-ylethyl)-2-(2-(phenylsulfonyl)ethylthio)nicotinamide |
| 69 | N-(1-(3,5-dimethylphenyl)methyl)-2-(2-(phenylsulfonyl)ethylthio)nicotinamide |
| 70 | N-(cyclohexylmethyl)-2-(2-(3-trifluoromethylphenylsulfonyl)ethylthio)-nicotinamide |
| 71 | (S)-N-(1-cyclohexylethyl)-2-(2-(phenylsulfonyl)ethylthio)nicotinamide |
| 72 | N-(1-(3,5-dimethylphenyl)ethyl)-2-(2-(phenylsulfonyl)ethylthio)nicotinamide |
| 73 | N-(thiophen-2-ylmethyl)-2-(2-(3-(trifluoromethyl)phenylthio)ethylthio)-nicotinamide |
| 74 | N-(cyclopentylmethyl)-2-(2-(phenylsulfonyl)ethylthio)nicotinamide |
| 75 | N-(cyclobutylmethyl)-2-(2-(phenylsulfonyl)ethylthio)nicotinamide |

(continued)

| | |
|---|---|
| 76 | N-((1,4-dioxan-2-yl)methyl)-2-(2-(phenylsulfonyl)ethylthio)nicotinamide |
| 77 | 2-(2-(phenylsulfonyl)ethylthio)-N-(4-(pyridin-2-yloxy)benzyl)nicotinamide |
| 78 | N-(2-methylbutyl)-2-(2-(phenylsulfonyl)ethylthio)nicotinamide |
| 79 | N-(2-ethylbutyl)-2-(2-(phenylsulfonyl)ethylthio)nicotinamide |
| 80 | N-(cyclopropylmethyl)-2-(2-(phenylsulfonyl)ethylthio)nicotinamide |
| 81 | N-(3-(2-methoxyethoxy)propyl)-2-(2-(phenylsulfonyl)ethylthio)nicotinamide |
| 82 | 2-(2-(phenylsulfonyl)ethylthio)-N-(1-(4-(trifluoromethylthio)phenyl)ethyl)-nicotinamide |
| 83 | N-(3-(1H-pyrazol-1-yl)benzyl)-2-(2-(phenylsulfonyl)ethylthio)nicotinamide |
| 84 | N-((2,3-dihydrobenzofuran-5-yl)methyl)-2-(2-(phenylsulfonyl)ethylthio)-nicotinamide |
| 85 | N-(4-phenoxybenzyl)-2-(2-(phenylsulfonyl)ethylthio)nicotinamide |
| 86 | N-(((1R,2S,5R)-6,6-dimethylbicyclo[3.1.1]heptan-2-yl)methyl)-2-(2-(phenyl-sulfonyl)ethylthio)nicotinamide |
| 87 | N-(thiophen-2-ylmethyl)-2-(2-(3-(trifluoromethyl)phenylsulfonyl)ethylthio)-nicotinamide |
| 88 | 2-(2-(phenylsulfonyl)ethylthio)-N-(3-(trifluoromethyl)benzyl)nicotinamide |
| 93 | N-isobutyl-2-(2-(phenylsulfonyl)ethylthio)nicotinamide |
| 94 | 2-[2-(benzenesulfonyl)ethylthio]-N-(2-tetrahydropyranylmethyl)-nicotinamide |
| 95 | 2-[2-(benzenesulfonyl)ethylthio]-N-[(5-methyl-2-thienyl)methyl]-nicotinamide |
| 96 | 2-[2-(benzenesulfonyl)ethylthio]-N-[(4-methyl-2-thienyl)methyl]-nicotinamide |
| 97 | N-(1-adamantylmethyl)-2-[2-(benzenesulfonyl)ethylthio]-nicotinamide |
| 98 | 2-[2-(benzenesulfonyl)ethylthio]-N-[(3-morpholinophenyl)methyl]-nicotinamide |
| 99 | 2-[2-(4-chlorophenyl)sulfonylethylthio]-N-(2-thienylmethyl)-nicotinamide |
| 100 | 2-[2-(4-fluorophenyl)sulfonylethylthio]-N-(2-thienylmethyl)-nicotinamide |
| 101 | N-(2-thienylmethyl)-2-[2-[3-(trifluoromethoxy)phenyl]sulfonylethylthio]-nicotinamide |
| 102 | N-(2-thienylmethyl)-2-[2-[4-(trifluoromethyl)phenyl]sulfonylethylthio]-nicotinamide |
| 103 | N-(2-thienylmethyl)-2-[2-[4-(trifluoromethoxy)phenyl]sulfonylethylthio]-nicotinamide |
| 104 | 2-[2-(m-tolylsulfonyl)ethylthio]-N-(2-thienylmethyl)-nicotinamide |
| 105 | 2-[2-(m-tolylthio)ethylthio]-N-(2-thienylmethyl)-nicotinamide |
| 106 | 2-[2-(3-fluorophenyl)sulfonylethylthio]-N-(2-thienylmethyl)-nicotinamide |
| 107 | 2-[2-(benzenesulfonyl)ethylthio]-N-(3,3-dimethylbutyl)-nicotinamide |
| 108 | 2-[2-(benzenesulfonyl)ethylthio]-N-(2-benzothiophenylmethyl)-nicotinamide |
| 109 | 2-[2-(phenylthio)ethylthio]-N-(2-thienylmethyl)-nicotinamide |
| 110 | 2-[2-(benzenesulfinyl)ethylthio]-N-(2-thienylmethyl)-nicotinamide |
| 111 | 2-(2-cyclohexylsulfonylethylthio)-N-(2-thienylmethyl)-nicotinamide |
| 112 | N-(2-thienylmethyl)-2-[2-[[2-(trifluoromethyl)phenyl]thio]ethylthio]-nicotinamide |
| 113 | N-(2-thienylmethyl)-2-[2-[2-(trifluoromethyl)phenyl]sulfinylethylthio]-nicotinamide |
| 114 | N-(2-thienylmethyl)-2-[2-[2-(trifluoromethyl)phenyl]sulfonylethylthio]-nicotinamide |
| 115 | 2-[2-(benzenesulfonyl)ethylthio]-N-[(5-chloro-2-thienyl)methyl]-nicotinamide |
| 116 | 2-[2-(2-fluorophenyl)sulfonylethylthio]-N-(2-thienylmethyl)-nicotinamide |
| 117 | 2-[2-[3,5-bis(trifluoromethyl)phenyl]sulfonylethylthio]-N-(2-thienylmethyl)-nicotinamide |
| 118 | 2-[2-(3-methoxyphenyl)sulfonylethylthio]-N-(2-thienylmethyl)-nicotinamide |
| 119 | 2-[2-(4-methoxyphenyl)sulfonylethylthio]-N-(2-thienylmethyl)-nicotinamide |
| 120 | 2-[2-(benzenesulfonyl)ethylthio]-N-(4-tetrahydrothiopyranylmethyl)-nicotinamide |
| 121 | 2-[2-(4-ethylphenyl)sulfonylethylthio]-N-(2-thienylmethyl)-nicotinamide |
| 122 | N-(2-thienylmethyl)-2-[2-[[4-(trifluoromethyl)phenyl]thio]ethylthio]-nicotinamide |
| 123 | 2-[2-(o-tolylthio)ethylthio]-N-(2-thienylmethyl)-nicotinamide |
| 124 | 2-[2-[(3-fluorophenyl)thio]ethylthio]-N-(2-thienylmethyl)-nicotinamide |
| 125 | 2-[2-[(3,4-difluorophenyl)thio]ethylthio]-N-(2-thienylmethyl)-nicotinamide |
| 126 | 2-[2-[(2,4-difluorophenyl)thio]ethylthio]-N-(2-thienylmethyl)-nicotinamide |
| 127 | 2-[2-(benzenesulfonyl)ethylthio]-N-[2-(2-thienyl)ethyl]-nicotinamide |
| 128 | 2-[2-(benzenesulfonyl)ethylthio]-N-phenthyl-nicotinamide |
| 129 | 2-[2-(benzenesulfonyl)ethylthio]-N-(3-phenylpropyl)-nicotinamide |
| 130 | 2-[2-(3,4-difluorophenyl)sulfonylethylthio]-N-(2-thienylmethyl)-nicotinamide |

(continued)

| | |
|---|---|
| 131 | 2-[2-(2,4-difluorophenyl)sulfonylethylthio]-N-(2-thienylmethyl)-nicotinamide |
| 132 | 2-[2-[(2-fluorophenyl)thio]ethylthio]-N-(2-thienylmethyl)-nicotinamide |
| 133 | 2-[2-[(4-fluorophenyl)thio]ethylthio]-N-(2-thienylmethyl)-nicotinamide |
| 134 | 2-[2-[(4-chlorophenyl)thio]ethylthio]-N-(2-thienylmethyl)-nicotinamide |
| 135 | 2-[2-(p-tolylthio)ethylthio]-N-(2-thienylmethyl)-nicotinamide |
| 136 | 2-[2-(benzenesulfonyl)ethylthio]-N-isopentyl-nicotinamide |
| 137 | 2-[2-(benzenesulfonyl)ethylthio]-N-(2-cyclopropylethyl)-nicotinamide |
| 138 | 2-[2-(benzenesulfonyl)ethylthio]-N-(2-cyclopentylethyl)-nicotinamide |
| 139 | N-(3,3-dimethylbutyl)-2-[2-[3-(trifluoromethyl)phenyl]sulfonylethylthio]-nicotinamide |
| 140 | N-(cyclopentylmethyl)-2-[2-[3-(trifluoromethyl)phenyl]sulfonylethylthio]-nicotinamide |
| 141 | 2-[2-(benzenesulfonyl)ethylthio]-6-methyl-N-(2-thienylmethyl)-nicotinamide |
| 142 | 2-[2-(benzenesulfonyl)ethylthio]-N-(2-thienylmethyl)-6-(trifluoromethyl)-nicotinamide |
| 143 | 2-[2-(benzenesulfonyl)ethylthio]-6-fluoro-N-(2-thienylmethyl)-nicotinamide |
| 144 | 2-[2-(benzenesulfonyl)ethylthio]-N-[(3-methylcyclohexyl)methyl]-nicotinamide |
| 145 | 2-[2-(benzenesulfonyl)ethylthio]-N-(cycloheptylmethyl)-nicotinamide |
| 146 | 2-[2-(benzenesulfonyl)ethylthio]-N-[(2-methylcyclohexyl)methyl]-nicotinamide |
| 147 | 2-[2-(benzenesulfonyl)ethylthio]-N-[(4-methylcyclohexyl)methyl]-nicotinamide |
| 148 | 2-[2-(benzenesulfonyl)ethylthio]-5-fluoro-N-(2-thienylmethyl)-nicotinamide |
| 149 | 2-[2-(benzenesulfonyl)ethylthio]-5-methyl-N-(2-thienylmethyl)-nicotinamide |
| 150 | 2-[2-(benzenesulfonyl)ethylthio]-N-(2-thienylmethyl)-5-(trifluoromethyl)-nicotinamide. |

**11.** Medicament comprising at least one substituted nicotinamide compound of the general formula I according to claim 1, wherein
n = 0, 1 or 2
p = 0 or 1
q = 0 or 1,
$R^1$ denotes aryl or heteroaryl, unsubstituted or mono- or poly-substituted; $C_{1-6}$-alkyl, $C_{3-10}$-cycloalkyl or heterocyclyl, unsubstituted or mono- or poly-substituted;
$R^2$ denotes H; $C_{1-6}$-alkyl, unsubstituted or mono- or poly-substituted;
$R^3$ denotes aryl or heteroaryl, unsubstituted or mono- or poly-substituted; $C_{1-6}$-alkyl or $C_{3-10}$-cycloalkyl, in each case unsubstituted or mono- or poly-substituted;
$R^4$, $R^5$, $R^6$ and $R^7$ independently of one another denote H; $C_{1-6}$-alkyl, unsubstituted or mono- or poly-substituted;
$R^8$, $R^9$ and $R^{10}$ independently of one another denote H, F, Cl, Br, O-$C_{1-6}$-alkyl, $CF_3$, $OCF_3$, $SCF_3$, $C_{1-6}$-alkyl;
in the form of the racemate; of the enantiomers, diastereoisomers, mixtures of the enantiomers or diastereoisomers or of an individual enantiomer or diastereoisomer; of the bases and/or salts of physiologically acceptable acids, and optionally one or more pharmaceutically acceptable auxiliary substances.

**12.** Medicament according to claim 11 from the group

| | |
|---|---|
| 89 | 2-(2-(phenylsulfonyl)ethytthio)-N-(thiophen-2-ylmethyl)nicotinamide |
| 90 | N-(pyridin-2-ylmethyl)-2-(2-(3-(trifluoromethyl)phenylsulfonyl)ethylthio)-nicotinamide |
| 91 | N-(pyridin-2-ylmethyl)-2-(2-(5-(trifluoromethyl)pyridin-2-ylsulfonyl)ethylthio)-nicotinamide |
| 92 | N-(thiophen-2-ylmethyl)-2-(2-(5-(trifluoromethyl)pyridin-2-ylsulfonyl)ethyl-thio)nicotinamide. |

**13.** Use of substituted nicotinamide compounds according to claim 1 or 11 in the preparation of a medicament for the treatment of pain, preferably of pain selected from the group consisting of acute pain, chronic pain, neuropathic pain, muscular pain and inflammatory pain.

**14.** Use of compounds according to claim 1 or 11 in the preparation of a medicament for the treatment of epilepsy, migraine, anxiety states and urinary incontinence.

## Revendications

1. Composés de nicotinamide substitués de formule générale I,

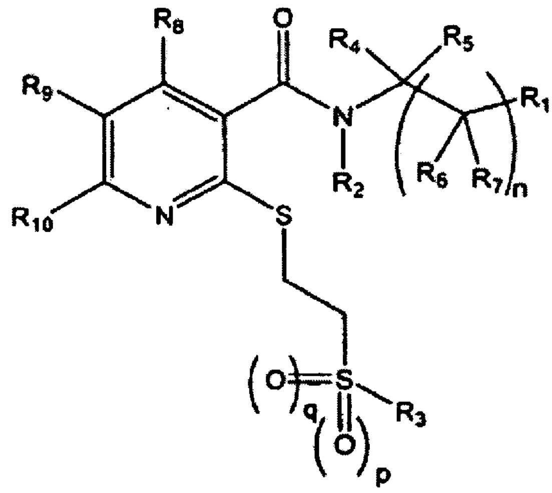

dans laquelle

n = 0, 1 ou 2
p = 0 ou 1
q = 0 ou 1
$R^1$ représente un groupe aryle ou hétéroaryle, non substitué ou substitué une ou plusieurs fois ; alkyle en $C_1$ à $C_6$, cycloalkyle en $C_3$ à $C_{10}$ ou hétérocyclyle, non substitué ou substitué une ou plusieurs fois ;
$R^2$ représente H ; un groupe alkyle en $C_1$ à $C_6$, non substitué ou substitué une ou plusieurs fois ;
$R^3$ représente un groupe aryle ou hétéroaryle, non substitué ou substitué une ou plusieurs fois ; alkyle en $C_1$ à $C_6$ ou cycloalkyle en $C_3$ à $C_{10}$, chaque fois non substitué ou substitué une ou plusieurs fois ;
$R^4$, $R^5$, $R^6$ et $R^7$ représentent indépendamment les uns des autres l'hydrogène ; un groupe alkyle en $C_1$ à $C_6$, non substitués ou substitués une ou plusieurs fois ;
$R^8$, $R^9$ et $R^{10}$ représentent indépendamment les uns des autres les groupes H, F, Cl, Br, O-alkyle en $C_1$ à $C_6$, $CF_3$, $OCF_3$ , $SCF_3$ , alkyle en $C_1$ à $C_6$ ;
sous réserve que lorsque $R^3$ est le 3-trifluorométhylphényle ou le 4-trifluorométhyl-2-pyridyle, $R^2$, $R^4$ et $R^5$ représentent H et n vaut 0, $R^1$ ne représente pas le 2-pyridyle ou le 2-thiényle ;
et
lorsque $R^3$ représente un groupe phényle ou méthyle, $R^2$, $R^4$ et $R^5$ représentent H et n vaut 0, $R^1$ ne représente pas un groupe 2-thiényle ; un reste alkyle en $C_1$ à $C_6$ comprenant des résidus hydrocarbonés saturés ou insaturés et le reste cycloalkyle et cycloalkyle en $C_3$ à $C_{10}$ comprenant des hydrocarbures saturés ou non saturés mais pas cycliques aromatiques ;
sous la forme du racémate ; des énantiomères, des diastéréoisomères, des mélanges des énantiomères ou des diastéréoisomères ou d'un seul énantiomère ou diastéréoisomère ; des bases et/ou des sels d'acides physiologiquement acceptables.

2. Dérivés de nicotinamide substitués selon la revendication 1, dans lesquels
« à substitution alkyle », « à substitution hétérocyclyle » et « à substitution cycloalkyle » signifient la substitution d'un résidu hydrogène par F, Cl, Br, I, -CN, $NH_2$, NH-alkyle en $C_1$ à $C_6$, NH-alkyle en $C_1$ à $C_6$-OH, alkyle en $C_1$ à $C_6$, N(alkyle en $C_1$ à $C_6)_2$, N(alkyle en $C_1$ à $C_6$-OH$)_2$, $NO_2$, SH, S-alkyle en $C_1$ à $C_6$, S-benzyle, O-alkyle en $C_1$ à $C_6$, OH, O-alkyle en $C_1$ à $C_6$-OH, =O, O-benzyle, C (=O) alkyle en $C_1$ à $C_6$, $CO_2H$, $CO_2$-alkyle en $C_1$ à $C_6$, phényle, phénoxy, morpholinyle, pipéridinyle, pyrrolidinyle ou benzyle ;
et « à substitution aryle » et « à substitution hétéroaryle » signifient la substitution simple ou multiple, par exemple double, triple ou quadruple, d'un ou plusieurs atomes d'hydrogène du système cyclique par F, Cl, Br, I, CN, $NH_2$, NH-alkyle en $C_1$ à $C_6$, NH-alkyle en $C_1$ à $C_6$-OH, N (alkyle en $C_1$ à $C_6)_2$, N (alkyle en $C_1$ à $C_6$-OH$)_2$, $NO_2$, SH, S-alkyle en $C_1$ à $C_6$, OH, O-alkyle en $C_1$ à $C_6$, O-alkyle en $C_1$ à $C_6$-OH, C (=O) alkyle en $C_1$ à $C_6$, C(=O)NH-alkyle en $C_1$ à $C_6$ ; o-pyridyle ; C(=O)-aryle ; C(=O)-N-morpholine ; C(=O)-pipéridine ; C(=O)-pyrrolidine ; C(=O)-pipérazine ; $NHSO_2$-alkyle en $C_1$ à $C_6$, NHCO-alkyle en $C_1$ à $C_6$, $CO_2H$, $CH_2SO_2$-phényle, $CO_2$-alkyle en $C_1$ à $C_6$ , $OCF_3$ ,

$SCF_3$ , $CF_3$ ,

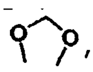, 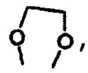,

alkyle en $C_1$ à $C_6$, pyrrolidinyle, pipéridinyle, morpholinyle, benzyloxy, phénoxy, phényle, pyridyle, alkylaryle, imidazolyle, pyrazolyle, thiényle ou furyle.

**3.** Dérivés de nicotinamide substitués selon l'une des revendications 1 ou 2, dans lesquels p et q valent 1.

**4.** Dérivés de nicotinamide substitués selon l'une des revendications 1 ou 2, dans lesquels $R^1$ représente un groupe tert-butyle ; phényle, pyridyle, thiényle, furyle ou cyclohexyle, non substitué ou substitué une ou plusieurs fois.

**5.** Dérivés de nicotinamide substitués selon la revendication 4, dans lesquels $R^1$ représente un groupe cyclohexyle ou phényle, non substitué ou substitué une ou plusieurs fois par F, $CH_3$, Cl, Br, $CF_3$, $OCH_3$, $SCF_3$ ou $OCF_3$ ; pyridyle, thiényle ou furyle, non substitué ou substitué une ou plusieurs fois par $CH_3$.

**6.** Dérivés de nicotinamide substitués selon l'une des revendications 1 ou 2, dans lesquels $R^2$ représente $CH_3$ ou H, en particulier H.

**7.** Dérivés de nicotinamide substitués selon l'une des revendications 1 ou 2, dans lesquels $R^4$, $R^5$, $R^6$ et $R^7$ représentent indépendamment les uns des autres H ou $CH^3$, en particulier H.

**8.** Dérivés de nicotinamide substitués selon l'une des revendications 1 ou 2, dans lesquels n vaut 0 ou 1, plus avantageusement 0.

**9.** Dérivés de nicotinamide substitués selon l'une des revendications 1 ou 2, dans lesquels $R^3$ représente un groupe aryle ou hétéroaryle, non substitué ou substitué une ou plusieurs fois,
de préférence
$R^3$ représente un groupe phényle non substitué ou substitué par $CF_3$ ou $CH_3$.

**10.** Dérivés de nicotinamide substitués selon la revendication 1 choisis dans le groupe
1 2-(2-(phénylsulfonyl)éthylthio)-N-(pyridine-2-ylméthyl)nicotinamide
2 2-(2-(phénylsulfonyl)éthylthio)-N-(pyridine-4-ylméthyl)nicotinamide
3 N-(3-fluorophénéthyl)-2-(2-(phénylsulfonyl)éthyl-thio)nicotinamide
4 N-méthyl-N-(3-méthylbenzyl)-2-(2-(phénylsulfonyl)éthylthio)nicotinamide
5 N-(4-méthylbenzyl)-2-(2-(phénylsulfonyl)éthyl-thio)nicotinamide
6 2-(2-(phénylsulfonyl)éthylthio)-N-(2-(tri-fluorométhyl)benzyl)nicotinamide
7 2-(2-(phénylsulfonyl)éthylthio)-N-(pyridine-3-ylméthyl)nicotinamide
8 N-(3,5-difluorobenzyl)-2-(2-(phénylsulfonyl) - éthylthio)nicotinamide
9 N-méthyl-N-phénéthyl-2-(2-(phénylsulfonyl)-éthylthio)nicotinamide
10 N-(3-méthoxybenzyl)-N-méthyl-2-(2-(phénylsulfonyl)-éthylthio)nicotinamide
11 N-(2-fluorobenzyl)-2-(2-(phénylsulfonyl) - éthylthio)nicotinamide
12 N-(3,4-difluorobenzyl)-2-(2-(phénylsulfonyl)-éthylthio)nicotinamide
13 N- (3-bromobenzyl)-N-méthyl-2-(2- (phénylsulfonyl) - éthylthio)nicotinamide
14 N-(2-méthoxybenzyl)-2-(2-(phénylsulfonyl)-éthylthio)nicotinamide
15 N-(3-fluorobenzyl)-2-(2-(phénylsulfonyl)éthyl-thio)nicotinamide
16 N-(furanne-2-ylméthyl)-N-méthyl-2-(2-(phénylsulfonyl)éthylthio)nicotinamide
17 N-(4-méthoxybenzyl)-2-(2-(phénylsulfonyl)éthyl-thio)nicotinamide
18 N-(2-chlorobenzyl)-2-(2-(phénylsulfonyl)éthyl-thio)nicotinamide
19 N-(3,4-dichlorobenzyl)-2-(2-(phénylsulfonyl)-éthylthio)nicotinamide
20 N-(4-fluorobenzyl)-2-(2-(phénylsulfonyl)éthyl-thio)nicotinamide
21 N-(2-méthoxyphénéthyl)-2-(2-(phénylsulfonyl)-éthylthio)nicotinamide
22 N-(2,6-difluorobenzyl)-2-(2-(phénylsulfonyl)-éthylthio)nicotinamide
23 N-(2-méthylbenzyl)-2-(2-(phénylsulfonyl)-éthylthio)nicotinamide
24 N-(3,5-diméthoxybenzyl)-2-(2-(phénylsulfonyl)-éthylthio)nicotinamide
25 N-(3-chlorobenzyl)-2-(2-(phénylsulfonyl)éthyl-thio)nicotinamide

26 N-(2,4-dichlorobenzyl)-2-(2-(phénylsulfonyl)-éthylthio)nicotinamide
29 N-(4-chlorobenzyl)-2-(2-(phénylsulfonyl)éthyl-thio)nicotinamide
30 N-(2,3-dichlorobenzyl)-2-(2-(phénylsulfonyl)-éthylthio)nicotinamide
31 N-(4-bromobenzyl)-N-méthyl-2-(2-(phénylsulfonyl)-éthylthio)nicotinamide
32 N-((1,3-dioxolanne-2-yl)méthyl)-N-méthyl-2-(2-(phénylsulfonyl)éthylthio)nicotinamide
33 N-benzyl-N-méthyl-2-(2-tosyléthylthio)nicotinamide
34 N-(pyridine-2-ylméthyl)-2-(2-tosyléthylthio)nicotinamide
35 N-(pyridine-4-ylméthyl)-2-(2-tosyléthylthio)nicotinamide
36 N-(thiophène-2-ylméthyl)-2-(2-tosyléthylthio)nicotinamide
37 N-(3-fluorophénéthyl)-2-(2-tosyléthylthio)nicotinamide
38 N-méthyl-N-(3-méthylbenzyl)-2-(2-tosyléthylthio)-nicotinamide
39 N-(furanne-2-ylméthyl)-2-(2-tosyléthylthio)nicotinamide
40 N-(pyridine-3-ylméthyl)-2-(2-tosyléthylthio)nicotinamide
41 N-(3,5-difluorobenzyl)-2-(2-tosyléthylthio)nicotinamide
42 N-(3-méthoxybenzyl)-N-méthyl-2-(2-tosyléthyl-thio)nicotinamide
43 N-(2-fluorobenzyl)-2-(2-tosyléthylthio)nicotinamide
44 N-(3-méthylbenzyl)-2-(2-tosyléthylthio)nicotinamide
45 N-(3,4-difluorobenzyl)-2-(2-tosyléthylthio)nicotinamide
46 N-(3-bromobenzyl)-N-méthyl-2-(2-tosyléthylthio)-nicotinamide
47 N-(4-méthoxybenzyl)-2-(2-tosyléthylthio)nicotinamide
48 N-(2-chlorobenzyl)-2-(2-tosyléthylthio)nicotinamide
49 N-(4-fluorobenzyl)-2-(2-tosyléthylthio)nicotinamide
50 N-(3,5-diméthoxybenzyl)-2-(2-tosyléthylthio)nicotinamide
51 N-(3-chlorobenzyl)-2-(2-tosyléthylthio)nicotinamide
52 2-(2-tosyléthylthio)-N-(3-trifluorométhyl)benzyl)nicotinamide
54 N-benzyl-2-(2-(phénylsulfonyl)éthylthio)nicotinamide
55 N-benzyl-N-méthyl-2-(2-(phénylsulfonyl)éthyl-thio)nicotinamide
56 N-(cyclohexylméthyl)-2-(2-(phénylsulfonyl)éthyl-thio)nicotinamide
57 2-(2-(phénylsulfonyl)éthylthio)-N-(1-(3-(tri-fluorométhyl)phényl)éthyl)nicotinamide
58 N-(2-cyclohexyléthyl)-2-(2-(phénylsulfonyl)éthyl-thio)nicotinamide
59 2-(2-(cyclohexylthio)éthylthio)-N-(thiophène-2-ylméthyl)nicotinamide
60 N-(néopentyl)-2-(2-(phénylsulfonyl)éthyl-thio)nicotinamide
61 N-(5-méthylfuranne-2-ylméthyl)-2-(2-(phénylsulfonyl)éthylthio)nicotinamide
62 N-(furanne-2-ylméthyl)-2-(2-(phénylsulfonyl)-éthylthio)nicotinamide
63 2-(2-(phénylsulfonyl)éthylthio)-N-(tétrahydro-2H-pyranne-4-ylméthyl)nicotinamide
64 2-(2-(phénylsulfonyl)éthylthio)-N-(4-(trifluoro-méthylthio)benzyl)nicotinamide
65 2-(2-(phénylsulfonyl)éthylthio)-N-(3-tolyl-méthyl)nicotinamide
66 (R)-N-(1-cyclohexyléthyl)-2-(2-(phénylsulfonyl)-éthylthio)nicotinamide
67 N-(1-(3,4-diméthylphényl)éthyl)-2-(2-(phénylsulfonyl)éthylthio)nicotinamide
68 N-(1-thiophène-2-yléthyl)-2-(2-(phénylsulfonyl)-éthylthio)nicotinamide
69 N-(1-(3,5-diméthylphényl)méthyl)-2-(2-(phénylsulfonyl)éthylthio)nicotinamide
70 N-(cyclohexylméthyl)-2-(2-(3-trifluorométhyl-phénylsulfonyl)éthylthio)nicotinamide
71 (S)-N-(1-cyclohexyléthyl)-2-(2-(phénylsulfonyl)-éthylthio)nicotinamide
72 N-(1-(3,5-diméthylphényl)éthyl)-2-(2-(phénylsulfonyl)éthylthio)nicotinamide
73 N-(thiophène-2-ylméthyl)-2-(2-(3-trifluoro-méthyl)phénylthio)éthylthio)nicotinamide
74 N-(cyclopentylméthyl)-2-(2-(phénylsulfonyl)-éthylthio)nicotinamide
75 N-(cyclobutylméthyl)-2-(2-(phénylsulfonyl)-éthylthio)nicotinamide
76 N-((1,4-dioxanne-2-yl)méthyl)-2-(2-(phénylsulfonyl)éthylthio)nicotinamide
77 2-(2-(phénylsulfonyl)éthylthio)-N-(4-(pyridine-2-yloxy)benzyl)nicotinamide
78 N-(2-méthylbutyl)-2-(2-(phénylsulfonyl)éthyl-thio)nicotinamide
79 N-(2-éthylbutyl)-2-(2-(phénylsulfonyl)éthyl-thio)nicotinamide
80 N-(cyclopropylméthyl)-2-(2-(phénylsulfonyl)éthyl-thio)nicotinamide
81 N-(3-(2-méthoxyéthoxy)propyl)-2-(2-(phénylsulfonyl)éthylthio)nicotinamide
82 2-(2-(phénylsulfonyl)éthylthio)-N-(1-(4-(tri-fluorométhylthio)phényl)éthyl)nicotinamide
83 N-(3-(1H-pyrazole-1-yl)benzyl)-2-(2-(phénylsulfonyl)éthylthio)nicotinamide
84 N-((2,3-dihydrobenzofuranne-5-yl)méthyl)-2-(2-(phénylsulfonyl)éthylthio)nicotinamide
85 N-(4-phénoxybenzyl)-2-(2-(phénylsulfonyl)éthyl-thio)nicotinamide
86 N-(((1R,2S,5R)-6,6-diméthylbicyclo[3.1.1]heptane-2-yl)méthyl)-2-(2-(phénylsulfonyl)éthylthio)nicotinamide

87 N-(thiophène-2-ylméthyl)-2-(2-(3-trifluoro-méthyl)phénylsulfonyl)éthylthio)nicotinamide
88 2-(2-(phénylsulfonyl)éthylthio)-N-(3-(trifluoro-méthyl)benzyl)nicotinamide
93 N-isobutyl-2-(2-(phénylsulfonyl)éthylthio)-nicotinamide
94 2-[2-(benzosulfonyl)éthylthio]-N-(2-tétrahydro-pyrannylméthyl)-nicotinamide
95 2-[2-(benzosulfonyl)éthylthio]-N-[(5-méthyl-2-thiényl)méthyl]-nicotinamide
96 2-[2-(benzosulfonyl)éthylthio]-N-[(4-méthyl-2-thiényl)méthyl]-nicotinamide
97 N-(1-adamantylméthyl)-2-[2-(benzosulfonyl)-éthylthio]-nicotinamide
98 2-[2-(benzosulfonyl)éthylthio]-N-[(3-morpho-linophényl)méthyl]-nicotinamide
99 2-[2-(4-chlorophényl)sulfonyléthylthio]-N-(2-thiénylméthyl)-nicotinamide
100 2-[2-(4-fluorophényl)sulfonyléthylthio]-N-(2-thiénylméthyl)-nicotinamide
101 N-(2-thiénylméthyl)-2-[2-(3-(trifluorométhoxy)-phényl]sulfonyléthylthio]-nicotinamide
102 N-(2-thiénylméthyl)-2-[2-(4-(trifluorométhyl)-phényl]sulfonyléthylthio]-nicotinamide
103 N-(2-thiénylméthyl)-2-[2-(4-(trifluorométhoxy)-phényl]sulfonyléthylthio]-nicotinamide
104 2-[2-(m-tolylsulfonyl)éthylthio]-N-(2-thiényl-méthyl)-nicotinamide
105 2-[2-(m-tolylthio)éthylthio]-N-(2-thiénylméthyl)-nicotinamide
106 2-[2-(3-fluorophényl)sulfonyléthylthio]-N-(2-thiénylméthyl)-nicotinamide
107 2-[2-(benzosulfonyl)éthylthio]-N-(3,3-diméthyl-butyl)-nicotinamide
108 2-[2-(benzosulfonyl)éthylthio]-N-(2-benzothio-phénylméthyl)-nicotinamide
109 2-[2-(phénylthio)éthylthio]-N-(2-thiénylméthyl)-nicotinamide
110 2-[2-(benzosulfinyl)éthylthio]-N-(2-thiényl-méthyl)-nicotinamide
111 2-(2-cyclohexylsulfonyléthylthio)-N-(2-thiényl-méthyl)-nicotinamide
112 N-(2-thiénylméthyl)-2-[2-[[2-(trifluorométhyl)-phényl]thio]éthylthio]-nicotinamide
113 N-(2-thiénylméthyl)-2-[2-[2-(trifluorométhyl)-phényl]sulfinyléthylthio]-nicotinamide
114 N-(2-thiénylméthyl)-2-[2-[2-(trifluorométhyl)-phényl]sulfonyléthylthio]-nicotinamide
115 2-[2-(benzosulfonyl)éthylthio]-N-[(5-chloro-2-thiényl)méthyl]-nicotinamide
116 2-[2-(2-fluorophényl)sulfonyléthylthio]-N-(2-thiénylméthyl)-nicotinamide
117 2-[2-[3,5-bis(trifluorométhyl)phényl]sulfonyl-éthylthio]-N-(2-thiénylméthyl)-nicotinamide
118 2-[2-(3-méthoxyphényl)sulfonyléthylthio]-N-(2-thiénylméthyl)-nicotinamide
119 2-[2-(4-méthoxyphényl)sulfonyléthylthio]-N-(2-thiénylméthyl)-nicotinamide
120 2-[2-(benzosulfonyl)éthylthio]-N-(4-tétra-hydrothiopyrannylméthyl)-nicotinamide
121 2-[2-(4-éthylphényl)sulfonyléthylthio]-N-(2-thiénylméthyl)-nicotinamide
122 N-(2-thiénylméthyl)-2-[2-[[4-(trifluorométhyl)-phényl]thio]éthylthio]-nicotinamide
123 2-[2-(o-tolylthio)éthylthio]-N-(2-thiénylméthyl)-nicotinamide
124 2-[2-[(3-fluorophényl)thio]éthylthio]-N-(2-thiénylméthyl)-nicotinamide
125 2-[2-[(3,4-difluorophényl)thio]êthylthio]-N-(2-thiénylméthyl)-nicotinamide
126 2-[2-[(2,4-difluorophényl)thio]éthylthio]-N-(2-thiénylméthyl)-nicotinamide
127 2-[2-(benzosulfonyl)éthylthio]-N-[2-(2-thié-nyl)éthyl]-nicotinamide
128 2-[2-(benzosulfonyl)éthylthio]-N-phénéthyl-nicotinamide
129 2-[2-(benzosulfonyl)éthylthio]-N-(3-phényl-propyl)-nicotinamide
130 2-[2-[(3,4-difluorophényl)sulfonyléthylthio]-N-(2-thiénylméthyl)-nicotinamide
131 2-[2-[(2,4-difluorophényl)sulfonyléthylthio]-N-(2-thiénylméthyl)-nicotinamide
132 2-[2-[(2-fluorophényl)thio]éthylthio]-N-(2-thié-nylméthyl)-nicotinamide
133 2-[2-[(4-fluorophényl)thio]éthylthio]-N-(2-thiénylméthyl)-nicotinamide
134 2-[2-[(4-chlorophényl)thio]éthylthio]-N-(2-thié-nylméthyl)-nicotinamide
135 2-[2-(p-tolylthio)éthylthio]-N-(2-thiénylméthyl)-nicotinamide
136 2-[2-(benzosulfonyl)éthylthio]-N-isopentyl-nicotinamide
137 2-[2-(benzosulfonyl)éthylthio]-N-(2-cyclopropyl-éthyl)-nicotinamide
138 2-[2-(benzosulfonyl)éthylthio]-N-(2-cyclopentyl-éthyl)-nicotinamide
139 N-(3,3-diméthylbutyl)-2-[2-[3-(trifuloro-méthyl)phényl]sulfonyléthylthio]-nicotinamide
140 N-(cyclopentylméthyl)-2-[2-[3-(trifluorométhyl)-phényl]sulfonyléthylthio]-nicotinamide
141 2-[2-(benzosulfonyl)éthylthio]-6-méthyl-N-(2-thiénylméthyl)-nicotinamide
142 2-[2-(benzosulfonyl)éthylthio]-N-(2-thiényl-méthyl)-6-(trifluorométhyl)-nicotinamide
143 2-[2-(benzosulfonyl)éthylthio]-6-fluoro-N-(2-thiénylméthyl)-nicotinamide
144 2-[2-(benzosulfonyl)éthylthio]-N-[(3-méthyl-cyclohexyl)méthyl]-nicotinamide
145 2-[2-(benzosulfonyl)éthylthio]-N-(cycloheptyl-méthyl)-nicotinamide
146 2-[2-(benzosulfonyl)éthylthio]-N-[(2-méthyl-cyclohexyl)méthyl]-nicotinamide
147 2-[2-(benzosulfonyl)éthylthio]-N-[(4-méthyl-cyclohexyl)méthyl]-nicotinamide
148 2-[2-(benzosulfonyl)éthylthio]-5-fluoro-N-(2-thiénylméthyl)-nicotinamide

149 2-[2-(benzosulfonyl)éthylthio]-5-méthyl-N-(2-thiénylméthyl)-nicotinamide
150 2-[2-(benzosulfonyl)éthylthio]-N-(2-thiényl-méthyl)-5-(trifluorométhyl)-nicotinamide.

**11.** Médicament contenant au moins un composé de nicotinamide substitué selon l'invention de formule générale 1 selon la revendication 1, dans lequel
n = 0,1 ou 2
p = 0 ou 1
q = 0 ou 1
$R^1$ représente un groupe aryle ou hétéroaryle, non substitué ou substitué une ou plusieurs fois ; alkyle en $C_1$ à $C_6$, cycloalkyle en $C_3$ à $C_{10}$ ou hétérocyclyle, non substitué ou substitué une ou plusieurs fois ;
$R^2$ représente H ; un groupe alkyle en $C_1$ à $C_6$, non substitué ou substitué une ou plusieurs fois ;
$R^3$ représente un groupe aryle ou hétéroaryle, non substitué ou substitué une ou plusieurs fois ; alkyle en $C_1$ à $C_6$ ou cycloalkyle en $C_3$ à $C_{10}$, chaque fois non substitué ou substitué une ou plusieurs fois ;
$R^4$, $R^5$, $R^6$ et $R^7$ représentent indépendamment les uns des autres l'hydrogène ; un groupe alkyle en $C_1$ à $C_6$, non substitués ou substitués une ou plusieurs fois ;
$R^8$, $R^9$ et $R^{10}$ représentent indépendamment les uns des autres l'hydrogène, les groupes F, Cl, Br, O-alkyle en $C_1$ à $C_6$ , $CF_3$ , $OCF_3$ , $SCF_3$ , alkyle en $C_1$ à $C_6$ ;
sous la forme du racémate ; des énantiomères, des diastéréoisomères, des mélanges des énantiomères ou des diastéréoisomères ou d'un seul énantiomère ou diastéréoisomère ; des bases et/ou des sels d'acides physiologiquement acceptables
et le cas échéant un ou plusieurs excipients pharmaceutiquement acceptables.

**12.** Médicament selon la revendication 11 choisi dans le groupe
89 2-(2-(phénylsulfonyl)éthylthio)-N-(thiophène-2-ylméthyl)nicotinamide
90 N-(pyridine-2-ylméthyl)-2-(2-(3-(trifluoro-méthyl)-phénylsulfonyl)éthylthio)nicotinamide
91 N-(pyridine-2-ylméthyl)-2-(2-(5-(trifluoro-méthyl)pyridine-2-ylsulfonyl)éthylthio)-nicotinamide
92 N-(thiophène-2-ylméthyl)-2-(2-(5-(trifluoro-méthyl)pyridine-2-ylsulfonyl)éthylthio)-nicotinamide.

**13.** Utilisation de composés de nicotinamide substitués selon la revendication 1 ou 11 pour la fabrication d'un médicament destiné au traitement de la douleur, de préférence la douleur choisie dans le groupe constitué par la douleur aiguë, la douleur chronique, la douleur neuropathique, la douleur musculaire et la douleur inflammatoire.

**14.** Utilisation de composés selon la revendication 1 ou 11 pour la fabrication d'un médicament destiné au traitement de l'épilepsie, de la migraine, des états d'angoisse et de l'incontinence urinaire.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20020128277 A
- WO 2005023802 A


### In der Beschreibung aufgeführte Nicht-Patentliteratur


- **PASSMORE et al.** *J Neurosci.,* 2003, vol. 23 (18), 7227-36
- **BLACKBURN-MUNRO ; JENSEN.** *Eur J Pharmacol.,* 2003, vol. 460 (2-3), 109-16
- **DOST et al.** *Naunyn Schmiedebergs Arch Pharmacol,* 2004, vol. 369 (4), 382-390
- **NIELSEN et al.** *Eur J Pharmacol.,* 2004, vol. 487 (1-3), 93-103
- **GRIBKOFF.** *Expert Opin Ther Targets,* 2003, vol. 7 (6), 737-748
- **KORSGAARD et al.** *J Pharmacol Exp Ther.,* 2005, vol. 14 (1), 282-92
- **WICKENDEN et al.** *Expert Opin Ther Pat,* 2004, vol. 14 (4), 457-469
- **STRENG et al.** *J Urol,* 2004, vol. 172, 2054-2058
- **DE SARRO et al.** *Naunyn-Schmiedeberg's Arch. Pharmacol.,* 2001, vol. 363, 330-336
- Remingtons Pharmaceutical Sciences. Mack Publishing Company, 1985
- **HAMILL OP ; MARTY A ; NEHER E ; SAKMANN B ; SIGWORTH FJ.** Improved patch-clamp techniques for high-resolution current recording from cells and cell-free membrane patches. *Pflugers Arch. 1,* August 1981, vol. 391 (2), 85-100
- **D. DUBUISSON ; S.G. DENNIS.** *Pain,* 1977, vol. 4, 161-174
- **T.J. CODERRE ; J. KATZ ; A.L. VACCARINO ; R. MELZACK.** *Pain,* 1993, vol. 52, 259
- **KIM,S.H. ; CHUNG,J.M.** An experimental model for peripheral neuropathy produced by segmental spinal nerve ligation in the rat. *Pain,* 1992, vol. 50, 355-363